# EUROPÄISCHE PATENTSCHRIFT

(11) **EP 2 084 151 B1**
(45) Veröffentlichungstag und Bekanntmachung des Hinweises auf die Patenterteilung: **29.05.2013**
(21) Anmeldenummer: 07818948.7
(22) Anmeldetag: 12.10.2007
(51) Int. Cl.: C07D 401/14, C07D 403/14, C07D 413/14, C07D 417/14, A61K 31/41, A61K 31/435, A61K 31/495, A61P 7/00, A61P 9/00

(54) **SUBSTITUIERTE DIHYDROPYRAZOLONE ZUR BEHANDLUNG KARDIOVASKULÄRER UND HÄMATOLOGISCHER ERKRANKUNGEN**
SUBSTITUTED DIHYDROPYRAZOLONES FOR TREATING CARDIOVASCULAR AND HAEMATOLOGICAL DISEASES
DIHYDROPYRAZOLONES SUBSTITUÉES UTILISÉES DANS LE TRAITEMENT DE MALADIES CARDIOVASCULAIRES ET HÉMATOLOGIQUES

(30) Priorität: 26.10.2006 DE 102006050516
(43) Veröffentlichungstag der Anmeldung: 05.08.2009
(60) Teilanmeldung: 11175197.0
(73) Patentinhaber: Bayer Intellectual Property GmbH, 40789 Monheim (DE)
(72) Erfinder: THEDE, Kai, 10405 Berlin (DE); FLAMME, Ingo, 51580 Reichshof (DE); OEHME, Felix, 42113 Wuppertal (DE); ERGÜDEN, Jens-Kerim, 42489 Wülfrath (DE); STOLL, Friederike, 40215 Düsseldorf (DE); SCHUHMACHER, Joachim, 42113 Wuppertal (DE); WILD, Hanno, 42113 Wuppertal (DE); KOLKHOF, Peter, 42113 Wuppertal (DE); BECK, Hartmut, 50733 Köln (DE); KELDENICH, Jörg, 42113 Wuppertal (DE); AKBABA, Metin, 40880 Ratingen (DE); JESKE, Mario, 42699 Solingen (DE)
(74) Vertreter: BIP Patents
(86) Internationale Anmeldenummer: PCT/EP2007/008877
(87) Internationale Veröffentlichungsnummer: WO 2008/067871

(56) Entgegenhaltungen:
- WO-A-2005/030121
- WO-A-2006/114213
- WO-A1-2006/114400

## Beschreibung

Die vorliegende Anmeldung betrifft neue, substituierte Dihydropyrazolon-Derivate, Verfahren zu ihrer Herstellung, sowie ihre Verwendung zur Herstellung von Arzneimitteln zur Behandlung und/oder Prophylaxe von Krankheiten, insbesondere von kardiovaskulären und hämatologischen Erkrankungen, von Nierenerkrankungen sowie zur Förderung der Wundheilung.

Eine mangelhafte Versorgung des menschlichen Organismus oder seiner Teile mit Sauerstoff, die entweder durch ihre Dauer und/oder ihr Ausmaß eine regelrechte Funktion des Organismus oder seiner Teile beeinträchtigt oder ihre Funktion völlig zum Erliegen bringt, wird als Hypoxie bezeichnet. Eine Hypoxie kann verursacht werden durch eine Verminderung des verfügbaren Sauerstoffs in der Atemluft (z.B. bei Aufenthalten in großer Höhe), durch Störungen der äußeren Atmung (z.B. infolge gestörter Funktion der Lungen oder Verlegung der Atemwege), durch eine Verminderung des Herzzeitvolumens (z.B. bei einer Herzinsuffizienz, einer akuten Rechtsherzüberlastung bei Lungenembolie), durch eine zu geringe Sauerstoff-Transportkapazität des Blutes (z.B. infolge einer Blutarmut (Anämie) oder Intoxikation z.B. mit Kohlenmonoxid), örtlich begrenzt durch eine Minderdurchblutung infolge von Gefäßverschlüssen (Ischämie-Zustände typischerweise z.B. des Herzens, der unteren Extremitäten oder des Gehirns, diabetische Makro- und Mikroangiopathie) oder auch durch erhöhten Sauerstoffbedarf des Gewebes (z.B. infolge erhöhter Muskelarbeit oder lokaler Entzündungen) [Eder, Gedigk (Hrsg.), Allgemeine Pathologie und pathologische Anatomie, 33. Aufl., Springer Verlag, Berlin, 1990].

Der menschliche Organismus ist bedingt fähig, sich an Situationen verminderter Sauerstoffversorgung akut und chronisch anzupassen. Neben einer Sofortantwort, welche u.a. durch vegetativnervöse Kontrollmechanismen eine Erhöhung des Herzzeitvolumens und des Atemzeitvolumens sowie eine lokale Erweiterung von Blutgefäßen einschließt, zieht die Hypoxie eine Veränderung der Transkription zahlreicher Gene nach sich. Die Funktion der Genprodukte dient dabei der Kompensation des Sauerstoffmangels. So werden mehrere Enzyme der Glykolyse und der Glukosetransporter 1 verstärkt exprimiert, wodurch die anaerobe ATP-Gewinnung gesteigert und ein Überleben des Sauerstoffmangels ermöglicht wird [Schmidt, Thews (Hrsg.), Physiologie des Menschen, 27. Aufl., Springer Verlag, Berlin, 1997; Löffler, Petrides (Hrsg.), Biochemie und Pathobiochemie, 7. Aufl., Springer Verlag, Berlin, 2003].

Ferner führt Hypoxie zur verstärkten Expression des vaskulären Endothelzellwachstumsfaktors, VEGF, wodurch in hypoxischen Geweben die Neubildung von Blutgefäßen (Angiogenese) angeregt wird. Hierdurch wird langfristig die Durchblutung ischämischer Gewebe verbessert. Bei verschiedenen Herzkreislauferkrankungen und Gefäßverschluß-Erkrankungen erfolgt diese Gegenregulation offenbar nur sehr unzureichend [Übersicht bei: Simons und Ware, Therapeutic angiogenesis in cardiovascular disease, Nat. Rev. Drug. Discov. 2 (11), 863-71 (2003)].

Ferner wird bei systemischer Hypoxie das vorwiegend in den interstitiellen Fibroblasten der Nieren gebildete Peptidhormon Erythropoietin verstärkt exprimiert. Hierdurch wird die Bildung roter Blutzellen im Knochenmark angeregt und damit die Sauerstoff-Transportkapazität des Blutes gesteigert. Dieser Effekt wurde und wird von Leistungssportlern beim sogenannten Höhentraining genutzt. Eine Abnahme der Sauerstoff-Transportkapazität des Blutes z.B. infolge einer Blutungsanämie verursacht normalerweise eine Zunahme der Erythropoietin-Produktion in der Niere. Bei bestimmten Formen der Anämie kann dieser Regelmechanismus gestört oder sein Sollwert niedriger eingestellt sein. So wird z.B. bei Patienten, die unter einer Niereninsuffizienz leiden, Erythropoietin im Nierenparenchym zwar produziert, jedoch bezogen auf die Sauerstoff-Transportkapazität des Blutes in deutlich verminderten Mengen, was eine sogenannte renale Anämie zur Folge hat. Insbesondere die renale Anämie, aber auch Tumor- und HIV-Infektion-bedingte Anämien werden üblicherweise durch parenterale Verabfolgung von rekombinantem humanen Erythropoietin (rhEPO) behandelt. Derzeit existiert zu dieser kostspieligen Therapie keine alternative Therapie mit einem oral verfügbaren Arzneistoff [Übersichten bei: Eckardt, The potential of erythropoietin and related strategies to stimulate erythropoiesis, Curr. Opin. Investig. Drugs 2(8), 1081-5 (2001); Berns, Should the target hemoglobin for patients with chronic kidney disease treated with erythropoietic replacement therapy be changed?, Semin. Dial. 18 (1), 22-9 (2005)]. Jüngere Untersuchungen belegen, dass Erythropoetin neben seiner die Erythropoese steigernden Wirkung auch eine hiervon unabhängige, schützende (anti-apoptotische) Wirkung auf hypoxische Gewebe, insbesondere das Herz und das Gehirn ausübt. Ferner mindert eine Therapie mit Erythropoetin neueren Studien zufolge an herzinsuffizienten Patienten den durchschnittlichen Morbiditäts-Schweregrad [Übersichten bei: Caiola und Cheng, Use of erythropoietin in heart failure management, Ann. Pharmacother. 38 (12), 2145-9 (2004); Katz, Mechanisms and treatment of anemia in chronic heart failure, Congest. Heart. Fail. 10 (5), 243-7 (2004)].

Den oben beschriebenen durch Hypoxie induzierten Genen ist gemeinsam, dass die Steigerung ihrer Expression unter Hypoxie durch den sogenannten Hypoxie-induzierbaren Transkriptionsfaktor (HIF) hervorgerufen wird. Bei HIF handelt es sich um einen heterodimeren Transkriptionsfaktor, der aus einer alpha- und einer beta-Untereinheit besteht. Es wurden drei HIF-alpha-Isoformen beschrieben, von denen HIF-1 alpha und HIF-2 alpha hoch homolog und von Bedeutung für die Hypoxie-induzierte Genexpression sind. Während die auch als ARNT (aryl hydrocarbon receptor nuclear translocator) bezeichnete beta-Untereinheit (von der 2 Isoformen beschrieben wurden) konstitutiv exprimiert ist, hängt die Expression der alpha-Untereinheit vom Sauerstoffgehalt in der Zelle ab. Unter Normoxie wird das HIF-alpha-Protein poly-ubiquitiniert und anschließend proteasomal degradiert. Unter Hypoxie ist diese Degradierung gehemmt, so dass HIF-alpha mit ARNT dimerisieren und seine Zielgene aktivieren kann. Das HIF-Dimer bindet hierbei an sogenannte Hypoxie-responsible Elemente (HRE) in den regulatorischen Sequenzen seiner Zielgene. Die HRE sind durch eine Konsensus-Sequenz definiert. Funktionelle HRE wurden in den regulatorischen Elementen zahlreicher Hypoxie-induzierter Gene nachgewiesen [Übersichten bei: Semenza, Hypoxia-inducible factor 1: oxygen homeostasis and disease pathophysiology, Trends Mol. Med. 7 (8), 345-50 (2001); Wenger und Gassmann, Oxygen(es) and the hypoxia-inducible factor-1, Biol. Chem. 378 (7), 609-16 (1997)].

Der molekulare Mechanismus, der dieser Regulation von HIF-alpha zugrunde liegt, wurde durch die Arbeiten mehrerer voneinander unabhängiger Forschergruppen aufgeklärt. Der Mechanismus ist Spezies-übergreifend konserviert: HIF-alpha wird durch eine als PHD oder EGLN bezeichnete Subklasse von Sauerstoff-abhängigen Prolyl-4-Hydroxylasen an zwei spezifischen Prolyl-Resten hydroxyliert (P402 und P564 der humanen HIF-1-alpha-Untereinheit). Bei den HIF-Prolyl-4-Hydroxylasen handelt es sich um Eisen-abhängige, 2-Oxoglutarat-umsetzende Dioxygenasen [Epstein et al., C. elegans EGL-9 and mammalian homologs define a family of dioxygenases that regulate HIF by prolyl hydroxylation, Cell 107 (1), 43-54 (2001); Bruick und McKnight, A conserved family of prolyl-4-hydroxylases that modify HIF, Science 294 (5545), 1337-40 (2001); Ivan et al., Biochemical purification and pharmacological inhibition of a mammalian prolyl hydroxylase acting on hypoxia-inducible factor, Proc. Natl. Acad. Sci. U.S.A. 99 (21), 13459-64 (2002)]. Die Enzyme wurden 2001 erstmals als Prolyl-Hydroxylasen annotiert [Aravind und Koonin, *The DNA-repair protein AlkB, EGL-9, and leprecan define new families of 2-oxoglutarate- and irondependent dioxygenases,* Genome Biol. 2 (3), research0007.1-0007.8, Epub 2001 Feb 19].

An die prolylhydroxylierte HIF-alpha-Untereinheit bindet das pVHL Tumor Suppressor Protein, welches zusammen mit Elongin B und C den sogenannten VBC-Komplex bildet, der die HIF-alpha-Untereinheit an eine E3 Ubiquitin-Ligase adaptiert. Da die Prolyl-4-Hydroxylierung der HIF-alpha-Untereinheit und ihre nachfolgende Degradierung in Abhängigkeit von der intrazellulären Konzentration des Sauerstoffs erfolgt, wurden die HIF-Prolyl-4-Hydroxylasen auch als zellulärer Sauerstoffsensor bezeichnet. Es wurden drei Isoformen dieser Enzyme identifiziert: EGLN1/PHD2, EGLN2/PHD1 und EGLN3/PHD3. Zwei dieser Enzyme (EGLN2/PHD1 und EGLN3/PHD3) werden selbst unter Hypoxie transkriptionell induziert und sind möglicherweise für die unter chronischer Hypoxie zu beobachtende Absenkung der HIF-alpha-Spiegel verantwortlich [Übersicht bei: Schofield und Ratcliffe, Oxygen sensing by HIF hydroxylases, Nat. Rev. Mol. Cell. Biol. 5 (5), 343-54 (2004)].

Eine selektive pharmakologische Hemmung der HIF-Prolyl-4-Hydroxylasen zieht die Steigerung der Genexpression HIF-abhängiger Zielgene nach sich und ist damit für die Therapie zahlreicher Krankheitsbilder von Nutzen. Insbesondere bei Erkrankungen des Herz-Kreislaufsystems ist von der Induktion neuer Blutgefäße sowie der Änderung der Stoffwechselsituation ischämischer Organe von aerober hin zu anaerober ATP-Gewinnung eine Besserung des Krankheitsverlaufs zu erwarten. Eine Verbesserung der Vaskularisierung chronischer Wunden fördert den Heilungsprozess, insbesondere bei schwer heilenden Ulcera cruris und anderen chronischen Hautwunden. Die Induktion von körpereigenem Erythropoietin bei bestimmten Krankheitsformen, insbesondere bei Patienten mit renaler Anämie, ist ebenfalls ein anzustrebendes therapeutisches Ziel.

Die bislang in der wissenschaftlichen Literatur beschriebenen HIF-Prolyl-4-Hydroxylase-Inhibitoren erfüllen nicht die an ein Arzneimittel zu stellenden Anforderungen. Es handelt sich hierbei entweder um kompetitive Oxoglutarat-Analoga (wie z.B. N-Oxalylglycin), die durch ihre sehr geringe Wirkstärke charakterisiert sind und daher in *in* vivo-Modellen bislang keine Wirkung im Sinne einer Induktion von HIF-Zielgenen gezeigt haben. Oder es handelt sich um Eisen-Komplexbildner (Chelatoren) wie Desferroxamin, die als unspezifische Hemmstoffe Eisen-haltiger Dioxygenasen wirken und, obwohl sie eine Induktion der Zielgene wie z.B. Erythropoetin *in vivo* herbeiführen, durch Komplexierung des verfügbaren Eisens offenbar der Erythropoese entgegenwirken.

Aufgabe der vorliegenden Erfindung ist die Bereitstellung neuer Verbindungen, die für die Behandlung von Erkrankungen, insbesondere kardiovaskulärer und hämatologischer Erkrankungen, eingesetzt werden können.

Im Rahmen der vorliegenden Erfindung werden nunmehr Verbindungen beschrieben, die als spezifische Hemmstoffe der HIF-Prolyl-4-Hydroxylasen wirken und aufgrund dieses spezifischen Wirkmechanismus *in vivo* nach parenteraler oder oraler Verabreichung die Induktion von HIF-Zielgenen, wie z.B. Erythropoetin, und der hierdurch verursachten biologischen Prozesse, wie z.B. Erythropoese, herbeiführen.

2-Heteroaryl-4-aryl-1,2-dihydropyrazolone mit bakterizider und/oder fungizider Wirkung werden in EP 165 448 und EP 212 281 offenbart. Die Verwendung von 2-Heteroaryl-4-aryl-1,2-dihydropyrazolonen als Lipoxygenase-Inhibitoren zur Behandlung von Atemwegs-, Herz-Kreislauf- und Entzündungserkrankungen wird in EP 183 159 beansprucht. 2,4-Diphenyl-1,2-dihydropyrazolone mit herbizider Aktivität werden in DE 2 651 008 beschrieben. Über die Herstellung und pharmakologischen Eigenschaften bestimmter 2-Pyridyl-1,2-dihydropyrazolone wird in *Helv. Chim. Acta 49* (1), 272-280 (1966) berichtet. In WO 96/12706, WO 00/51989 und WO 03/074550 werden Verbindungen mit Dihydropyrazolon-Partialstruktur zur Behandlung unterschiedlicher Erkrankungen beansprucht, und in WO 2006/101903 werden Hydroxy- oder Alkoxy-substituierte Bipyrazole zur Behandlung neuropsychiatrischer Erkrankungen offenbart. Weiterhin werden in WO 03/051833 und WO 2004/089303 Heteroaryl-substituierte Pyrazol-Derivate zur Behandlung von Schmerz und verschiedenen ZNS-Erkrankungen beschrieben. Zwischenzeitlich sind in WO 2006/114213 2,4-Dipyridyl-1,2-dihydropyrazolone als Inhibitoren von HIF-Prolyl-4-Hydroxylasen offenbart worden.

Über die Röntgenkristallstruktur der Verbindung 3-Methyl-1-(pyridin-2-yl)-4-(1-pyridin-2-yl-3-methyl-1*H-*pyrazol-5-yl)-2*H-*3-pyrazolin-5(1*H*)-on (andere Bezeichnung: 5,5'-Dimethyl-2,2'-dipyridin-2-yl-1',2'-dihydro-2*H*,3'*H* 3,4'-bipyrazol-3'-on) wird in *Acta Crystallogr., Section E: Structure Reports Online* E57 (11), o1126-o1127 (2001) *[Chem. Abstr.* 2001:796190] berichtet. Die Synthese bestimmter 3',5-Dimethyl-2-phenyl-1'-(1,3-thiazol-2-yl)-1'*H*,2*H*-3,4'-bipyrazol-5'-ol-Derivate ist in *Indian* J. Heterocyclic Chem. 3 (1), 5-8 (1993) *[Chem. Abstr.* 1994:323362] beschrieben. Über Herstellung und Tautomerie einzelner 4-(Pyrazol-5-yl)-pyrazolin-5-on-Derivate wird in J. Heterocyclic Chem. 27 (4), 865-870 (1990) *[Chem. Abstr.* 1991:428557] berichtet. Eine therapeutische Anwendung ist für die in diesen Publikationen genannten Verbindungen bislang nicht beschrieben worden. In WO 2007/008541 wird die Verbindung 2-*tert*.-Butyl-1'-[4-(4-chlorphenyl)-1,3-thiazol-2-yl]-3',5-dimethyl-1'*H*,2*H*-3,4'-bipyrazol-5'-ol als ein Testbeispiel aufgeführt.

Gegenstand der vorliegenden Erfindung sind Verbindungen der allgemeinen Formel (I) 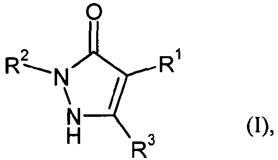 in welcher
- R¹: für eine Heteroaryl-Gruppe der Formel 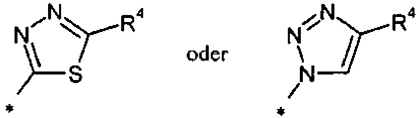 steht, worin
* die Verknüpfungsstelle mit dem Dihydropyrazolon-Ring bedeutet und
R⁴ Wasserstoff, Fluor, Chlor, Brom, Cyano, (C₁-C₄)-Alkyl, Trifluormethyl, Hydroxymethyl, (C₁-C₄)-Alkoxy, Trifluormethoxy, Hydroxycarbonyl oder (C₁-C₄)-Alkoxycarboxyl bedeutet,
- R²: für eine Heteroaryl-Gruppe der Formel 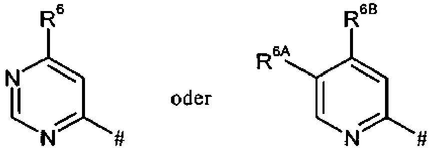 steht, worin
# die Verknüpfungsstelle mit dem Dihydropyrazolon-Ring bedeutet und
R⁶, R^{6A} und R^{6B} gleich oder verschieden sind und unabhängig voneinander Wasserstoff oder einen Substituenten ausgewählt aus der Reihe Fluor, Chlor, Brom, Cyano, (C₁-C₆)-Alkyl, Trifluormethyl, Hydroxy, (C₁-C6₎-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Heterocycloalkyl, Phenyl und 5- oder 6-gliedriges Heteroaryl bedeuten, wobei
(C₁-C₆)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können, und
- R³: für Wasserstoff steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

(C₁-C₆)-Alkyl und (C₁-C₄-)Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, sec.-Butyl, *tert.*-Butyl*,* 1-Ethylpropyl, *n*-Pentyl und *n*-Hexyl.

(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy, *tert*.-Butoxy, *n*-Pentoxy und *n-*Hexoxy.

Mono-(C₁₋C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino und *tert.*-Butylamino.

Di-(C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N-*Dimethylamino, *N,N-*Diethylamino, *N-*Ethyl-*N-*methylamino, N-Methyl-*N*-*n*-propylamino, *N*-Isopropyl-*N*-*n*-propylamino, *N,N-*Diisopropylamino, *N-n*-Butyl-*N-*methylamino und *N-tert.*-Butyl-*N*-methylamino.

(C₁-C₄)-Alkoxycarbonyl) steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, *n*-Propoxycarbonyl, Isopropoxycarbonyl, n-Butoxycarbonyl und *tert*.-Butoxycarbonyl.

4- bis 6-gliedriges Heterocycloalkyl steht im Rahmen der Erfindung für einen mono- oder gegebenenfalls bicyclischen, gesättigten oder eine Doppelbindung enthaltenden Heterocyclus mit insgesamt 4 bis 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Azetidinyl, Oxetanyl, Thietanyl, Pyrrolidinyl, Pyrrolinyl, Pyrazolidinyl, Dihydropyrazolyl, Tetrahydrofuranyl, Thiolanyl, 1,3-Oxazolidinyl, 1,3-Thiazolidinyl, Piperidinyl, Tetrahydropyridyl, Piperazinyl, Tetrahydropyranyl, Dihydropyranyl, Tetrahydrothiopyranyl, 1,3-Dioxanyl, 1,4-Dioxanyl, Morpholinyl und thiomorpholinyl. Bevorzugt im Rahmen der Erfindung ist ein monocyclischer, gesättigter 4- bis 6-gliedriger Heterocycloalkyl-Rest mit insgesamt 4 bis 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Azetidinyl, Oxetanyl, Thietanyl, Pyrrolidinyl, Pyrazolidinyl, Tetrahydrofuranyl, Thiolanyl, 1,3-Oxazolidinyl, Piperidinyl, Piperazinyl, Tetrahydropyranyl, Tetrahydrothiopyranyl, 1,3-Dioxanyl, 1,4-Dioxanyl, Morpholinyl und Thiomorpholinyl. Besonders bevorzugt ist ein 4- bis 6-gliedriger Heterocycloalkyl-Rest mit insgesamt 4 bis 6 Ringatomen, der ein oder zwei Ring-Heteroatome aus der Reihe N und/oder O enthält, wie beispielsweise Pyrrolidinyl, Tetrahydrofuranyl, Piperidinyl, Piperazinyl, Tetrahydropyranyl und Morpholinyl.

5- oder 6-gliedriges Heteroaryl steht im Rahmen der Erfindung für einen aromatischen Heterocyclus (Heteroaromaten) mit insgesamt 5 bzw. 6 Ringatomen, der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl, Triazinyl. Bevorzugt sind 5- oder 6-gliedrige Heteroaryl-Reste mit bis zu drei Ring-Heteroatomen aus der Reihe N, O und/oder S, wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl.

Halogen schließt im Rahmen der Erfindung Fluor, Chlor, Brom und Iod ein. Bevorzugt sind Fluor, Chlor und Brom, besonders bevorzugt Fluor und Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Besonders bevorzugt ist die Substitution mit einem oder zwei gleichen oder verschiedenen Substituenten.

Bevorzugt im Rahmen der vorliegenden Erfindung sind Verbindungen der Formel (I), in welcher
- R¹: für eine Heteroaryl-Gruppe der Formel 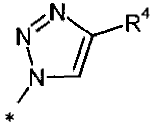 steht, worin
* die Verknüpfungsstelle mit dem Dihydropyrazolon-Ring bedeutet und
R⁴ Wasserstoff, Fluor, Chlor, Brom, Cyano, (C₁-C₄)-Alkyl, Trifluormethyl, Hydroxymethyl, (C₁-C₄)-Alkoxy, Trifluormethoxy, Hydroxycarbonyl oder (C₁-C₄)-Alkoxycarbonyl bedeutet,
- R²: für eine Heteroaryl-Gruppe der Formel 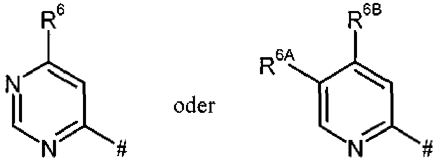 steht, worin
# die Verknüpfungsstelle mit dem Dihydropyrazolon-Ring bedeutet und
R⁶, R^{6A} und R^{6B} gleich oder verschieden sind und unabhängig voneinander Wasserstoff oder einen Substituenten ausgewählt aus der Reihe Fluor, Chlor, Brom, Cyano, (C₁-C₆)-Alkyl, Trifluormethyl, Hydroxy, (C₁-C₆)-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Heterocycloalkyl, Phenyl und 5- oder 6-gliedriges Heteroaryl bedeuten, wobei
(C₁-C₆)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können, und
- R³: für Wasserstoff steht,
sowie ihre Salze, Solvate und Solvate der Salze.

Bevorzugt im Rahmen der vorliegenden Erfindung sind auch Verbindungen der Formel (I), in welcher
- R¹: für eine Heteroaryl-Gruppe der Formel 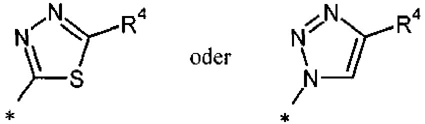 steht, worin
* die Verknüpfungsstelle mit dem Dihydropyrazolon-Ring bedeutet und
R⁴ Wasserstoff, Fluor, Chlor, Brom, Cyano, (C₁-C₄)-Alkyl, Trifluormethyl, Hydroxymethyl, (C₁-C₄)-Alkoxy, Trifluormethoxy, Hydroxycarbonyl oder (C₁-C₄)-Alkoxycarbonyl bedeutet,
- R²: für eine Heteroaryl-Gruppe der Formel 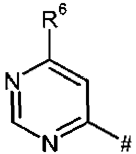 steht, worin
# die Verknüpfungsstelle mit dem Dihydropyrazolon-Ring bedeutet und
R⁶ Wasserstoff oder einen Substituenten ausgewählt aus der Reihe Fluor, Chlor, Brom, Cyano, (C₁-C₆-Alkyl, Trifluormethyl, Hydroxy, (C₁-C₆)-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Heterocycloalkyl, Phenyl und 5- oder 6-gliedriges Heteroaryl bedeutet, wobei
(C₁-C₆)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können, und
- R³: für Wasserstoff steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die erfindungsgemäßen 1,2-Dihydropyrazol-3-on-Derivate der Formel (I) können auch in der tautomeren 1*H*-Pyrazol-5-ol-Form (I') vorliegen (siehe nachfolgendes Schema 1); beide tautomeren Formen werden von der vorliegenden Erfindung ausdrücklich umfasst.

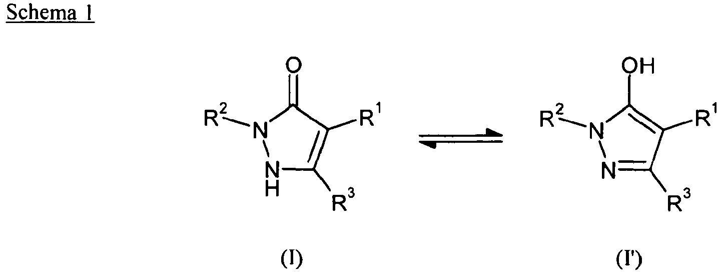

Weiterer Gegenstand der Erfindung ist ein Verfahren zur Herstellung der erfindungsgemäßen Verbindungen der Formel (I), dadurch gekennzeichnet, dass man eine Verbindung der Formel (II) 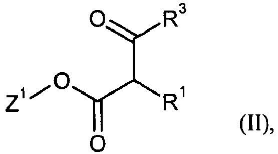 in welcher R¹ und R³ die oben angegebenen Bedeutungen aufweisen und
- Z¹: für Methyl oder Ethyl steht,
in einem inerten Lösungsmittel gegebenenfalls in Gegenwart einer Säure mit einer Verbindung der Formel (III) 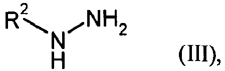 in welcher R² die oben angegebene Bedeutung aufweist,
zu Verbindungen der Formel (IV) 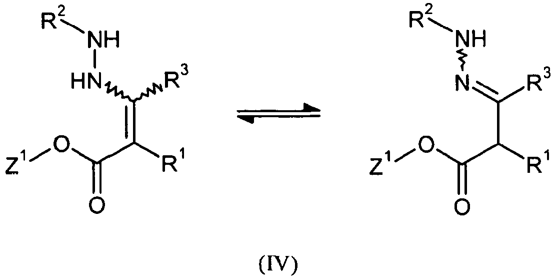 in welcher Z¹, R¹, R² und R³ die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I) cyclisieren,
und die Verbindungen der Formel (I) gegebenenfalls mit den entsprechenden (i) Lösungsmitteln und/oder (ii) Basen oder Säuren in ihre Solvate, Salze und/oder Solvate der Salze überführt.

Die erfindungsgemäßen Verbindungen der Formel (I), in welcher R³ Wasserstoff bedeutet, können auch dadurch hergestellt werden, dass man zunächst eine Verbindung der Formel (V) 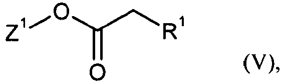 in welcher Z¹ und R¹ die oben angegebenen Bedeutungen aufweisen,
mit einer Verbindung der Formel (VI) 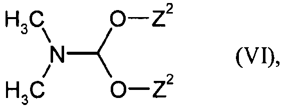 in welcher
- Z²: für Methyl oder Ethyl steht,
zu Verbindungen der Formel (VII) 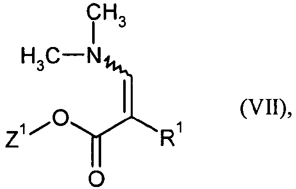 in welcher Z¹ und R¹ die oben angegebenen Bedeutungen aufweisen,
kondensiert und anschließend in Gegenwart einer Säure mit einer Verbindung der Formel (III) zu Verbindungen der Formel (IV-A) 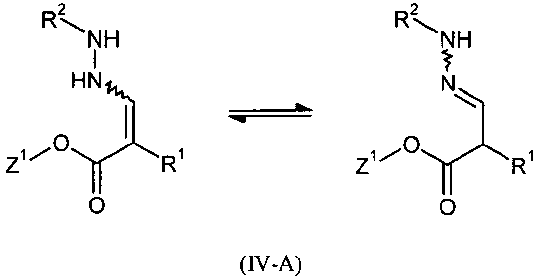 in welcher Z¹, R¹ und R² die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I), worin R³ für Wasserstoff steht, cyclisieren.

Weitere erfindungsgemäße Verbindungen können gegebenenfalls auch hergestellt werden durch Umwandlungen von funktionellen Gruppen einzelner Substituenten, insbesondere den unter R¹ und R² aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile oder elektrophile Substitution, Oxidation, Reduktion, Hydrierung, Übergangsmetall-katalysierte Kupplungsreaktionen, Alkylierung, Acylierung, Aminierung, Veresterung, Esterspaltung, Veretherung, Etherspaltung, Bildung von Carbonamiden, Sulfonamiden, Carbamaten und Harnstoffen, sowie die Einführung und Entfernung temporärer Schutzgruppen.

Als inerte Lösungsmittel für die Verfahrensschritte (II) + (III) → (IV), (IV) → (I), (VII) + (III) → (IV-A) und (IV-A) → (I) eignen sich insbesondere Ether wie Diethylether, Methyl-tert.-butylether, 1,2-Dimethoxyethan, Tetrahydrofuran und Dioxan, oder Alkohole wie Methanol, Ethanol, n-Propanol, iso-Propanol, n-Butanol und tert.-Butanol. Bevorzugt werden Methanol, Ethanol, Tetrahydrofuran oder Gemische dieser Lösungsmittel eingesetzt.

Der Verfahrensschritt (V) + (VI) → (VII) wird bevorzugt in Dimethylformamid als Lösungsmittel oder auch in Gegenwart eines Überschusses an (VI) ohne weiteres Lösungsmittel durchgeführt. Gegebenenfalls kann die Reaktion auch vorteilhaft unter Mikrowellen-Bestrahlung erfolgen. Die Umsetzung geschieht im Allgemeinen in einem Temperaturbereich von +20°C bis +150°C, bevorzugt bei +80°C bis +120°C [vgl. auch J.P. Bazureau et al., *Synthesis* 1998, 967; *ibid.* 2001 (4), 581].

Die Verfahrensschritte (II) + (III) → (IV) und (VII) + (III) → (IV-A) können gegebenenfalls vorteilhaft unter Zusatz einer Säure durchgeführt werden. Hierfür eignen sich übliche anorganische oder organische Säuren wie beispielsweise Chlorwasserstoff, Essigsäure, Trifluoressigsäure, Methansulfonsäure, p-Toluolsulfonsäure oder Campher-10-sulfonsäure. Bevorzugt werden Essigsäure oder insbesondere Campher-10-sulfonsäure oder p-Toluolsulfonsäure verwendet.

Die Umsetzung (II) + (III) → (IV) erfolgt im Allgemeinen in einem Temperaturbereich von 0°C bis +100°C, bevorzugt bei +10°C bis +50°C. Die Reaktion (VII) + (III) → (IV-A) wird im Allgemeinen in einem Temperaturbereich von +20°C bis +120°C, bevorzugt bei +50°C bis +100°C durchgeführt.

Die Verfahrenssequenzen (II) + (III) → (IV) → (I) und (VII) + (III) → (IV-A) → (I) können unter zweistufiger Reaktionsführung oder auch als Eintopf-Reaktion, ohne Isolierung der Zwischenstufe (IV) bzw. (IV-A), durchgeführt werden. Für letztere Variante ist insbesondere die Umsetzung der Komponenten unter Mikrowellen-Bestrahlung geeignet; die Reaktion erfolgt hierbei im Allgemeinen in einem Temperaturbereich von +50°C bis +200°C, bevorzugt bei +100°C bis +180°C.

Teilweise tritt ein Ringschluss zu (I) auch bereits schon bei der Herstellung von (IV) bzw. (IV-A) ein; die Cyclisierung kann dann gegebenenfalls durch *in situ*-Behandlung des Reaktionsgemisches mit einer Base vervollständigt werden.

Als Base für einen solchen separaten Cyclisierungsschritt (IV) → (I) bzw. (IV-A) → (I) eignen sich übliche anorganische oder organische Basen. Hierzu gehören insbesondere Alkalihydroxide wie beispielsweise Natrium- oder Kaliumhydroxid, Alkali- oder Erdalkalicarbonate wie Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, oder Alkalihydride wie Natriumhydrid. Bevorzugt werden Natriummethanolat oder -ethanolat verwendet.

Die Basen-induzierte Umsetzung (IV) → (I) bzw. (IV-A) → (I) erfolgt im Allgemeinen in einem Temperaturbereich von 0°C bis +60°C, bevorzugt bei 0°C bis +30°C.

Alle Verfahrensschritte können bei normalem, erhöhtem oder bei erniedrigtem Druck durchgeführt werden (z.B. von 0.5 bis 5 bar). Im Allgemeinen arbeitet man bei Normaldruck.

Die Verbindungen der Formel (II) können nach literaturüblichen Methoden zur C-Acylierung von Carbonsäureestern aus Verbindungen der Formel (V) hergestellt werden. Die Verbindungen der Formeln (III), (V) und (VI) sind kommerziell erhältlich, literaturbekannt oder können in Analogie zu in der Literatur beschriebenen Verfahren hergestellt werden.

Die Herstellung der erfindungsgemäßen Verbindungen kann durch das folgende Reaktionsschema 2 veranschaulicht werden:

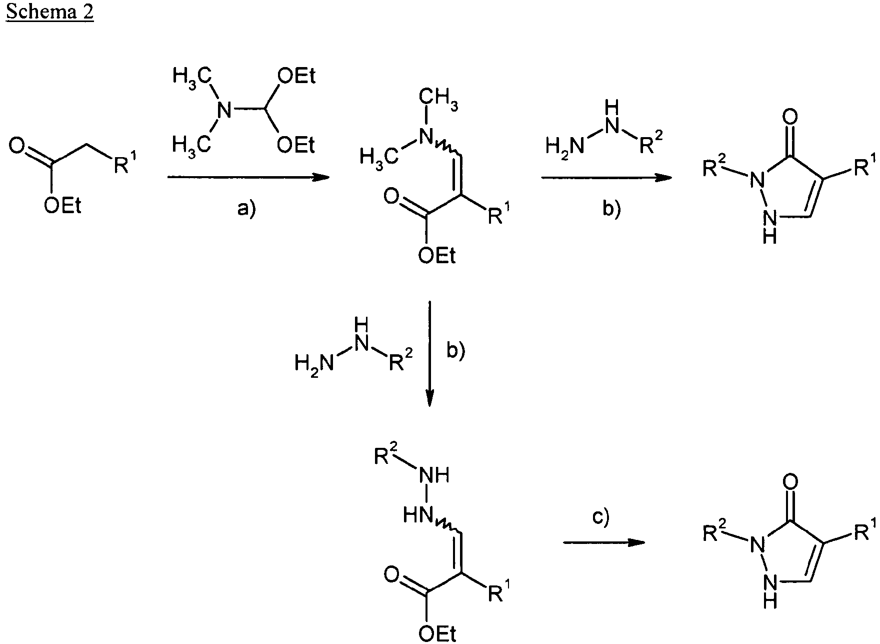

[a): DMF, 16 h, +100°C; b): Ethanol, cat. Campher-10-sulfonsäure, +78°C; c): NaOEt, Ethanol, 1 h, RT].

Die erfindungsgemäßen Verbindungen zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen zeichnen sich als spezifische Hemmstoffe der HIF-Prolyl-4-Hydroxylasen aus.

Die erfindungsgemäßen Verbindungen können aufgrund ihrer pharmakologischen Eigenschaften zur Behandlung und/oder Prophylaxe von kardiovaskulären Krankheiten, insbesondere von Herzinsuffizienz, koronarer Herzkrankheit, Angina pectoris, Myokardinfarkt, Schlaganfall, Arteriosklerose, essentieller, pulmonaler und maligner Hypertonie sowie peripherer arterieller Verschlusskrankheit eingesetzt werden.

Die erfindungsgemäßen Verbindungen eignen sich weiterhin zur Behandlung und/oder Prophylaxe von Störungen der Blutbildung, wie z.B. idiopathischen Anämien, renaler Anämie und Anämien in Begleitung einer Tumor-Erkrankung (insbesondere einer Chemotherapie-induzierten Anämie), einer Infektion (insbesondere HIV-Infektion) oder einer anderen entzündlichen Erkrankung wie z.B. rheumatoider Arthritis. Die erfindungsgemäßen Verbindungen sind darüber hinaus geeignet zur unterstützenden Behandlung von Anämien infolge Blutverlust, Eisenmangel-Anämie, Vitaminmangel-Anämie (z.B. infolge Vitamin B12-Mangel oder infolge Folsäure-Mangel), hypoplastischer und aplastischer Anämie, hämolytischer Anämie oder zur unterstützenden Behandlung von Anämien in Folge von Eisenverwertungsstörungen (sideroachrestische Anämie) oder Anämien infolge anderer endokriner Störungen (z.B. Hypothyreose).

Die Verbindungen sind ferner geeignet zur Steigerung des Hämatokrits mit dem Ziel der Gewinnung von Blut zur Eigenblutspende vor Operationen.

Die erfindungsgemäßen Verbindungen können außerdem zur Behandlung und/oder Prophylaxe von operationsbedingten Ischämiezuständen und deren Folgeerscheinungen nach chirurgischen Eingriffen, insbesondere Eingriffen am Herzen unter Verwendung einer Herz-Lungenmaschine (z.B. Bypass-Operationen, Herzklappen-Implantationen), Eingriffen an den Halsschlagadern, Eingriffen an der Körperschlagader (Aorta) und Eingriffen mit instrumenteller Öffnung oder Durchdringung der Schädelkalotte verwendet werden. Die Verbindungen eignen sich ferner zur allgemeinen Behandlung und/oder Prophylaxe bei chirurgischen Eingriffen mit dem Ziel einer Beschleunigung der Wundheilung und Verkürzung der Rekonvaleszenzzeit.

Die Verbindungen sind darüber hinaus zur Behandlung und Prophylaxe von Folgeerscheinungen akuter und protrahierter Ischämiezustände des Gehirns geeignet (z.B. Schlaganfall, Geburtsasphyxie).

Die Verbindungen können ferner zur Behandlung und/oder Prophylaxe von Krebs und zur Behandlung und/oder Prophylaxe einer im Zuge der Behandlung von Krebs auftretenden Beeinträchtigung des Gesundheitszustandes insbesondere nach Therapie mit Zytostatika, Antibiotika und Bestrahlungen eingesetzt werden.

Die Verbindungen eignen sich ferner zur Behandlung und/oder Prophylaxe von Erkrankungen des rheumatischen Formenkreises und anderen den Autoimmunerkrankungen zuzurechnenden Krankheitsformen und insbesondere zur Behandlung und/oder Prophylaxe einer im Zuge der medikamentösen Behandlung derartiger Erkrankungen auftretenden Beeinträchtigung des Gesundheitszustandes.

Weiterhin können die erfindungsgemäßen Verbindungen eingesetzt werden zur Behandlung und/oder Prophylaxe von Erkrankungen des Auges (z.B. Glaukom), des Gehirns (z.B. Morbus Parkinson, Morbus Alzheimer, Demenz, chronisches Schmerzempfinden), von chronischen Nierenerkrankungen, Niereninsuffizienz und akutem Nierenversagen sowie zur Förderung der Wundheilung.

Weiterhin eignen sich die Verbindungen zur Behandlung und/oder Prophylaxe einer insbesondere im höheren Lebensalter gehäuft auftretenden allgemeinen körperlichen Schwäche bis hin zur Kachexie.

Ferner eignen sich die Verbindungen zur Behandlung und/oder Prophylaxe sexueller Dysfunktion.

Außerdem sind die Verbindungen zur Behandlung und/oder Prophylaxe von Diabetes mellitus und seinen Folgeerkrankungen, wie z.B. diabetischer Makro- und Mikroangiopathie, diabetischer Nephropathie und Neuropathie, geeignet.

Weiterhin eignen sich die erfindungsgemäßen Verbindungen zur Behandlung und/oder Prophylaxe von fibrotischen Erkrankungen z.B. des Herzens, der Lunge und der Leber.

Insbesondere sind die erfindungsgemäßen Verbindungen auch zur Prophylaxe und Behandlung der Frühgeborenenretinopathie (Retinopathia praematurorum) geeignet.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt: ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika sowie Antibiotika.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Phosphodiesterase (PDE)-Inhibitor, wie beispielhaft und vorzugsweise Milrinone, Amrinone, Pimobendan, Cilostazol, Sildenafil, Vardenafil oder Tadalafil, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Tumor-Chemotherapeutikum verabreicht, beispielhaft und vorzugsweise aus der Gruppe der Platin-Komplexe, wie z.B. Cisplatin und Carboplatin, der Alkylantien, wie z.B. Cyclophosphamid und Chlorambucil, der Antimetabolite, wie z.B. 5-Fluoruracil und Methotrexat, der Topoisomerase-Hemmer, wie z.B. Etoposid und Camptothecin, der Antibiotika, wie z.B. Doxorubicin und Daunorubicin, oder der Kinase-Inhibitoren, wie z.B. Sorafenib und Sunitinib.

Bei einer bevorzugten Ausführungsform der Erfindung werden die erfindungsgemäßen Verbindungen in Kombination mit einem Antibiotikum verabreicht, beispielhaft und vorzugsweise aus der Gruppe der Penicilline, Cephalosporine oder Chinolone, wie z.B. Ciprofloxacin und Moxifloxacin.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- cat.: katalytisch
- d: Tag(e)
- DCI: direkte chemische Ionisation (bei MS)
- DMF: Dimethylformamid
- DMSO: Dimethylsulfoxid
- d. Th.: der Theorie (bei Ausbeute)
- EI: Elektronenstoß-Ionisation (bei MS)
- ESI: Elektrospray-Ionisation (bei MS)
- Et: Ethyl
- GC-MS: Gaschromatographie-gekoppelte Massenspektroskopie
- h: Stunde(n)
- HPLC: Hochdruck-, Hochleistungsflüssigchromatographie
- konz.: konzentriert
- LC-MS: Flüssigchromatographie-gekoppelte Massenspektroskopie
- Meth.: Methode
- min: Minute(n)
- MS: Massenspektroskopie
- NMR: Kernresonanzspektroskopie
- Rₜ: Retentionszeit (bei HPLC)
- RT: Raumtemperatur
- TFA: Trifluoressigsäure
- THF: Tetrahydrofuran

### LC-MS-, GC-MS- und HPLC-Methoden:

### Methode 1 (LC-MS):

Instrument: Micromass Platform LCZ mit HPLC Agilent Serie 1100; Säule: Thermo Hypersil GOLD 3 p, 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 2 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 6 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.1 min 100% A; Fluss: 2.5 ml/min; Ofen: 55°C; UV-Detektion: 210 nm.

### Methode 7 (LC-MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 8 (LC-MS):

Instrument: Micromass Quattro Micro MS mit HPLC Agilent Serie 1100; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 9 (LC-MS):

Instrument: Micromass Quattro LCZ mit HPLC Agilent Serie 1100; Säule: Phenomenex Synergi 2.5µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208-400 nm.

### Methode 10 (LC-MS):

Instrument: Micromass QuattroPremier mit Waters UPLC Acquity; Säule: Thermo Hypersil GOLD 1.9µ 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 11 (HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 12 (HPLC):

Säule: Kromasil 100 C18 5 µm, 250 mm x 20 mm; Eluent A: 0.2%-ige Trifluoressigsäure, Eluent B: Acetonitril; Gradient: 0.0 min 95% A → 10 min 5% A → 15 min 5% A → 15.1 min 95% A → 20 min 95% A; Ofen: 30°C; Fluss: 25 ml/min; UV-Detektion: 240 nm.

### Methode 13 (LC-MS):

Instrument: Micromass Quattro LCZ mit HPLC Agilent Serie 1100; Säule: Phenomenex Onyx Monolithic C18, 100 mm x 3 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 14 (GC-MS):

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 15 (präparative HPLC):

Säule: Chromatorex C18 5 µm, 250 mm x 20 mm; Eluent A: wässrige 0.1%-ige Diisopropylethylamin-Lösung, Eluent B: Acetonitril; Gradient: 0.0 min 60% A → 4 min 60% A; Ofen: 30°C; Fluss: 25 ml/min; UV-Detektion: 260 nm.

### Methode 16 (präparative LC-MS):

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Methode 17 (präparative HPLC):

Säule: Kromasil 100 C18 5 µm, 250 mm x 20 mm; Eluent A: wässrige 0.1%-ige Diisopropylethylamin-Lösung, Eluent B: Acetonitril; Gradient: 0.0 min 95% A → 10 min 65% A → 10.1 min 95% A → 15 min 95% A; Ofen: 40°C; Fluss: 25 ml/min; UV-Detektion: 210 nm.

### Ausgangsverbindungen und Intermediate:

### Beispiel 1A

### 2-Hydrazino-4-methylpyridin

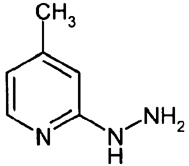

3.33 g (30.0 mmol) 2-Fluor-4-methylpyridin werden in 40 ml 2-Ethoxyethanol vorgelegt, die Lösung mit 14.6 ml (15.0 g, 300 mmol) Hydrazinhydrat versetzt und der Ansatz in der Siedehitze (150°C Badtemperatur) 16 h lang gerührt. Die Reaktionslösung wird danach am Rotationsverdampfer eingeengt, der Rückstand auf 100 ml Wasser gegeben und mit Essigsäureethylester extrahiert (dreimal je 100 ml). Die vereinigten organischen Phasen werden über Natriumsulfat getrocknet, filtriert und eingeengt. Der erhaltene Rückstand wird im Vakuum getrocknet.
Ausbeute: 1.90 g (51% d. Th.)
¹H-NMR (400 MHz, DMSO-d₆): δ = 7.83 (d, 1H), 7.22 (s, 1H), 6.51 (s, 1H), 6.38 (d, 1H), 4.04 (s, 2H), 2.17 (s, 3H).
LC-MS (Methode 1): Rₜ = 0.80 min; MS (ESIpos): m/z = 124 [M+H]⁺.

### Beispiel 2A

### 3-(Dimethylamino)-2-[5-(trifluormethyl)-1,3,4-thiadiazol-2-yl]acrylsäuremethylester

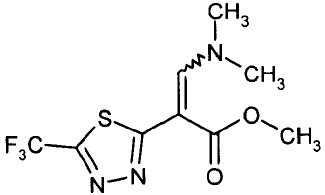

3.05 g (13.5 mmol) 5-(Trifluormethyl)-1,3,4-thiadiazol-2-ylessigsäuremethylester [Herstellung siehe DE 42 40 168-A1] werden in 6.9 ml (40.5 mmol) Dimethylformamid-diethylacetal über Nacht bei 100°C erhitzt. Nach dem Abkühlen engt man ein und reinigt den Rückstand mittels präparativer HPLC (RP18-Säule; Laufmittel: Acetonitril/Wasser-Gradient).
Ausbeute: 2.8 g (74% d. Th.)
¹H-NMR (400 MHz, DMSO-d₆): δ = 8.28 (s, 1H), 3.74 (s, 3H), 3.32 (s, 6H).
LC-MS (Methode 4): Rₜ = 1.88 min; MS (ESIpos): m/z = 282 [M+H]⁺.

### Beispiel 3A

### 3-(Dimethylamino)-2-(1H-1,2,3-triazol-1-yl)acrylsäureethylester

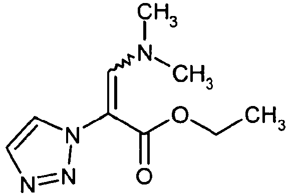

Die Darstellung der Titelverbindung erfolgt analog zu Beispiel 2A ausgehend von 1.00 g (6.45 mmol) 2-(1*H*-1,2,3-Triazol-1-yl)essigsäureethylester.
Ausbeute: 1.4 g (100% d. Th.)
¹H-NMR (400 MHz, DMSO-d₆): δ = 8.10 (d, 1H), 7.78 (d, 1H), 7.65 (s, 1H), 4.03 (q, 2H), 3.06 (br. s, 3H), 2.10 (br. s, 3H), 1.12 (t, 3H).
LC-MS (Methode 5): Rₜ = 1.40 min; MS (ESIpos): m/z = 211 [M+H]⁺.

### Beispiel 4A

### (6-Hydrazinopyridin-3-yl)methanol

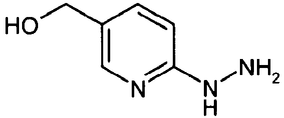

220.0 g (1.5 mol) (6-Chlorpyridin-3-yl)methanol [Evans et al., *Organic Letters* **2001,** *19*, 3009-3012] werden in 746 ml (767.1 g, 15.3 mol) Hydrazinhydrat vorgelegt und bei einer Badtemperatur von 150°C 5 h lang gerührt. Man konzentriert die Reaktionsmischung im Vakuum auf, nimmt den Rückstand mit 500 ml Wasser auf und gibt 86.0 g (1.5 mol) Kaliumhydroxid hinzu. Es wird 15 min gerührt, das Wasser dann am Rotationsverdampfer fast vollständig entfernt und die Wasserreste mehrmals mit Toluol azeotrop abdestilliert. Der ölige Rückstand wird in Ethanol verrührt, auf ca. 10°C gekühlt, das ausfallende Kaliumchlorid abfiltriert, das Filtrat eingeengt und der Rückstand mit Diethylether versetzt und verrührt. Abschließend wird das Produkt abfiltriert, der Filterrückstand mit Diethylether gewaschen und die Kristalle im Vakuum getrocknet.
Ausbeute: 149.0 g (68% d. Th.)
LC-MS (Methode 1): Rₜ = 0.46 min; MS (ESIpos): m/z = 140 [M+H]⁺;
¹H-NMR (400 MHz, DMSO-d₆): δ = 7.91 (d, 1H), 7.40 (dd, 1H), 7.29 (s, 1H), 6.66 (d, 1H), 4.94 (br. s, 1H), 4.34-4.28 (m, 2H), 4.04 (br. s, 2H).

### Beispiel 5A

### 1-(6-Hydrazinopyridin-3-yl)-N-methylmethanamin

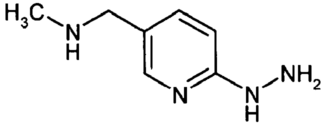

1.0 g (6.4 mmol) 1-(6-Chlorpyridin-3-yl)-*N*-methylmethanamin [Herstellung siehe EP 0 556 684-A1] werden in 1.5 ml (1.6 g, 31.9 mmol) Hydrazinhydrat vorgelegt und 12 h in der Siedehitze bei einer Badtemperatur von 150°C gerührt. Die abgekühlte Reaktionslösung wird eingeengt und der Rückstand im Vakuum getrocknet. Man erhält 1.1 g der Titelverbindung, welche ohne weitere Reinigung eingesetzt wird.
LC-MS (Methode 1): Rₜ = 0.52 min; MS (ESIpos): m/z = 153 [M+H]⁺.

### Beispiel 6A

### 6-Hydrazinonicotinsäure

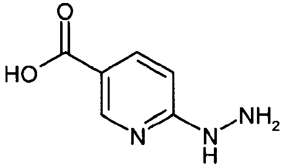

5.0 g (31.7 mmol) 6-Chlornicotinsäure und 30.9 ml (31.8 g, 634.7 mmol) Hydrazinhydrat werden in 10 ml Ethanol vorgelegt und 16h in der Siedehitze bei 100°C Badtemperatur gerührt. Das Lösungsmittel und überschüssiges Hydrazinhydrat werden am Rotationsverdampfer abdestilliert, der Rückstand in Wasser aufgenommen, anschließend mit 1.8 g (31.7 mmol) Kaliumhydroxid versetzt und 15 min gerührt. Man entfernt das Lösungsmittel vollständig am Rotationsverdampfer, trocknet den Rückstand im Vakuum und erhält 7.5 g Rohprodukt, das als solches weiter umgesetzt wird.
LC-MS (Methode 1): Rₜ = 0.48 min; MS (ESIpos): m/z = 154 [M+H]⁺;
¹H-NMR (400 MHz, DMSO-d₆): δ = 8.49 (d, 1H), 7.89 (br. s, 1H), 7.84 (dd, 1H), 6.63 (d, 1H), 5.37 (br. s, 2H).

### Beispiel 7A

### 4-Chlor-6-hydrazinopyrimidin

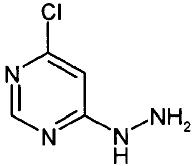

In eine Lösung von 20.0 g (134.3 mmol) 4,6-Dichlorpyrimidin in 300 ml Ethanol werden unter Rühren 11.8 ml (12.1 g, 241.6 mmol) Hydrazinhydrat bei RT zugetropft. Tritt eine Trübung der Lösung während der Zudosierung des Hydrazinhydrats auf, gibt man weiteres Ethanol (ca. 400 ml) hinzu. Die Reaktionslösung wird 12 h bei RT nachgerührt. Zur Aufarbeitung wird der ausgefallene Feststoff abfiltriert, der Filterrückstand zweimal mit je 150 ml Wasser und zweimal mit je 100 ml Diethylether gewaschen und das Produkt im Vakuum getrocknet. Aus der eingeengten Mutterlauge wird eine weitere kristalline Produktfraktion erhalten.
Ausbeute: 16.8 g (87% d. Th.)
LC-MS (Methode 1): Rₜ = 1.17 min; MS (ESIpos): m/z = 145 [M+H]⁺;
¹H-NMR (400 MHz, DMSO-d₆): δ = 8.81 (s, 1H), 8.17 (br. s, 1 H), 6.75 (s, 1 H), 4.48 (br. s, 2H).

### Beispiel 8A

### 4-Hydrazino-6-piperidin-1-ylpyrimidin

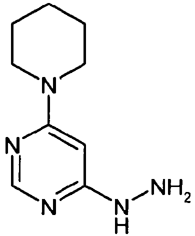

### Stufe a): 4-Chlor-6-piperidin-1-ylpyrimidin

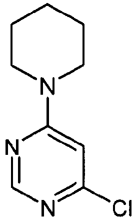

Ein Gemisch aus 10.0 g (67.1 mmol) 4,6-Dichlorpyrimidin und 5.7 g (67.1 mmol) Piperidin in 100 ml Wasser wird 16 h bei einer Badtemperatur von 115°C gerührt. Nach Abkühlen auf RT wird der Niederschlag abfiltriert, mit Wasser gewaschen und im Vakuum getrocknet.
Ausbeute: 6.4 g (47% d. Th.)
LC-MS (Methode 4): Rₜ = 2.16 min; MS (ESIpos): m/z = 198 [M+H]⁺;
¹H-NMR (400 MHz, DMSO-d₆): δ = 8.29 (s, 1H), 6.92 (s, 1H), 3.65-3.58 (m, 4H), 1.66-1.62 (m, 2H), 1.60-1.48 (m, 4H).

### Stufe b): 4-Hydrazino-6-piperidin-1-ylpyrimidin

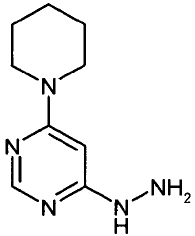

In eine Lösung von 6.0 g (30.4 mmol) 4-Chlor-6-piperidin-1-ylpyrimidin in 50 ml Ethanol werden unter Rühren 17.7 ml (18.2 g, 364.2 mmol) Hydrazinhydrat bei RT zugetropft. Die Reaktionslösung wird 16h bei 80°C nachgerührt. Zur Aufarbeitung wird im Vakuum eingeengt, der Rückstand in Wasser verrührt, der ausgefallene Feststoff abfiltriert, der Filterrückstand zweimal mit je 150 ml Wasser und zweimal mit je 100 ml Diethylether gewaschen und das Produkt im Vakuum getrocknet.
Ausbeute: 4.0 g (69% d. Th.)
LC-MS (Methode 1): Rₜ = 2.06 min; MS (ESIpos): m/z = 194 [M+H]⁺;
¹H-NMR (400 MHz, DMSO-d₆): δ = 7.91 (s, 1H), 7.54 (br. s, 1H), 5.89 (s, 1H), 4.11 (br. s, 2H), 3.50-3.47 (m, 4H), 1.61-1.58 (m, 2H), 1.51-1.46 (m, 4H).

### Beispiele 9A

### 2-Hydrazino-5-(methylsulfonyl)pyridin

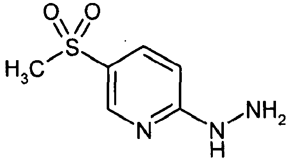

2.0 g (8.5 mmol) 2,5-Bis(methylsulfonyl)pyridin [Woods et al., J. Heterocycl. Chem. 1984, 21, 97-101] werden in 15 ml Ethanol mit 1.7 ml (1.7 g, 34.0 mmol) Hydrazinhydrat versetzt und 4 h unter Rückfluss gerührt. Zur Aufarbeitung wird die Reaktionslösung auf 15°C abgekühlt, der ausgefallene Feststoff abfiltriert, der Filterrückstand mit Ethanol und Diethylether gewaschen und das Produkt im Vakuum getrocknet.
Ausbeute: 1.4 g (89% d. Th.)
LC-MS (Methode 1): Rₜ = 0.51 min; MS (ESIpos): m/z = 188 [M+H]⁺;
¹H-NMR (400 MHz, DMSO-d₆): δ = 8.56 (s, 1H), 8.38 (d, 1H), 7.81 (dd, 1H), 6.79 (d, 1H), 4.42 (s, 2H), 3.11 (s, 3H).

### Beispiel 10A

### 5-Brom-2-hydrazinopyridin

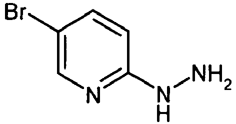

Eine Lösung von 1.8 g (9.5 mmol) 5-Brom-2-chlorpyridin in 25 ml Ethanol wird bei RT unter Rühren mit 9.3 ml (9.5 g, 190.2 mmol) Hydrazinhydrat versetzt und dann 46 h bei 90°C gerührt. Nach dem Einengen des Reaktionsgemisches im Vakuum wird der Rückstand in Wasser verrührt, der Feststoff abfiltriert, mit Wasser und Diethylether gewaschen und im Vakuum getrocknet.
Ausbeute: 0.8 g (44% d. Th.)
LC-MS (Methode 8): Rₜ = 0.50 min; MS (ESIpos): m/z = 188 [M+H]⁺;
¹H-NMR (400 MHz, DMSO-d₆): δ = 8.02 (d, 1H), 7.66 (s, 1H), 7.58 (dd, 1H), 6.69 (d, 1H), 4.16 (s, 2H).

### Beispiel 11A

### 1-(6-Hydrazinopyrimidin-4-yl)azetidin-3-ol

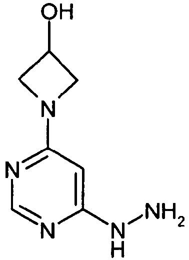

### Stufe a): 1-(6-Chlorpyrimidin-4-yl)azetidin-3-ol

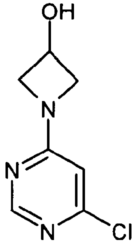

auf 90°C (wobei das 2,4-Dichlorpyrimidin erkennbar flüchtig ist und sich kristallin am Kühler niederschlägt). Man entfernt das Lösungsmittel im Vakuum, trocknet den Rückstand und erhält 10.4 g des Rohprodukts, welches ohne weitere Aufreinigung umgesetzt wird.
LC-MS (Methode 10): R, = 0.36 min; MS (ESIpos): m/z = 186 [M+H]⁺;
¹H-NMR (400 MHz, DMSO-d₆): δ = 8.29 (s, 1H), 6.50 (s, 1H), 4.63-4.57 (m, 1H), 4.26 (t, 2H), 3.82-3.75 (m, 2H).

### Stufe b): 1-(6-Hydrazinopyrimidin-4-yl)azetidin-3-ol

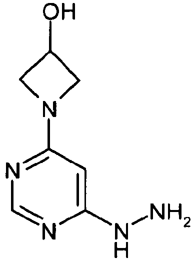

960 mg (5.2 mmol) 1-(6-Chlorpyrimidin-4-yl)azetidin-3-ol und 2.5 ml (51.7 mmol) Hydrazinhydrat werden in einem Gemisch aus 10 ml Ethanol und 10 ml THF vorgelegt. Man setzt 1 h bei 130°C in einer *single* mode-Mikrowelle (CEM Explorer) um. Man konzentriert das Gemisch auf ca. 20-50% des ursprünglichen Flüssigkeitsvolumens auf und lässt 48 h bei RT stehen. Der Überstand wird vom gebildeten Feststoff abdekantiert und der Feststoff dreimal mit jeweils 1.5 ml kaltem Ethanol gewaschen. Man trocknet im Hochvakuum.
Ausbeute: 300 mg (32% d. Th.)
LC-MS (Methode 8): Rₜ = 0.23 min; MS (ESIpos): m/z = 182 [M+H]⁺;
¹H-NMR (400 MHz, DMSO-d₆): δ = 7.88 (s, 1H), 7.63 (s, 1H), 5.68 (br. s, 1H), 5.52 (s, 1H), 4.55 (br. s, 1H), 4.18-4.06 (m, 4H), 3.65-3.60 (m, 2H).

### Beispiel 12A

### 5-(2,2-Dimethylpropoxy)-2-hydrazinopyridin

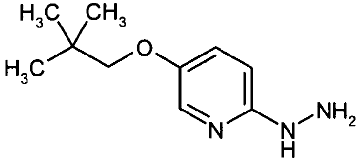

### Stufe a): 2-Chlor-5-(2,2-dimethylpropoxy)pyridin

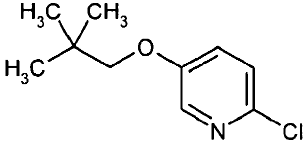

5.2 g (40.0 mmol) 6-Chlorpyridin-3-ol, 11.9 g (60.0 mmol) 1-Iod-2,2-dimethylpropan, 19.6 g (60.0 mmol) Cäsiumcarbonat und 120 ml Diethylenglykoldimethylether werden auf fünf jeweils gleich große Portionen aufgeteilt und portionsweise in einer *single* mode-Mikrowelle (CEM Explorer) bei 160°C für 4 h umgesetzt. Man vereinigt danach die erhaltenen fünf Reaktionsgemische, filtriert den Feststoff ab, wäscht den Feststoff mit Diethylenglykoldimethylether nach und vereinigt Filtrat und Waschlösungen. Der Großteil des Lösungsmittels wird entfernt und die aufkonzentrierte Lösung (ca. 50 ml) mit 300 ml Wasser versetzt. Man verrührt 30 min, filtriert den erhaltenen Feststoff ab, wäscht einmal mit Wasser und trocknet im Hochvakuum.
Ausbeute: 7.0 g (88% d. Th.)
LC-MS (Methode 8): Rₜ = 2.47 min; MS (ESIpos): m/z = 200 [M+H]⁺;
¹H-NMR (400 MHz, CDCl₃): δ = 8.05 (d, 1H), 7.25-7.15 (m, 2H), 3.61 (s, 2H), 1.03 (s, 9H).

### Stufe b): 5-(2,2-Dimethylpropoxy)-2-hydrazinopyridin

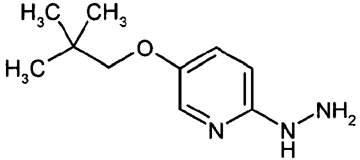

6.2 g (30.8 mmol) 2-Chlor-5-(2,2-dimethylpropoxy)pyridin werden zusammen mit 60 ml (1.2 mol) Hydrazinhydrat auf vier jeweils gleich große Portionen aufgeteilt und mit jeweils 10 ml Ethanol versetzt. Jede Portion wird in einer *single mode*-Mikrowelle (CEM Explorer) bei 170°C (200 Watt) für jeweils 12 h umgesetzt. Man vereinigt danach die vier Gemische und entfernt das Lösungsmittel. Der Rückstand wird in Essigsäureethylester aufgenommen und je einmal mit gesättigter Natriumhydrogencarbonat-Lösung und gesättigter Natriumchlorid-Lösung gewaschen. Man trocknet über Magnesiumsulfat und entfernt das Lösungsmittel im Vakuum.
Ausbeute: 6.0 g (76% d. Th.)
LC-MS (Methode 8): Rₜ = 1.28 min; MS (ESIpos): m/z = 196 [M+H]⁺;
¹H-NMR (400 MHz, CDCl₃): δ = 7.84 (s, 1H), 7.17 (dd, 1H), 6.68 (d, 1H), 5.54 (br. s, 1H), 3.80 (br. s, 2H), 3.56 (s, 2H), 1.02 (s, 9H).

### Beispiel 13A

### 2-Chlor-5-(methoxymethyl)pyridin

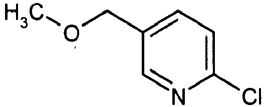

2.6 g (23.0 mmol) Kalium-tert.-butylat werden in 50 ml THF gelöst. Man gibt 3.0 g (20.9 mmol) (6-Chlorpyridin-3-yl)methanol hinzu und rührt 15 min bei RT. Man gibt dann 4.4 g (31.3 mmol) Iodmethan hinzu und rührt ca. 30 min, bis die leicht exotherme Reaktion abgeklungen ist. Man entfernt das Lösungsmittel, nimmt den Rückstand in Dichlormethan auf und wäscht zweimal mit Wasser. Man trocknet über Magnesiumsulfat, engt ein und reinigt den Rückstand säulenchromatographisch an Kieselgel (Biotage-Chromatographie, Laufmittel: Cyclohexan/Essigsäureethylester 85:15).
Ausbeute: 2.2 g (68% d. Th.)
LC-MS (Methode 1): Rₜ = 2.62 min; MS (ESIpos): m/z = 158 [M+H]⁺;
¹H-NMR (400 MHz, CDCl₃): δ = 8.34 (d, 1H), 7.65 (dd, 1H), 7.32 (d, 1H), 4.45 (s, 2H), 3.41 (s, 3 H).

### Beispiel 14A

### 2-Brom-4,5-dimethylpyridin

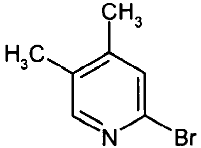

71.3 g (0.8 mol) 2-(Dimethylamino)-ethanol werden in 500 ml n-Hexan vorgelegt und auf 0°C abgekühlt. Man gibt langsam 1.0 Liter (1.6 mol) n-Butyllithium-Lösung (1.6 M in n-Hexan) hinzu und rührt 15 min bei 0°C. Man tropft dann eine Lösung von 17.9 g (166.7 mmol) 3,4-Lutidin in 500 ml n-Hexan hinzu und rührt 1 h bei 0°C. Anschließend wird auf-78°C abgekühlt und mit einer Lösung von 331.7 g (1.0 mol) Tetrabrommethan in 1.0 Liter THF versetzt. Man rührt 1 h bei -78°C nach und lässt das Reaktionsgemisch danach auf RT erwärmen. Man kühlt wieder auf 0°C ab und tropft langsam 1.5 Liter Wasser hinzu. Man trennt die Phasen, wäscht die organische Phase mit Wasser, trocknet über Magnesiumsulfat und engt im Vakuum ein. Der Rückstand wird zunächst an ca. 1 kg Kieselgel vorgereinigt (Laufmittel: Cyclohexan/Essigsäureethylester 9:1, dann 7:3). Die produkthaltigen Fraktionen werden vereinigt und im Vakuum eingeengt. Der Rückstand wird dann nochmals an Kieselgel gereinigt (Laufmittel: Cyclohexan/Essigsäureethylester 9:1). Das so erhaltene Produkt enthält ca. 10% des regioisomeren 2-Brom-3,4-dimethylpyridin.
Ausbeute: 6.7 g (20% d. Th.)
GC-MS (Methode 14): Rₜ = 4.24 min; MS (ESIpos): m/z = 187 [M+H]⁺;
¹H-NMR (400 MHz, CDCl₃): δ = 8.07 (s, 1H), 7.25 (s, 1H), 2.24 (s, 3H), 2.18 (s, 3H).

### Beispiel 15A

### tert.-Butyl[(6-chlorpyridin-3-yl)methyl]carbamat

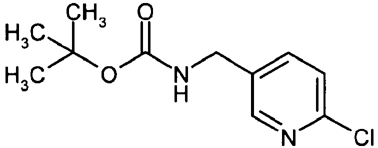

25.0 g (175.3 mmol) 5-Aminomethyl-2-chlorpyridin werden in 175 ml Dichlormethan vorgelegt. Man gibt 175 ml 10%-ige Natronlauge hinzu und tropft eine Lösung von 38.3 g (175.3 mmol) Di-tert.-butyldicarbonat in 175 ml Dichlormethan hinzu. Man rührt 16 h bei RT. Man verdünnt dann mit 175 ml Dichlormethan, trennt die organische Phase ab und extrahiert die wässrige Phase mit 175 ml Dichlormethan. Die vereinigten organischen Phasen werden über Magnesiumsulfat getrocknet und im Vakuum eingeengt. Das Produkt wird im Hochvakuum getrocknet.
Ausbeute: 42.0 g (99% d. Th.)
LC-MS (Methode 7): Rₜ = 1.58 min; MS (ESIpos): m/z = 243 [M+H]⁺;
¹H-NMR (400 MHz, CDCl₃): δ = 8.31 (d, 1H), 7.61 (dd, 1H), 7.30 (d, 1H), 4.99 (br. s, 1H), 4.30 (d, 2H), 1.46 (s, 9H).

### Beispiel 16A

### 4-(6-Hydrazinopyrimidin-4-yl)morpholin

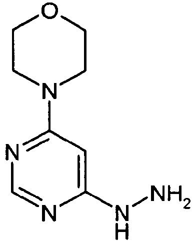

### Stufe a): 4-(6-Chlorpyrimidin-4-yl)morpholin

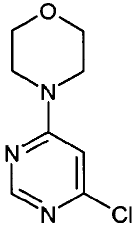

45.0 g (302.1 mmol) 4,6-Dichlorpyrimidin werden in 450 ml Wasser vorgelegt. Man gibt 26.3 g (302.1 mmol) Morpholin hinzu und rührt 16 h bei 90°C. Man kühlt danach auf 0°C ab und fltriert den entstandenen Niederschlag ab. Man wäscht den Niederschlag einmal mit 50 ml Wasser und trocknet an der Luft.
Ausbeute: 51.0 g (85% d. Th.)
LC-MS (Methode 4): Rₜ = 1.09 min; MS (ESIpos): m/z = 200 [M+H]⁺;
¹H-NMR (400 MHz, DMSO-d₆): δ = 8.35 (s, 1H), 6.95 (s, 1H), 3.62 (s, 8H).

### Stufe b): 4-(6-Hydrazinopyrimidin-4-yl)morpholin

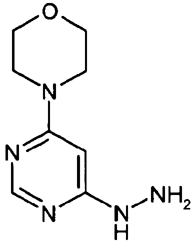

53.0 g (2.7 mol) 4-(6-Chlorpyrimidin-4-yl)morpholin werden in 260 ml Ethanol vorgelegt. Man gibt 132.9 g (2.7 mol) Hydrazinhydrat hinzu und rührt 16 h unter Rückfluss. Man kühlt danach auf RT ab und entfernt destillativ ca. die Hälfte des Lösungsmittels. Man kühlt auf 0°C ab und filtriert den entstandenen Feststoff ab. Man wäscht mit kaltem Ethanol nach und trocknet den Feststoff zunächst an der Luft und anschließend im Vakuum.
Ausbeute: 35.0 g (68% d. Th.)
LC-MS (Methode 1): Rₜ = 0.17 min; MS (ESIpos): m/z = 196 [M+H]⁺;
¹H-NMR (400 MHz, DMSO-d₆): δ = 7.94 (s, 1H), 7.70 (s, 1H), 5.91 (s, 1H), 4.15 (s, 2H), 3.66-3.60 (m, 4H), 3.45-3.37 (m, 4H).

### Beispiel 17A

### 2-Hydrazinopyrazin

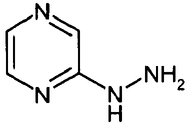

20.0 g (174.6 mmol) 2-Chlorpyrazin werden zu 60.0 ml (61.7 g, 1.2 mol) Hydrazinhydrat getropft. Man rührt 45 min bei 120°C Badtemperatur. Zur Aufarbeitung lässt man die abgekühlte Reaktionsmischung für 12 h bei 2°C stehen, filtriert die ausgefallenen Kristalle ab und wäscht den Filterrückstand zweimal mit Petrolether. Der Rückstand wird anschließend aus Toluol umkristallisiert.
Ausbeute: 6.5 g (34% d. Th.)
LC-MS (Methode 1): Rₜ = 0.49 min; MS (ESIpos): m/z = 111 [M+H]⁺;
¹H-NMR (400 MHz, DMSO-d₆): δ = 8.11 (s, 1H), 7.94 (s, 1H), 7.90 (s, 1H), 7.70 (d, 1H), 4.29 (br. s, 2H).

### Beispiel 18A

### 5-(tert.-Butoxymethyl)-2-hydrazinopyridin

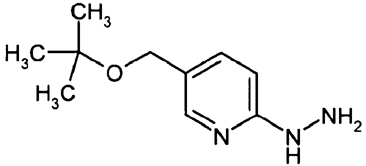

### Stufe a): 5-(tert.-Butoxymethyl)-2-chlorpyridin

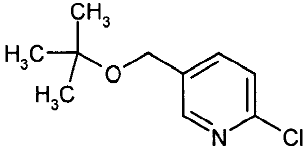

7.2 g (50.0 mmol) (6-Chlorpyridin-3-yl)methanol werden in 50 ml Dichlormethan vorgelegt. Man gibt 25.1 g (115.0 mmol) Di-tert.-butyldicarbonat und 1.2 g (5.0 mmol) Magnesiumperchlorat hinzu und rührt 24 h bei 40°C. Man kühlt dann auf RT ab, gibt weitere 12.5 g (87.1 mmol) Di-*tert*.-butyldicarbonat sowie 600 mg (2.7 mmol) Magnesiumperchlorat hinzu und rührt erneut für 2.5 h unter Rückfluss. Man gibt nochmals 12.5 g (87.1 mmol) Di-tert.-butyldicarbonat hinzu und rührt weitere 3 h unter Rückfluss. Danach verdünnt man mit Dichlormethan und wäscht einmal mit Wasser und einmal mit gesättigter Natriumchlorid-Lösung. Man trocknet über Magnesiumsulfat, engt ein und reinigt den Rückstand säulenchromatographisch an Kieselgel (Laufmittel: Cyclohexan/Essigsäureethylester 85:15).
Ausbeute: 7.9 g (79% d. Th.)
LC-MS (Methode 10): Rₜ = 1.12 min; MS (ESIpos): m/z = 200 [M+H]⁺;
¹H-NMR (400 MHz, DMSO-d₆): δ = 8.36 (d, 1H), 7.78 (dd, 1H), 6.98 (d, 1H), 4.45 (s, 2H), 1.22 (s, 9H).

### Stufe b): 5-(tert.-Butoxymethyl)-2-hydrazinopyridin

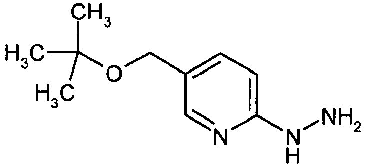

7.9 g (39.6 mmol) 5-(tert.-Butoxymethyl)-2-chlorpyridin werden zusammen mit 19.8 g (395.6 mmol) Hydrazinhydrat auf drei jeweils gleich große Portionen aufgeteilt und mit jeweils 15 ml Ethanol versetzt. Jede Portion wird in einer *single* mode-Mikrowelle (CEM Explorer) bei 170°C für jeweils 4 h umgesetzt. Man vereinigt danach die drei Gemische und entfernt das Lösungsmittel. Der Rückstand wird in Essigsäureethylester aufgenommen und einmal mit gesättigter Natriumhydrogencarbonat-Lösung gewaschen. Die wässrige Phase wird einmal mit Essigsäureethylester extrahiert. Die beiden Essigsäureethylester-Phasen werden vereinigt und einmal mit gesättigter Natriumchlorid-Lösung gewaschen. Man trocknet über Magnesiumsulfat und entfernt das Lösungsmittel. Der erhaltene Rückstand wird in Petrolether verrührt und der Feststoff abfiltriert.
Ausbeute: 1.6 g (21% d. Th.)
LC-MS (Methode 10): Rₜ = 0.77 min; MS (ESIpos): m/z = 196 [M+H]⁺;
¹H-NMR (400 MHz, CDCl₃): δ = 8.08 (s, 1H), 7.50 (dd, 1H), 6.68 (d, 1H), 5.77 (br. s, 1H), 4.32 (s, 2H), 3.80 (br. s, 2H), 1.28 (s, 9H).

### Beispiel 19A

### 6-Hydrazinopyridin-3-carbonitril

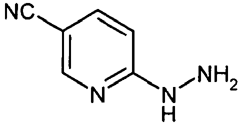

2.0 g (14.4 mmol) 6-Chlornicotinsäurenitril werden in 7.0 ml (7.3 g, 144.4 mmol) Hydrazinhydrat 15 min lang bei 100°C Badtemperatur gerührt. Die auf RT abgekühlte Reaktionsmischung wird mit Wasser verdünnt und 30 min bei RT gerührt. Man filtriert den ausgefallenen Niederschlag ab, wäscht den Filterrückstand mit Wasser, trocknet die Kristalle über Nacht an der Luft und kristallisiert aus Essigsäureethylester um.
Ausbeute: 1.5 g (80% d. Th.)
LC-MS (Methode 1): R, = 0.51 min; MS (ESIpos): m/z = 135 [M+H]⁺;
¹H-NMR (400 MHz, DMSO-d₆) = 8.56 (s, 1H), 8.35 (s, 1H), 7.73 (d, 1H), 6.75 (m, 1H), 4.42 (s, 1H).

### Beispiel 20A

### 2-Hydrazino-5-methylpyridin

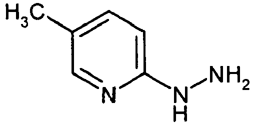

1.0 g (7.8 mmol) 2-Chlor-5-methylpyridin werden in 5.7 ml (5.9 g, 117.6 mmol) Hydrazinhydrat 12 h unter Rückfluss gerührt. Die abgekühlte Reaktionsmischung wird mit 10 ml Ethylenglykolmonoethylether versetzt und das Lösungsmittel anschließend am Rotationsverdampfer vollständig entfernt. Man wiederholt diesen Arbeitschritt zweimal, versetzt den Rückstand dann mit Dichlormethan, filtriert den Niederschlag ab, konzentriert das Filtrat im Vakuum auf und trocknet den Rückstand im Vakuum.
Ausbeute: 644 mg (67% d. Th.)
LC-MS (Methode 8): Rₜ = 0.35 min; MS (ESIpos): m/z = 124 [M+H]⁺.

### Beispiel 21A

### 4-Cyclopropyl-6-hydrazinopyrimidin

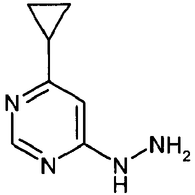

Ausbeute: 0.8 g (69% d. Th.)
GC-MS (Methode 14): Rₜ = 5.72 min; MS (ESIpos): m/z = 151 [M+H]⁺;
¹H-NMR (400 MHz, DMSO-d₆): δ = 8.33 (br. s, 1H), 8.19 (s, 1H), 6.59 (s, 1H), 4.85 (br. s, 2H), 1.90-1.87 (m, 1H), 0.96-0.85 (m, 4H).

Auf analoge Weise werden die in der folgenden Tabelle 1 aufgeführten Verbindungen aus dem angegebenen Edukt gemäß der folgenden Vorschrift hergestellt:

Es wird Hydrazinhydrat oder eine 1 M Lösung von Hydrazin in THF verwendet. Die Umsetzung kann auch in einer *single mode*-Mikrowelle (CEM Explorer oder Emrys Optimizer) erfolgen, typischerweise in Ethanol oder THF bei 150°C über einen Zeitraum von etwa 15 min bis 4 h. Die Aufreinigung erfolgt wie bei der Darstellung von Beispiel 9A beschrieben oder auf ähnliche Weise, z.B. durch Waschen des ausgefallenen Feststoffs mit Wasser und Umkristallisation aus Essigsäureethylester oder durch Verrühren mit Essigsäureethylester oder Petrolether. Überschüssiges Hydrazinhydrat kann durch Aufnehmen des Rohprodukts in z.B. Essigsäureethylester und Waschen mit gesättigter Natriumhydrogencarbonat-Lösung entfernt werden.

**Tabelle 1**

| **Beispiel Nr.** | **Struktur** | **Edukt; Darstellung analog [Beispiel]; Ausbeute (% d. Th.)** | **MS (ESI) [M+H]⁺; LC-MS/ GC-MS (Methode)** | **¹H-NMR (400 MHz)** |
|---|---|---|---|---|
| **22A** | 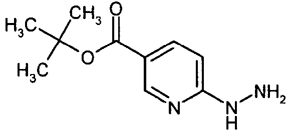 | 6-Chlornicotinsäure*-tert.-*butylester [19A] 93% | m/z = 210; Rₜ = 2.28 min (1) | (DMSO-d₆): δ = 8.48 (d, 1H), 8.30 (s, 1H), 7.82 (dd, 1H), 6.70 (d, 1H), 4.35 (s, 2H), 1.50 (s, 9H). |
| **23A** | 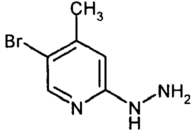 | 5-Brom-2-chlor-4-picolin [5A] 67% | m/z = 202; Rₜ = 0.86 min (8) | (DMSO-d₆): δ = 7.99 (s, 1H), 7.52 (s, 1H), 6.70 (s, 1H), 5.51 (br. s, 1H), 4.11 (br. s, 2H), 2.22 (s, 3H). |
| **24A** | 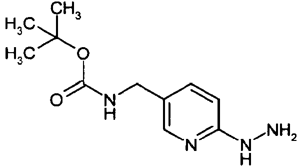 | 15A [19A] 37% | m/z = 239; Rₜ = 1.00 min (8) | |
| **25A** | 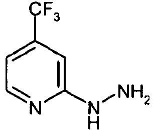 | 2-Chlor-4-(trifluormethyl)pyridin [19A] 91% | m/z = 178; Rₜ = 0.27 min (10) | (CDCl₃): δ = 8.23 (d, 1H), 7.00 (s, 1H), 6.83 (dd, 1H), 6.30 (br. s, 1H), 3.75 (br. s, 2H). |
| **26A** | 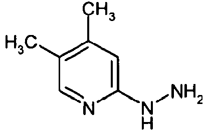 | 14A [19A] 65% | m/z = 138; Rₜ = 0.75 min (8) | (CDCl₃): δ = 7.86 (s, 1H), 6.51 (s, 1H), 5.61 (br. s, 1H), 3.72 (br. s, 2H), 2.20 (s, 3H), 2.12 (s, 3H). |
| **27A** | 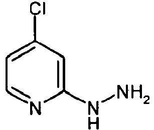 | 2,4-Dichlorpyridin [19A] 47% | m/z = 144; Rₜ = 0.23 min (8) | |
| **28A** | 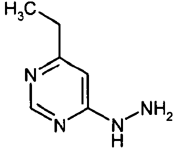 | 4-Chlor-6-ethyl-pyrimidin [US 5,468,751] [19A] | m/z = 139; Rₜ = 4.94 min (14) | |
| **29A** | 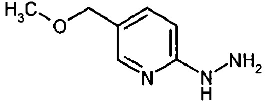 | 13A [19A] 45% | m/z = 154; Rₜ = 0.20 min (6) | (CDCl₃): δ = 8.08 (d, 1H), 7.50 (dd, 1H), 6.70 (d, 1H), 5.98 (br. s, 1H), 4.33 (s, 2H), 3.72 (br. s, 2H), 3.35 (s, 3H). |

### Beispiel 30A

### 4-Hydrazino-6-(4-pyrrolidin-1-ylpiperidin-1-yl)pyrimidin

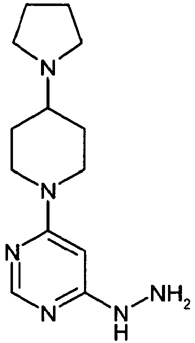

Ein Gemisch aus 2.0 g (13.8 mmol) der Verbindung aus Beispiel 7A und 4.3 g (27.7 mmol) 4-Pyrrolidin-1-ylpiperidin in 20 ml Wasser wird 16 h bei 100°C gerührt. Der ausgefallene Feststoff wird abfiltriert, zunächst mit Ethanol, dann mit Diethylether gewaschen und im Vakuum getrocknet.
Ausbeute: 3.0 g (82% d. Th.)
LC-MS (Methode 8): Rₜ = 0.21 min; MS (ESIpos): m/z = 263 [M+H]⁺;
¹H-NMR (400 MHz, DMSO-d₆): δ = 7.92 (s, 1H), 7.58 (s, 1H), 5.91 (s, 1H), 4.17-4.07 (m, 4H), 2.95-2.85 (m, 2H), 2.54 (s, 2H), 2.52-2.46 (m, 2H), 2.24-2.15 (m, 1H), 1.90-1.80 (m, 2H), 1.71-1.61 (m, 4H), 1.39-1.24 (m, 2H).

### Beispiel 31A

### 4-Hydrazino-6-[4-(2-methoxyethyl)piperazin-1-yl]pyrimidin

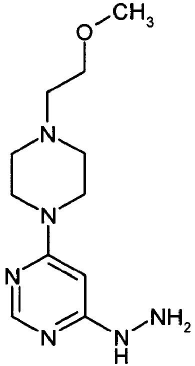

Ein Gemisch aus 1.0 g (6.9 mmol) der Verbindung aus Beispiel 7A und 1.1 g (7.6 mmol) 1-(2-Methoxyethyl)piperazin in 10 ml Wasser wird 2 h bei 100°C gerührt. Weitere 0.9 g (6.2 mmol) 1-(2-Methoxyethyl)piperazin werden hinzugefügt, und das Reaktionsgemisch wird 16 h bei 100°C weiter gerührt. Nach dem Einengen im Vakuum wird der Rückstand in Acetonitril verrührt. Der ausgefallene Feststoff wird abfiltriert, zunächst mit Ethanol, dann mit Diethylether gewaschen und im Vakuum getrocknet.
Ausbeute: 0.8 g (42% d. Th.)
LC-MS (Methode 8): Rₜ = 0.22 min; MS (ESIpos): m/z = 253 [M+H]⁺;
¹H-NMR (400 MHz, DMSO-d₆): δ = 7.93 (s, 1H), 7.64 (s, 1H), 5.91 (s, 1H), 4.14 (s, 2H), 3.50-3.40 (m, 6H), 3.24 (s, 3H), 2.47-2.39 (m, 4H).

### Beispiel 32A

### [4-(Trifluormethyl)-1H-imidazol-1-yl]essigsäureethylester

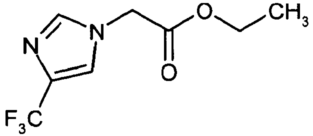

2.0 g (14.7 mmol) 4-(Trifluormethyl)-1*H-*imidazol werden in 5.5 ml (4.7 g, 14.7 mmol) 21%-iger Natriumethylat-Lösung in Ethanol vorgelegt und mit 1.8 ml (2.7 g, 16.2 mmol) Bromessigsäureethylester versetzt. Die Reaktionsmischung wird 16 h bei RT gerührt. Zur Aufarbeitung wird der ausgefallene Feststoff abfiltriert, der Filterrückstand mit Ethanol gewaschen und das Filtrat im Vakuum aufkonzentriert. Der Rückstand wird mit Diisopropylether versetzt, nochmals filtriert, das Filtrat wieder am Rotationsverdampfer eingeengt und der Rückstand im Vakuum getrocknet. Das Produkt fällt als 9:1-Gemisch der beiden Regioisomere an und wird als solches weiter umgesetzt.
Ausbeute: 3.3 g (95% d. Th.)
LC-MS (Methode 1): Rₜ = 1.75 min + 1.80 min; MS (ESIpos): m/z = 223 [M+H]⁺;
¹H-NMR (400 MHz, DMSO-d₆): δ = 7.93 (s, 1H), 7.82 (s, 1H), 5.04 (s, 2H), [5.07 (s, 2H)], 4.18 (q, 2H), [4.12 (q, 2H)], 1.22 (t, 3H), [1.19 (t, 3H)].

### Beispiel 33A

### [4-Cyano-1H-imidazol-1-yl]essigsäureethylester

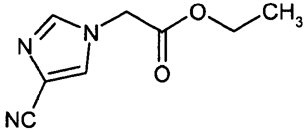

3.3 g (35.3 mmol) 1*H*-Imidazol-4-carbonitril [Matthews et al., J. Org. Chem. 1986, 51, 3228-3231] werden in 13.2 ml (11.5 g, 35.3 mmol) 21%-iger Natriumethylat-Lösung in Ethanol vorgelegt und mit 4.3 ml (6.5 g, 38.9 mmol) Bromessigsäureethylester versetzt. Die Reaktionsmischung wird 16 h bei RT gerührt. Zur Aufarbeitung wird der ausgefallene Feststoff abfiltriert, der Filterrückstand mit Ethanol gewaschen und das Filtrat im Vakuum aufkonzentriert. Der Rückstand wird mit Diisopropylether versetzt, nochmals filtriert, das Filtrat wieder am Rotationsverdampfer eingeengt und der Rückstand im Vakuum getrocknet.
Ausbeute: 3.8 g (60% d. Th.)
LC-MS (Methode 1): Rₜ = 1.17 min; MS (ESIpos): m/z = 180 [M+H]⁺;
¹H-NMR (400 MHz, DMSO-d₆): δ = 8.12 (s, 1H), 7.88 (s, 1H), 5.06 (s, 2H), 4.18 (q, 2H), 1.22 (t, 3H).

### Beispiel 34A

### (4-Methyl-1H-imidazol-1-yl)essigsäureethylester

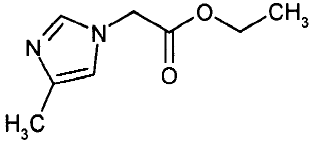

4.4 g (53.6 mmol) 4-Methyl-1*H*-imidazol werden in 20.0 ml (17.4 g, 53.6 mmol) 21%-iger Natriumethylat-Lösung in Ethanol vorgelegt und mit 6.5 ml (9.8 g, 58.9 mmol) Bromessigsäureethylester versetzt. Die Reaktionsmischung wird 16 h bei RT gerührt. Zur Aufarbeitung wird der ausgefallene Feststoff abfiltriert, der Filterrückstand mit Ethanol gewaschen und das Filtrat im Vakuum aufkonzentriert. Der Rückstand wird säulenchromatographisch an Kieselgel (Laufmittel: Acetonitril/Wasser 9:1) aufgereinigt. Das Produkt fällt als 3:2-Gemisch der beiden Regioisomere an und wird als solches weiter umgesetzt.
Ausbeute: 1.8 g (20% d. Th.)
LC-MS (Methode 1): Rₜ = 1.02 min; MS (ESIpos): m/z = 169 [M+H]⁺;
¹H-NMR (400 MHz, DMSO-d₆): δ = 7.48 (s, 1H), [7.52 (s, 1H)], 6.82 (s, 1H), [6.64 (s, 1H)], 4.86 (s, 2H), [4.88 (s, 2H)], 4.22-4.11 (m, 2H), 2.07 (s, 3H), [2.06 (s, 3H)], 1.25-1.19 (m, 3H).

### Beispiel 35A

### 2-Methyl-1H-imidazol-1-yl)essigsäureethylester

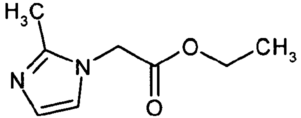

2.0 g (24.4 mmol) 2-Methyl-1*H*-imidazol werden in 9.1 ml (7.9 g, 24.4 mmol) 21%-iger Natriumethylat-Lösung in Ethanol vorgelegt und mit 2.9 ml (4.5 g, 26.8 mmol) Bromessigsäureethylester versetzt. Die Reaktionsmischung wird 16 h bei RT gerührt. Zur Aufarbeitung wird der ausgefallene Feststoff abfiltriert, der Filterrückstand mit Ethanol gewaschen und das Filtrat im Vakuum aufkonzentriert. Der Rückstand wird in Diisopropylether verrührt, abermals filtriert und das Filtrat wieder im Vakuum eingeengt.
Ausbeute: 2.9 g (70% d. Th.)
LC-MS (Methode 1): Rₜ = 0.49 min; MS (ESIpos): m/z = 169 [M+H]⁺.

### Beispiel 36A

### (4-Methyl-1H-1,2,3-triazol-1-yl)essigsäureethylester

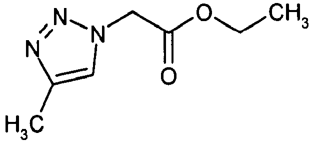

2.0 g (14.2 mmol) (4-Methyl-1*H*-1,2,3-triazol-1-yl)essigsäure werden in 30 ml Ethanol gelöst und mit 10 Tropfen konz. Schwefelsäure versetzt. Die Reaktionsmischung wird 16 h bei RT gerührt. Zur Aufarbeitung wird die Mischung im Vakuum aufkonzentriert, der Rückstand mit Essigsäureethylester versetzt und die Suspension mit halbkonzentrierter Natriumhydrogencarbonat-Lösung gewaschen. Die organische Phase wird über Magnesiumsulfat getrocknet, das Lösungsmittel am Rotationsverdampfer vollständig entfernt und der Feststoff im Vakuum getrocknet.
Ausbeute: 1.5 g (61 % d. Th.)
LC-MS (Methode 1): Rₜ = 2.33 min; MS (ESIpos): m/z = 170 [M+H]⁺;
¹H-NMR (400 MHz, DMSO-d₆): δ = 7.82 (s, 1H), 5.31 (s, 2H), 4.17 (q, 2H), 2.25 (s, 3H), 1.21 (t, 3H).

Die in Tabelle 2 aufgeführten Verbindungen werden aus dem angegebenen Edukt analog zu Beispiel 36A hergestellt:

**Tabelle 2**

| **Beispiel Nr.** | **Struktur** | **Edukt; Ausbeute (% d. Th.)** | **MS (ESI) [M+H]⁺; LC-MS (Methode)** | **¹H-NMR (400 MHz, DMSO-d₆)** |
|---|---|---|---|---|
| **37A** | 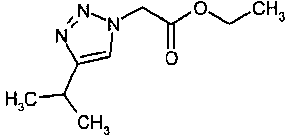 | (4-Isopropyl-1*H*-1,2,3-triazol-1-yl)-essigsäure 100% | m/z = 198; Rₜ = 1.68 min (4) | δ = 7.84 (s, 1H), 5.32 (s, 2H), 4.18 (q, 2H), 3.02-2.98 (m, 1H), 1.28-1.19 (m, 9H). |
| **38A** | 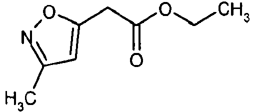 | (3-Methylisoxazol-5-yl)essigsäure 80% | m/z = 170; Rₜ = 2.70 min (1) | δ = 6.28 (s, 1H), 4.12 (q, 2H), 3.95 (s, 2H), 2.21 (s, 3H), 1.21 (t, 3H). |

### Beispiel 39A

### 2-(1H-1,2,3-Triazol-1-yl)essigsäureethylester

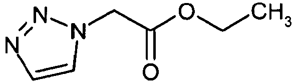

129.2 g (5.6 mol) Natrium werden langsam zu 4.0 Liter Ethanol gegeben. Anschließend werden 400.0 g (5.6 mol) 1,2,3-1*H*-Triazol zugesetzt sowie 623 ml (938.2 g, 5.6 mol) Bromessigsäureethylester bei 20-25°C Innentemperatur zugetropft. Es wird 48 h bei RT gerührt. Der ausgefallene Feststoff wird abfiltriert, das Ethanol im Vakuum entfernt und erneut filtriert. Der Rückstand wird in Essigsäureethylester aufgenommen, filtriert, erneut im Vakuum eingeengt und destillativ an einer 30 cm-Kolonne aufgereinigt. Das Produkt wird bei einer Badtemperatur von 140°C, einer Kopftemperatur von 60-115°C und einem Druck von 1 mbar erhalten.
Ausbeute: 440.0 g (50% d. Th.)
HPLC (Methode 11): Rₜ = 1.58 min;
LC-MS (Methode 1): Rₜ = 0.71 min; MS (ESIpos): m/z = 156 [M+H]⁺.

### Beispiel 40A

### 1H-Imidazol-1-ylessigsäureethylester

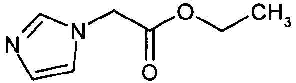

118.2 g (5.1 mol) Natrium werden langsam zu 2.5 Liter Ethanol gegeben. Anschließend werden 350.0 g (5.1 mol) Imidazol zugesetzt sowie 570 ml (858.6 g, 5.1 mol) Bromessigsäureethylester bei 20-25°C Innentemperatur zugetropft. Es wird 24 h bei RT gerührt. Der ausgefallene Feststoff wird abfiltriert, das Ethanol im Vakuum entfernt und erneut filtriert. Der Rückstand wird säulenchromatographisch an Kieselgel aufgereinigt (Laufmittel: Essigsäureethylester).
Ausbeute: 639.0 g (81% d. Th.)
GC-MS (Methode 14): Rₜ = 4.55 min; MS (ESIpos): m/z = 155 [M+H]⁺.

### Beispiel 41A

### (4-Cyano-1H-1,2,3-triazol-1-yl)essigsäureethylester

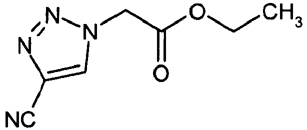

4.1 g (31.9 mmol) Azidoessigsäureethylester und 2.8 g (31.9 mmol) 2-Chloracrylsäurenitril werden in 32 ml Wasser 16 h bei einer Badtemperatur von 80°C gerührt. Nach Abkühlen auf RT wird die Lösung mit 1 N Salzsäure sauer gestellt und mit Essigsäureethylester extrahiert. Die organische Phase wird über Natriumsulfat getrocknet, filtriert und im Vakuum eingeengt. Der Rückstand wird mit 50 ml Ethanol und 10 Tropfen konz. Schwefelsäure versetzt und 16 h unter Rückfluss gerührt. Zur Aufarbeitung wird die Reaktionsmischung im Vakuum aufkonzentriert, der Rückstand mit Essigsäureethylester versetzt, die Suspension mit halbkonzentrierter Natriumhydrogencarbonat-Lösung gewaschen und die organische Phase über Natriumsulfat getrocknet. Das Lösungsmittel wird am Rotationsverdampfer vollständig entfernt und der Feststoff im Vakuum getrocknet.
Ausbeute: 1.5 g (25% d. Th.)
LC-MS (Methode 7): Rₜ = 0.96 min; MS (ESIpos): m/z = 181 [M+H]⁺;
¹H-NMR (400 MHz, DMSO-d₆): δ = 9.06 (s, 1H), 5.57 (s, 2H), 4.19 (q, 2H), 1.22 (t, 3H).

### Beispiel 42A

### 3-(Dimethylamino)-2-(1H-imidazol-1-yl)aerylsäureethylester

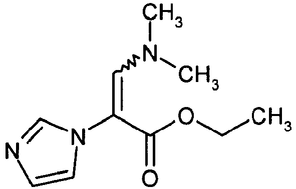

38.0 g (244.9 mmol) der Verbindung aus Beispiel 39A werden in 126 ml (108.1 g, 734.7 mmol) Dimethylformamid-diethylacetal 16 h bei 90°C Badtemperatur gerührt. Nach dem Abkühlen engt man im Vakuum ein, verrührt mit Diisopropylether, filtriert den Feststoff ab und wäscht diesen abschließend mit Diisopropylether.
Ausbeute: 49.0 g (95% d. Th.)
LC-MS (Methode 4): Rₜ = 2.42 min; MS (ESIpos): m/z = 211 [M+H]⁺;
¹H-NMR (400 MHz, DMSO-d₆): δ = 7.52 (s, 1H), 7.49 (s, 1H), 7.05 (s, 1H), 6.91 (s, 1H), 4.02 (q, 2H), 2.63 (br. s, 6H), 1.12 (t, 3H).

### Beispiel 43A

### 3-(Dimethylamino)-2-(1H-1,2,4-triazol-1-yl)acrylsäureethylester

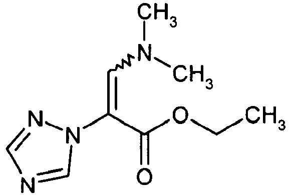

1.9 g (9.8 mmol) [1,2,4]Triazol-1-ylessigsäureethylester [Ainsworth et al., J. Am. Chem. Soc. 1955, 77, 621-623] und 3.6 ml (2.9 g, 19.6 mmol) Dimethylformamid-diethylacetal werden bei 100°C Badtemperatur 12 h lang gerührt. Zur Aufarbeitung wird die abgekühlte Reaktionslösung am Rotationsverdampfer eingeengt und der Rückstand im Vakuum getrocknet.
Ausbeute: 2.3 g (90% Reinheit, 100% d.Th.)
LC-MS (Methode 1): Rₜ = 2.32 min; MS (ESIpos): m/z = 211 [M+H]⁺;
¹H-NMR (400 MHz, DMSO-d₆): δ = 8.48 (s, 1H), 8.04 (s, 1H), 7.61 (s, 1H), 4.03 (q, 2H), 3.04 (br. s, 3H), 2.25 (br. s, 3H), 1.12 (t, 3H).

### Beispiel 44A

### 3-(Dimethylamino)-2-[4-(trifluormethyl)-1H-imidazol-1-yl]acrylsäureethylester

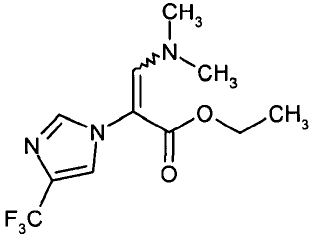

38.0 g (170.8 mmol) der Verbindung aus Beispiel 32A und 58.5 ml (50.3 g, 341.6 mmol) Dimethylformamid-diethylacetal werden bei 100°C Badtemperatur 16 h lang gerührt. Zur Aufarbeitung wird die abgekühlte Reaktionslösung am Rotationsverdampfer eingeengt und der Rückstand im Vakuum getrocknet.
Ausbeute: 49.5 g (97% d.Th.)
LC-MS (Methode 8): Rₜ = 1.68 min; MS (ESIpos): m/z = 278 [M+H]⁺;
¹H-NMR (400 MHz, DMSO-d₆): δ = 7.81 (s, 1H), 7.27 (s, 1 H), 7.58 (s, 1H), 4.03 (q, 2H), 2.68 (br. s, 6H), 1.13 (t, 3H).

### Beispiel 45A

### 3-(Dimethylamino)-2-[4-cyano-1H-imidazol-1-yl]acrylsäureethylester

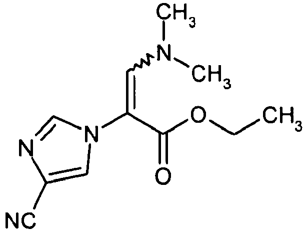

3.8 g (21.4 mmol) der Verbindung aus Beispiel 33A und 7.4 ml (6.3 g, 42.8 mmol) Dimethylformamid-diethylacetal werden bei 100°C Badtemperatur 16 h lang gerührt. Zur Aufarbeitung wird die abgekühlte Reaktionslösung am Rotationsverdampfer eingeengt und der Rückstand im Vakuum getrocknet.
Ausbeute: 5.0 g (73% Reinheit, 73% d.Th.)
LC-MS (Methode 1): Rₜ = 2.69 min; MS (ESIpos): m/z = 235 [M+H]⁺;
¹H-NMR (400 MHz, DMSO-d₆): δ = 8.13 (s, 1H), 7.85 (s, 1H), 7.58 (s, 1H), 4.03 (q, 2H), 2.69 (br. s, 6H), 1.12 (t, 3H).

### Beispiel 46A

### 3-(Dimethylamino)-2-[4-methyl-1H-imidazol-1-yl]acrylsäureethylester

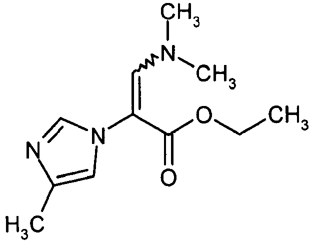

310 mg (1.5 mmol, 80% Reinheit) der Verbindung aus Beispiel 34A und 0.5 ml (434 mg, 3.0 mmol) Dimethylformamid-diethylacetal werden bei 100°C Badtemperatur 16 h lang gerührt. Zur Aufarbeitung wird die abgekühlte Reaktionslösung am Rotationsverdampfer eingeengt und der Rückstand im Vakuum getrocknet. Das Produkt fällt als 3:2-Gemisch der beiden Regioisomere an und wird als solches weiter umgesetzt.
Ausbeute: 329 mg (99% d.Th.)
LC-MS (Methode 1): Rₜ = 2.05 min; MS (ESIpos): m/z = 224 [M+H]⁺;
¹H-NMR (400 MHz, DMSO-d₆): δ = 7.50 (s, 1H), [7.58 (s, 1H)], 7.32 (d, 1H), [7.38 (d, 1H)], 6.73 (s, 1H), [6.66 (s, 1H)], 4.04-3.98 (m, 2H), 2.64 (br. s, 6H), 2.08 (s, 3H), [1.97 (s, 3H)], 1.12 (t, 3H).

Die in Tabelle 3 aufgeführten Verbindungen werden aus dem angegebenen Edukt und Dimethylformamid-diethylacetal analog zu Beispiel 43A hergestellt:

**Tabelle 3**

| **Beispiel Nr.** | **Struktur** | **Edukt; Ausbeute (% d. Th.)** | **MS (ESI) [M+H]⁺; LC-MS (Methode)** | **¹H-NMR (400 MHz, DMSO-d₆)** |
|---|---|---|---|---|
| **47A** | 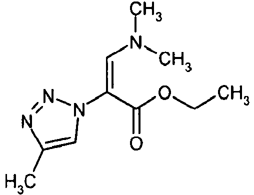 | 36A | m/z = 225; Rₜ = 2.59 min (1) | δ = 7.78 (s, 1H), 7.61 (s, 1H), 4.02 (q, 2H), 3.05 (br. s, 3H), 2.12 (br. s, 3H), 1.13 (t, 3H). |
| **48A** | 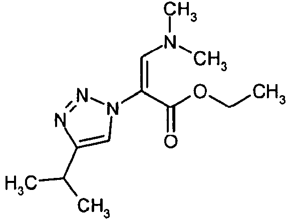 | 37A | m/z = 253; Rₜ = 3.00 min (1) | δ = 7.79 (s, 1H), 7.62 (s, 1H), 4.03 (q, 2H), 3.08 (br. s, 3H), 3.00-2.97 (m, 1H), 2.09 (br. s, 3H), 1.23 (d, 6H), 1.13 (t, 3H). |
| **49A** | 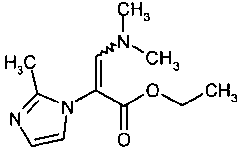 | 35A | m/z = 224; Rₜ = 2.03 min (1) | |
| **50A** | 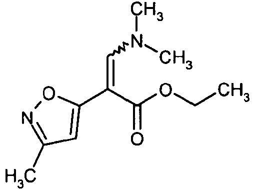 | 38A 75% | m/z = 225; Rₜ =2.91 min (1) | δ = 7.64 (s, 1H), 6.13 (s, 1H), 4.03 (q, 2H), 2.81 (br. s, 6H), 2.21 (s, 3H), 1.12 (t, 3H). |
| **51A** | 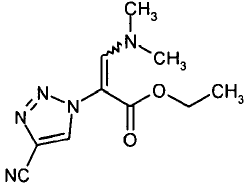 | 41A | m/z = 236; Rₜ = 1.34 min (8) | δ = 9.14 (s, 1H), 7.75 (s, 1H), 4.04 (q, 2H), 3.15 (br. s, 3H), 2.18 (br. s, 3H), 1.13 (t, 3h). |

### Beispiel 52A

### 2-(6-Chlorpyrimidin-4-yl)-4-(1H-1,2,3-triazol-1-yl)-1,2-dihydro-3H-pyrazol-3-on-Hydrochlorid

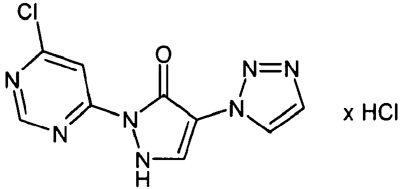

10.0 g (47.7 mmol) der Verbindung aus Beispiel 3A und 8.3 g (57.1 mmol) der Verbindung aus Beispiel 7A werden in 100 ml Ethanol vorgelegt und mit 1.5 ml (2.2 g, 19.0 mmol) TFA versetzt. Es wird 12 h unter Rückfluss gerührt. Anschließend gibt man der abgekühlten Reaktionsmischung eine 4 M Lösung von Chlorwasserstoff in Dioxan im Überschuss zu, rührt ca. 1 h aus, filtriert die ausgefallenen Kristalle ab und wäscht den Filterrückstand mit Dioxan und Ethanol. Das so erhaltene Zwischenprodukt wird in 150 ml Ethanol gelöst, mit 50 ml einer 25%-igen methanolischen Natriummethylat-Lösung versetzt und 2 h bei RT gerührt. Anschließend wird die Reaktionsmischung mit 1 N Salzsäure auf pH 5 gestellt, weitere 2 h bei RT ausgerührt, der Feststoff abfiltriert, der Filterrückstand mit Ethanol gewaschen und das Produkt im Vakuum getrocknet.
Ausbeute: 7.0 g (49% d.Th.)
LC-MS (Methode 10): Rₜ = 1.20 min; MS (ESIpos): m/z = 264 [M+H]⁺.

### Beispiel 53A

### 2-(6-Chlorpyrimidin-4-yl)-4-(1H-imidazol-1-yl)-1,2-dihydro-3H-pyrazol-3-on-Hydrochlorid

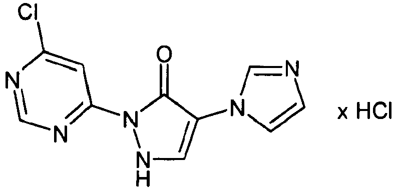

10.0 g (47.8 mmol) der Verbindung aus Beispiel 42A und 8.3 g (57.3 mmol) der Verbindung aus Beispiel 7A werden in 100 ml Ethanol vorgelegt und mit 1.5 ml (2.2 g, 19.0 mmol) TFA versetzt. Es wird 12 h unter Rückfluss gerührt. Man filtriert die ausgefallenen Kristalle ab, wäscht den Filterrückstand mit Ethanol nach und trocknet das Zwischenprodukt über Nacht im Vakuum. Dieses wird dann in 20 ml Methanol suspendiert, mit 100 ml einer 4 M Lösung von Chlorwasserstoff in Dioxan versetzt und 1 h bei RT nachgerührt. Der Feststoff wird abfiltriert, der Filterrückstand mit Dioxan, Essigsäureethylester und Diisopropylether gewaschen und das Produkt im Vakuum getrocknet.
Ausbeute: 4.6 g (32% d.Th.)
HPLC (Methode 11): Rₜ = 2.81 min; MS (ESIpos): m/z = 263 [M+H]⁺;
¹H-NMR (400 MHz, DMSO-d₆): δ = 9.46 (s, 1H), 8.96 (s, 1H), 8.56 (s, 1H), 8.51 (d, 1H), 8.07-8.04 (m, 1 H), 7.85-7.82 (m, 1H).

### Beispiel 54A

### 2-(6-Morpholin-4-ylpyrimidin-4-yl)-3-oxo-2,3-dihydro-1H-pyrazol-4-ethylester

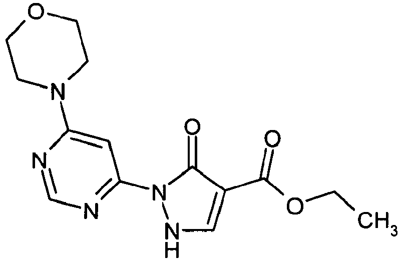

1.4 g (10.0 mmol) Kaliumcarbonat werden in 50 ml Wasser gelöst. Dazu gibt man 2.0 g (10.0 mmol) der Verbindung aus Beispiel 16A und anschließend 2.2 g (10.0 mmol) Ethoxymethylenmalonsäurediethylester und rührt dann 2 h bei 100°C. Man lässt auf RT abkühlen, filtriert den Feststoff ab, wäscht zweimal mit Wasser und trocknet im Hochvakuum.
Ausbeute: 2.4 g (75% d. Th.)
LC-MS (Methode 8): Rₜ = 1.31 min; MS (ESIpos): m/z = 320 [M+H]⁺;
¹H-NMR (400 MHz, DMSO-d₆): δ = 8.30 (s, 1H), 7.97 (s, 1H), 7.48 (s, 1H), 4.00 (q, 2H), 3.75-3.61 (m, 4H), 3.55-3.45 (m, 4H), 1.18 (t, 3H).

### Ausführungsbeispiele:

### Beispiel 1

### 2-Pyridin-2-yl-4-(1H-1,2,3-triazol-1-yl)-1,2-dihydro-3H-pyrazol-3-on

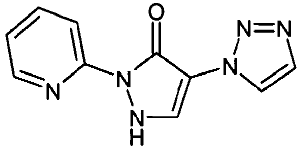

250 mg (1.19 mmol) der Verbindung aus Beispiel 3A, 108 mg (0.99 mmol) 2-Hydrazinopyridin und 23 mg (99 µmol) Campher-10-sulfonsäure werden in 5 ml wasserfreiem Ethanol gelöst und über Nacht unter Rückfluss erhitzt. Man gibt erneut insgesamt viermal jeweils 108 mg (0.99 mmol) 2-Hydrazinopyridin hinzu und erhitzt weiter unter Rückfluss, bis der Umsatz der Verbindung aus Beispiel 3A vollständig ist. Nach dem Abkühlen reinigt man die Reaktionsmischung durch mehrmalige präparative HPLC (RP18-Säule; Laufmittel: Acetonitril/Wasser-Gradient unter Zusatz von 0.1 % konz. Salzsäure) und erhält 6 mg (2% d. Th.) der Titelverbindung.
¹H-NMR (400 MHz, DMSO-d₆): δ = 8.52 (d, 1H), 8.44 (s, 1H), 8.37 (s, 1H), 8.29-8.23 (m, 1H), 8.11-8.06 (m, 1H), 7.90 (s, 1H), 7.43-7.38 (m, 1H).
LC-MS (Methode 2): Rₜ = 1.17 min; MS (ESIpos): m/z = 229 [M+H]⁺.

### Beispiel 2

### 2-(4-Methylpyridin-2-yl)-4-(1H-1,2,3-triazol-1-yl)-1,2-dihydro-3H-pyrazol-3-on

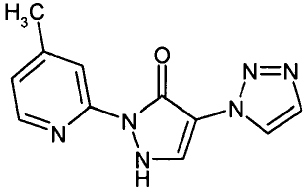

250 mg (1.19 mmol) der Verbindung aus Beispiel 3A, 122 mg (0.99 mmol) der Verbindung aus Beispiel 1A und 23 mg (99 µmol) Campher-10-sulfonsäure werden in 5 ml wasserfreiem Ethanol gelöst und über Nacht unter Rückfluss erhitzt. Man lässt danach abkühlen und reinigt die Reaktionsmischung mittels präparativer HPLC (RP18-Säule; Laufmittel: Acetonitril/Wasser-Gradient) und nachfolgender Flash-Chromatographie an Kieselgel (Laufmittel: Dichlormethan/Methanol-Gradient) vor. Erneute Reinigung mittels präparativer HPLC (RP18-Säule; Laufmittel: Acetonitril/Wasser-Gradient) ergibt schließlich 21 mg (9% d. Th.) der Titelverbindung.
¹H-NMR (400 MHz, DMSO-d₆): δ = 8.42 (s, 1H), 8.38 (d, 1H), 8.29 (s, 1H), 8.09 (s, 1H), 7.90 (s, 1H), 7.27 (d, 1H), 2.46 (s, 3H).
LC-MS (Methode 4): Rₜ = 1.05 min; MS (ESIpos): m/z = 243 [M+H]⁺.

### Beispiel 3

### 2-(6-Morpholin-4-yl-pyrimidin-4-yl)-4-[5-(trifluormethyl)-1,3,4-thiadiazol-2-yl]-1,2-dihydro-3H-pyrazol-3-on

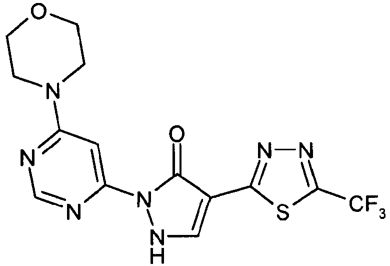

2.43 g (8.62 mmol) der Verbindung aus Beispiel 2A, 1.53 g der Verbindung aus Beispiel 16A und 182 mg (784 µmol) Campher-10-sulfonsäure werden in 35 ml wasserfreiem Methanol gelöst und über Nacht unter Rückfluss erhitzt. Nach dem Abkühlen engt man ein, nimmt den Rückstand erneut in 325 ml Methanol auf, setzt 0.5 ml (8.62 mmol) einer 25%-igen methanolischen Natriummethylat-Lösung hinzu und erhitzt wiederum über Nacht unter Rückfluss. Nach dem Abkühlen wird der entstandene Niederschlag abgesaugt, mit Diethylether gewaschen, in wenig Wasser suspendiert, mit einem Überschuss an 1 M Salzsäure versetzt und die Suspension wieder eingeengt. Der Rückstand wird mit Wasser und Diethylether gewaschen und getrocknet. Man erhält 1.34 g (43% d. Th.) der Titelverbindung.
¹H-NMR (400 MHz, DMSO-d₆): δ = 8.55 (s, 1H), 8.26 (s, 1H), 7.49 (s, 1H), 3.84-3.70 (m, 8H).
LC-MS (Methode 4): R, = 1.68 min; MS (ESIpos): m/z = 400 [M+H]⁺.

### Beispiel 4

### 2-(6-Piperidin-1-yl-pyrimidin-4-yl)-4-[5-(trifluormethyl)-1,3,4-thiadiazol-2-yl]-1,2-dihydro-3H-pyrazol-3-on

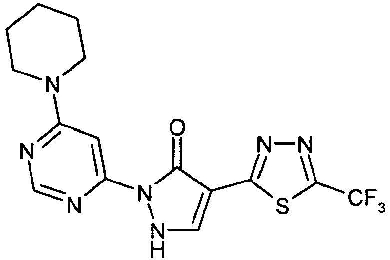

240 mg (854 µmol) der Verbindung aus Beispiel 2A, 150 mg (776 µmol) der Verbindung aus Beispiel 8A und 18 mg (78 µmol) Campher-10-sulfonsäure werden in 3 ml wasserfreiem Ethanol gelöst und über Nacht unter Rückfluss erhitzt. Nach dem Abkühlen setzt man 64 mg (931 µmol) Natriumethylat hinzu und rührt weiter über Nacht bei RT. Der entstandene Niederschlag wird abgesaugt, mit Ethanol und Diethylether gewaschen und getrocknet. Das Filtrat wird eingeengt und der Rückstand mittels präparativer HPLC (RP18-Säule; Laufmittel: Acetonitril/Wasser-Gradient) gereinigt. Die beiden so erhaltenen Zwischenproduktfraktionen werden zusammen in 5 ml Methanol suspendiert, mit 15 mg (278 µmol) Natriummethylat versetzt und über Nacht unter Rückfluss erhitzt. Nach dem Abkühlen reinigt man das Reaktionsgemisch mittels präparativer HPLC (RP18-Säule; Laufmittel: Acetonitril/Wasser-Gradient) und erhält 26 mg (8% d. Th.) der Titelverbindung.
¹H-NMR (400 MHz, DMSO-d₆): δ = 8.33 (s, 1H), 7.87 (s, 1H), 7.76 (s, 1H), 3.63-3.58 (m, 4H), 1.68-1.51 (m, 6H).
LC-MS (Methode 3): Rₜ = 2.23 min; MS (ESIpos): m/z = 398 [M+H]⁺.

Die in Tabelle 4 aufgeführten Verbindungen werden aus den entsprechenden Edukten analog zu Beispiel 4 hergestellt:

**Tabelle 4**

| **Beispiel Nr.** | **Struktur** | **Edukte; Ausbeute (% d. Th.)** | **MS (ESI) [M+H]⁺; LC-MS: R, (Meth.)** | **¹H-NMR (400 MHz, DMSO-d₆)** |
|---|---|---|---|---|
| **5** | 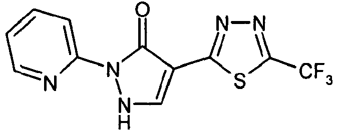 | 2A; 33% | m/z = 314; 1.60 min (4) | δ = 8.38 (d, 1H), 8.27 (d, 1H), 7.86 (s, 1H), 7.83-7.77 (m, 1H), 7.10 (dd, 1H). |
| **6** | 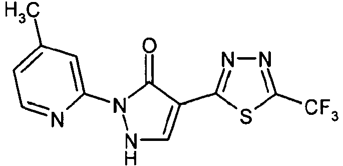 | 2A, 1A; 36% | m/z = 328; 1.71 min (4) | δ = 8.21 (d, 1H), 8.12 (s, 1H), 7.84 (s, 1H), 6.95 (d, 1H), 2.35 (s, 3H). |

### Beispiel 7

### 2-(4-Methylpyridin-2-yl)-4-(1H-1,2,3-triazol-1-yl)-1,2-dihydro-3H-pyrazol-3-on-Hydrochlorid

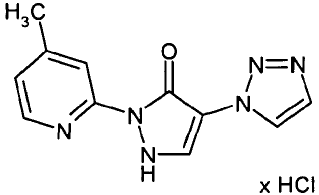

3.7 g (17.6 mmol) der Verbindung aus Beispiel 3A und 2.6 g (21.1 mmol) der Verbindung aus Beispiel 1A werden in 100 ml wasserfreiem Ethanol gelöst und mit 0.7 ml (1.0 g, 8.8 mmol) TFA versetzt. Es wird 16 h bei einer Badtemperatur von 100°C gerührt. Man lässt danach auf RT abkühlen und engt im Vakuum ein. Der Rückstand wird in 35 ml Acetonitril gelöst und bei RT mit 9 ml (35.2 mmol) einer 4 N Lösung von Chlorwasserstoff in Dioxan versetzt. Der Niederschlag wird abgetrennt und mit 35 ml Acetonitril gewaschen. Der Feststoff wird mit Diisopropylether gewaschen, anschließend in 10 ml Methanol auf 40°C erwärmt, mit 10 ml Essigsäureethylester versetzt, abfiltriert und mit 5 ml eines 1:1-Gemisches aus Methanol und Essigsäureethylester sowie 2 ml Essigsäureethylester gewaschen.
Ausbeute: 2.0 g (40% d. Th.)
LC-MS (Methode 2): Rₜ = 1.05 min; MS (ESIpos): m/z = 243 [M+H]⁺;
¹H-NMR (400 MHz, DMSO-d₆): δ = 8.42-8.40 (m, 1H), 8.37 (d, 1H), 8.29 (s, 1H), 8.09 (s, 1H), 7.89 (d, 1H), 7.27 (d, 1H), 2.46 (s, 3H).

### Beispiel 8

### 6-[5-Oxo-4-(1H-1,2,3-triazol-1-yl)-2,5-dihydro-1H-pyrazol-1-yl]nicotinsäureethylester-Hydrochlorid

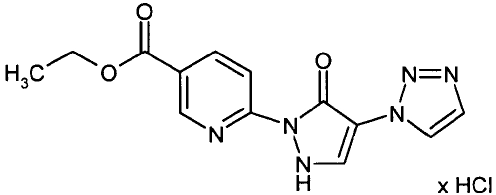

227 mg (1.1 mmol) der Verbindung aus Beispiel 3A, 245 mg (1.1 mmol) 6-Hydrazinonicotinsäureethylester [Herstellung siehe WO 2006/114213] und 50 mg (0.2 mmol) Campher-10-sulfonsäure werden in 6 ml Ethanol 3 h unter Rückfluss gerührt. Die abgekühlte Reaktionsmischung wird danach mit weiteren 490 mg (2.2 mmol) 6-Hydrazinonicotinsäureethylester und 37 mg (0.2 mmol) p-Toluolsulfonsäure versetzt und 1 d in der Siedehitze gerührt. Zur Aufarbeitung wird die Reaktionsmischung im Vakuum aufkonzentriert und der Rückstand mittels präparativer HPLC (Methode 12) chromatographiert. Das aus der HPLC-Trennung erhaltene, lyophilisierte Trifluoracetat-Salz wird mit 4 ml einer 4 N Lösung von Chlorwasserstoff in Dioxan versetzt, die Suspension teilweise eingeengt, der Feststoff abfiltriert, der Filterrückstand mit Diethylether gewaschen und das Produkt im Vakuum getrocknet.
Ausbeute: 139 mg (38% d. Th.)
LC-MS (Methode 1): Rₜ = 2.88 min; MS (ESIpos): m/z = 301 [M+H]⁺;
¹H-NMR (400 MHz, DMSO-d₆): δ = 9.00 (s, 1H), 8.60-8.57 (m, 1H), 8.53-8.46 (m, 2H), 8.45 (s, 1H), 7.91 (s, 1H), 4.38 (q, 2H), 1.35 (t, 3H).

### Beispiel 9

### 2-(6-Piperidin-1-ylpyrimidin-4-yl)-4-(1H-1,2,3-triazol-1-yl)-1,2-dihydro-3H-pyrazol-3-on-Hydrochlorid

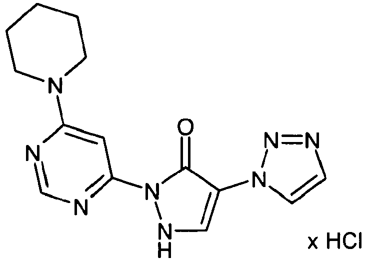

400 mg (1.8 mmol) der Verbindung aus Beispiel 3A, 338 mg (1.8 mmol) der Verbindung aus Beispiel 8A und 60 mg (0.4 mmol) p-Toluolsulfonsäure werden in einer Mischung aus 2 ml THF und 2 ml Ethanol vorgelegt und 1h bei 140°C in einer *single mode*-Mikrowelle (Emrys Optimizer) umgesetzt. Die abgekühlte Reaktionsmischung wird im Vakuum aufkonzentriert und der Rückstand mit 2 ml einer 4 N Lösung von Chlorwasserstoff in Dioxan, Diethylether und Acetonitril versetzt. Man filtriert den ausgefallenen Niederschlag ab und chromatographiert den Filterrückstand mittels präparativer HPLC (RP18-Säule; Laufmittel: Acetonitril/Wasser-Gradient unter Zusatz von 0.1% Ameisensäure im Wasser). Das hieraus erhaltene, lyophilisierte Formiat-Salz wird mit 4 ml einer 4 N Lösung von Chlorwasserstoff in Dioxan versetzt, die Suspension teilweise eingeengt, der Feststoff abfiltriert, der Filterrückstand mit Diethylether gewaschen und das Produkt im Vakuum getrocknet.
Ausbeute: 75 mg (12% d. Th.)
LC-MS (Methode 1): Rₜ = 2.75 min; MS (ESIpos): m/z = 313 [M+H]⁺;
¹H-NMR (400 MHz, DMSO-d₆): δ = 8.51 (s, 1H), 8.38 (s, 1H), 8.21 (s, 1H), 7.85 (s, 1H), 7.38 (s, 1H), 3.76-3.72 (m, 4H), 1.74-1.63 (m, 2H), 1.59 (s, 9H).

### Beispiel 10

### 2-[5-(Hydroxymethyl)pyridin-2-yl]-4-(1H-1,2,3-triazol-1-yl)-1,2-dihydro-3H-pyrazol-3-on

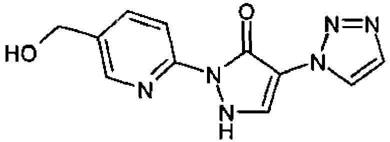

15,1 g (71.9 mmol) der Verbindung aus Beispiel 3A und 10.0 g (71.9 mmol) der Verbindung aus Beispiel 4A werden in 375 ml Ethanol gelöst und mit 1.7 g (7.2 mmol) Campher-10-sulfonsäure versetzt. Es wird 16 h zum Rückfluss erhitzt. Nach dem Abkühlen auf RT wird die Reaktionsmischung eingeengt und der Rückstand an Kieselgel säulenchromatographisch aufgereinigt (Laufmittel: Dichlormethan/Methanol 9:1, dann 1:1). Die Produktfraktion wird im Vakuum eingeengt, der Rückstand in Diisopropylether verrührt, abfiltriert und getrocknet.
Ausbeute: 1.0 g (5% d. Th.)
LC-MS (Methode 1); Rₜ = 2.14 min; MS (ESIpos): m/z = 259 [M+H]⁺;
¹H-NMR (400 MHz, DMSO-d₆): δ = 8.46 (d, 1H), 8.43 (s, 1H), 8.34 (s, 1H), 8.23 (d, 1H), 8.01 (dd, 1H), 7.90 (s, 1H), 5.43 (br. s, 1H), 4.58 (s, 2H).

### Beispiel 11

### 1-[3-Oxo-2-(6-piperidin-1-ylpyrimidin-O-yl)-2,3-dihydro-1H-pyrazol-4-yl]-1H-1,2,3-triazol-4-carbonitril-Hydrochlorid

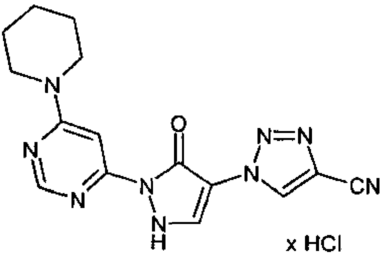

400 mg (1.7 mmol) der Verbindung aus Beispiel 11 und 328 mg (1.7 mmol) der Verbindung aus Beispiel 8A werden in 4 ml Ethanol gelöst und mit 59 mg (0.3 mmol) p-Toluolsulfonsäure versetzt. Es wird t h in einer *single mode*-Mikrowelle (Emrys Optimizer) bei 120°C umgesetzt. Nach Abkühlen auf RT wird die Reaktionsmischung eingeengt und der Rückstand mittels präparativer HPLC (RP18-Säule; Laufmittel: Acetonitril/Wasser-Gradient unter Zusatz von 0.1% Ameisensäure im Wasser) gereinigt. Das hierbei anfallende Formiat-Salz wird durch Zusatz von 0.2 ml einer 4 N Lösung von Chlorwasserstoff in Dioxan in das Hydrochlorid überführt.
Ausbeute: 4 mg (1% d. Th.)
LC-MS (Methode 8): Rₜ = 1.73 min; MS (ESIpos): m/z = 338 [M+H]⁺;
¹H-NMR (400 MHz, DMSO-d₆): δ = 9.35 (s, 1H), 8.47 (s, 1H), 8.47 (s, 1H), 8.33 (s, 1H), 7.05 (s, 1H), 3.75-3.65 (m, 4H), 1.67-1.64 (m, 2H), 1.57-1.55 (m, 4H).

### Beispiel 12

### 2-[6-(Dimethylamino)pyrimidin-4-yl]-4-(1H-1,2,3-triazol-1-yl)-1,2-dihydro-3H-pyrazol-3-on

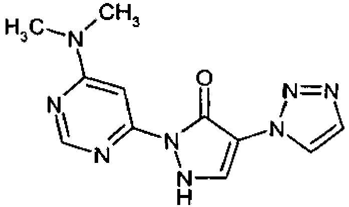

3.7 g (16.5 mmol) der Verbindung aus Beispiel 3A, 2.4 g der Verbindung aus Beispiel 7A und 0.6 g (3.3 mmol) p-Toluolsulfonsäure werden in einer Mischung aus 10 ml Ethanol und 5 ml THF 1 h lang bei 140°C in einer *single mode*-Mikrowelle (Emrys Optimizer) umgesetzt. Der dabei ausgefallene Niederschlag wird abfiltriert, der Filterrückstand mit einer Mischung aus Ethanol und Diethylether gewaschen und das Produkt im Vakuum getrocknet.
Ausbeute: 0.8 g (17% d. Th.)
LC-MS (Methode 10): R₁ = 0.47 min; MS (ESIpos): m/z = 273 [M+H]⁺;
¹H-NMR (400 MHz, DMSO-d₆): δ = 8.53 (s, 1H), 8.38 (s, 1H), 8.20 (s, 1H), 7.85 (s, 1H), 7.22 (s, 1H), 3.21 (s, 6H).

### Beispiel 13

### 2-(5-Brompyridin-2-yl)-4-(1H-1,2,3-triazol-1-yl)-1,2-dihydro-3H-pyrazol-3-on-Hydrochlorid

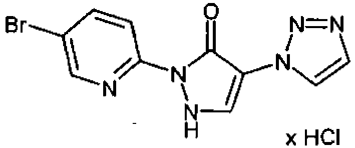

Ein Gemisch aus 280 mg (1.3 mmol) der Verbindung aus Beispiel 3A, 250 mg (1.3 mmol) der Verbindung aus Beispiel 10A und 46 mg (0.3 mmol) p-Toluolsulfonsäure in 5 ml THF wird 30 min bei 170°C in einer *single mode*-Mikrowelle (Emrys Optimizer) umgesetzt. Nach Zugabe von 2 ml Ameisensäure zur Reaktionslösung wird der ausgefallene Feststoff abfiltriert, mit 3 ml einer 4 N Lösung von Chlorwasserstoff in Dioxan verrührt, erneut abfiltriert, mit Diethylether gewaschen und im Vakuum getrocknet.
Ausbeute: 181 mg (40% d. Th.)
LC-MS (Methode 8): Rₜ = 1.50 min; MS (ESIpos): m/z = 306 [M+H]⁺;
¹H-NMR (400 MHz, DMSO-d₆): δ = 8.66 (s, 1H), 8.48 (s, 1H), 8.44 (s, 1H), 8.33-8.25 (m, 2H), 7.90(s, 1H).

### Beispiel 14

### 2-(6-Chlorpyrimidin-4-yl)-4-(1H-1,2,3-triazol-1-yl)-1,2-dihydro)-3H-pyrazol-3-on-Hydrochlorid

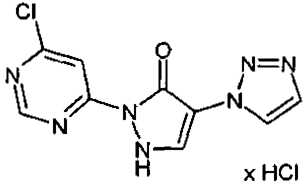

Ein Gemisch aus 7.3 g (34.6 mmol) der Verbindung aus Beispiel 3A und 6.0 g (41.5 mmol) der Verbindung aus Beispiel 7A in 70 ml Ethanol wird mit 1.1 ml (1.6 g, 13.8 mmol) TFA versetzt und 20 h bei 100°C gerührt. Der ausgefallene Feststoff wird abfiltriert und das Filtrat im Vakuum eingeengt. Der Rückstand wird in 100 ml Ethanol suspendiert, mit 30 ml 30%-iger Natriummethylat-Lösung in Methanol versetzt und 1.5 h bei RT gerührt. Nach Zugabe von 42 ml einer 4 N Lösung von Chlorwasserstoff in Dioxan (pH = 5-6) wird nach 30 min Rühren der Feststoff abfiltriert, zunächst mit Ethanol, dann mit Diethylether gewaschen und im Vakuum getrocknet. Eine weitere Reinigung erfolgt durch mehrmaliges Verrühren in Ethanol und Acetonitril. Anschließend werden 10 ml einer 4 N Lösung von Chlorwasserstoff in Dioxan hinzugefügt und 16 h bei RT gerührt. Das Produkt wird abfiltriert, zunächst mit Acetonitril, dann mit Diethylether gewaschen und im Vakuum getrocknet.
Ausbeute: 7.9 g (76% d. Th.)
LC-MS (Methode 8): Rₜ = 1.20 min; MS (ESIpos): m/z = 264 [M+H]⁺;
¹H-NMR (400 MHz, DMSD-d₆); δ = 8.97 (s, 1H), 8.59 (s, 1H), 8.47 (s, 1H), 8.43 (s, 1H), 7.89 (s, 1H).

Die in Tabelle 5 aufgeführten Verbindungen werden aus den entsprechenden Edukten analog der angegebenen Beispiele hergestellt:

**Tabelle 5**

| **Beispiel Nr.** | **Struktur** | **Edukte; Darstellung analog [Beispiel]; Ausbeute (% d. Th.)** | **MS (ESI) [M+H]⁺; LC-MS/ HPLC: R, (Methode)** | **¹H-NMR (400 MHz, DMSO-d₆)** |
|---|---|---|---|---|
| **15** | 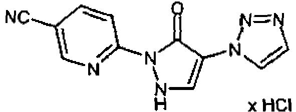 | 3A, 19A [68] 11% | m/z = 254; 2.41 min (Methode 1) | δ = 9.00 (s, 1H), 8.62 (s, 1H), 8.58-8.41 (m, 3H), 7.91 (s, 1H). |
| **16** | 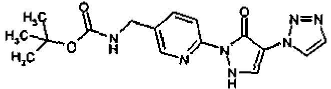 | 3 A, 24A [68] 3% | m/z = 358; 0.91 min (Methode 10) | δ = 8.43 (s, 1H), 8.38 (d, 1H), 8.34 (s, 1H), 8.22 (d, 1H), 7.92 (d, 1H), 7.89 (s, 1H), 7.51 (t, 1H), 4.19 (d, 2H), 1.39 (s, 9H). |
| **17** | 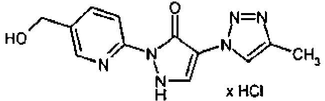 | 20A, 4A [69] 9% | m/z = 273; 2.33 min (Methode 1) | δ = 8.44 (s, 1H), 8.29 (s, 1H), 8.21 (s, 1H), 8.14 (s, 1H), 7.99 (s, 1H), 4.58 (s, 2H), 2.32 (s, 3H). |
| **18** | 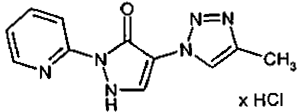 | 20A [69] 6% | m/z = 243; 2.50 min (Methode 1) | δ = 8.52 (d, 1H), 8.32 (s, 1H), 8.25 (d, 1H), 8.15 (s, 1H), 8.09 (t, 1H), 7.40 (dd, 1H), 2.31 (s, 3H). |
| **19** | 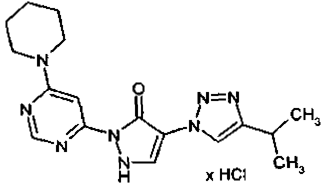 | 48A, 8A [69] 12% | m/z = 355; 3.88 min (Methode 11) | δ = 8.52 (s, 1H), 8.20 (s, 1H), 8.14 (s, 1H), 7.42 (s, 1H), 3.75-3.71 (m, 4H), 3.07-3.03 (m, 1H), 1.73-1.52 (m, 6H), 1.28 (d, 6H). |
| **20** | 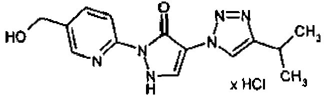 | 48A, 4A [69] 15% | m/z= 301; 2.71 min (Methode 1) | δ = 8.45 (s, 1H), 8.32 (s, 1H), 8.21 (d, 1H), 8.16 (s, 1H), 8.01 (s, 1H), 4.58 (s, 2H), 3.09-3.06 (m, 1H), 1.28 (d, 6H). |
| **21** | 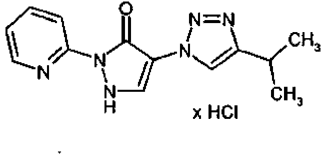 | 48A [69] 11% | m/z = 271; 2.70 min (Methode 1) | δ = 8.52 (d, 1H), 8.33 (s, 1H), 8.26 (d, 1H), 8.15 (s, 1 H), 8.07 (t, 1H), 7.41 (dd, 1H), 3.10-3.07 (m, 1H), 1.28 (d, 6H). |
| **22** | 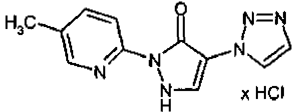 | 3A, 20A [68] 26% | m/z =243; 1.27 min (Methode 8) | δ = 8.43 (s, 1H), 8.34 (d, 2H), 8.14 (d, 1H), 7.92-7.90 (m, 2H), 2.36 (s, 3H). |
| **23** | 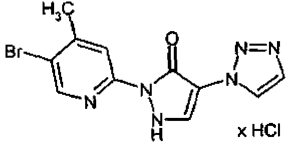 | 3A, 23A [68] 10% | m/z = 321; 0.93 min (Methode 10) | δ = 8.61 (s, 1H), 8.44-8.41 (m, 2H), 8.30 (s, 1H), 7.90 (s, 1H), 2.47 (s, 3H). |
| **24** | 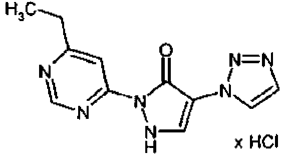 | 3A, 28A [68] 7% | m/z = 258; 1.15 min (Methode 10) | δ = 9.05 (s, 1H), 8.63 (s, 1H), 8.45 (d, 1H), 8.30 (s, 1H), 7.91 (d, 1H), 2.85 (q, 2H), 1.27 (t, 3H). |
| **25** | 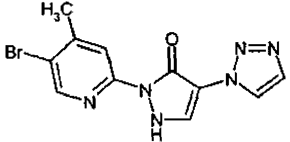 | 3A, 23A [68] 17% | m/z = 321; 0.92 min (Methode 10) | δ = 8.61 (s, 1H), 8.48-8.42 (m, 2H), 8.31 (s, 1H), 7.91 (s, 1H), 2.47 (s, 3H). |

### Beispiel 26

### 6-[5-Oxo-4-(1H-1,2,3-triazol-1-yl)-2,5-dihydro-1H-pyrazol-1-yl]pyridin-3-carbonsäure-tert.-butylester-Hydrochlorid

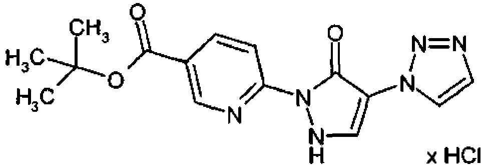

3.2 g (15.0 mmol) der Verbindung aus Beispiel 3A werden in 100 ml Ethanol vorgelegt. Man gibt 3.1 g (15.0 mmol) der Verbindung aus Beispiel 22A und 571 mg (3.0 mmol) p-Toluolsulfonsäure-Monohydrat hinzu und rührt 16 h unter Rückfluss. Man engt das Gemisch dann ein und reinigt den Rückstand mittels präparativer HPLC (RP18-Säule; Laufmittel: Acetonitrit/Wasser-Gradient unter Zusatz von 0.1% TFA). Man vereinigt die produkthaltigen Fraktionen, entfernt den Großteil des Lösungsmittels und filtriert den entstandenen Feststoff ab. Man trocknet diesen im Hochvakuum und versetzt anschließend mit einer 4 N Lösung von Chlorwasserstoff in Dioxan. Man rührt 1 h bei RT, filtriert den Feststoff ab und trocknet im Hochvakuum.
Ausbeute: 1.6 g (28% d. Th.)
LC-MS (Methode 1): Rₜ = 3.32 min; MS (ESIpos): m/z = 329 [M+H]⁺;
¹H-NMR (400 MHz, DMSO-d₆): δ = 8.94 (s, 1H), 8.53 (s, 1H), 8.50-8.40 (m, 3H), 7.91 (s, 1H), 1.58 (s, 9H).

### Beispiel 27

### 6-[5-Oxo-4-(1H-1,2,3-triazol-1-yl)-2,5-dihydro-1H-pyrazol-1-y1]pyridin-3-carbon säure-Hydrochlorid

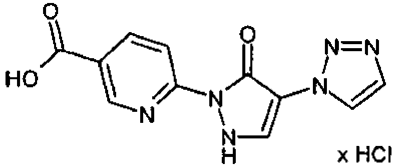

50 mg (0.1 mmol) der Verbindung aus Beispiel 26 werden in 1 ml einer 1:1 -Mischung aus Dichlormethan und TFA gelöst und 1 h bei RT gerührt. Die Reaktionslösung wird im Vakuum aufkonzentriert, der Rückstand in 2 ml 1 N Salzsäure suspendiert und anschließend lyophilisiert.
Ausbeute: 42 mg (99% d. Th.)
LC-MS (Methode 9): Rₜ = 0.82 min; MS (ESIpos): m/z = 273 [M+H]⁺;
¹H-NMR (400 MHz, DMSO-d₆): δ = 8.99 (s, 1H), 8.53 (s, 1H), 8.51-8.41 (m, 3H), 7.91 (s, 1H).

### Beispiel 28

### 6-[5-Oxo-4-(1H-1,2,3-triazol-1-yl)-2,5-dihydro-1H-pyrazol-1-yl]pyridin-3-carbonsäure

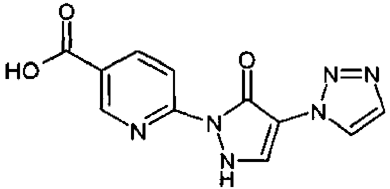

491 mg (1.8 mmol, 80% Reinheit) der Verbindung aus Beispiel 3A und 289 mg (1.6 mmol) 6-Hydrazinonicotinsäureethylester [Herstellung siehe WO 2006/114213] werden in 10 ml Essigsäure 12 h bei RT gerührt. Zur Reaktionsmischung werden danach nochmals 120 mg (0.7 mmol) 6-Hydrazinonicotinsäureethylester gegeben und erneut 13 h bei RT gerührt. Nach weiteren zwei Tagen Stehen bei RT wird die Reaktionslösung im Vakuum aufkonzentriert, der Rückstand mit Essigsäureethylester aufgenommen, mit gesättigter Natriumhydrogencarbonat-Lösung neutral gewaschen, die organische Phase über Natriumsulfat getrocknet, filtriert und am Rotationsverdampfer eingeengt. Das so erhaltene Zwischenprodukt wird in 10 ml Ethanol gelöst, mit 0.3 ml (1.8 mmol) 30%-iger Natriummethylat-Lösung in Methanol versetzt und 17 h bei RT gerührt. Man stellt die Reaktionslösung mit 1 N Salzsäure auf pH 5 ein und rührt weitere 2 h bei RT nach. Es wird im Vakuum eingeengt, der Rückstand mit Acetonitril versetzt, der ausgefallene Niederschlag abfiltriert, der Filterrückstand mit Diethylether gewaschen und im Vakuum getrocknet. Der so erhaltene Ester wird mit 9.4 ml 0.1 M ethanolischer Kaliumhydroxid- Lösung versetzt und 16 h bei RT gerührt. Die Reaktionslösung wird mit 1 N Salzsäure auf pH 2 eingestellt, das Lösungsmittel am Rotationsverdampfer entfernt, der Rückstand mit Wasser aufgenommen und mit Essigsäureethylester extrahiert. Man filtriert die aus der wässrigen Phase ausfallenden Kristalle ab, wäscht den Filterrückstand mit Diethylether und trocknet das Produkt im Vakuum.
Ausbeute: 32 mg (7% d. Th.)
LC-MS (Methodel): Rₜ = 2.31 min; MS (ESIpos): m/z = 273 [M+H]';
¹H-NMR (400 MHz, DMSO-d₆): δ = 13.49 (br. s, 1H), 9.00 (s, 1H), 8.71-8.33 (m, 4H), 8.41 (s, 1H).

### Beispiel 29

### 6-[5-Oxo-4-[1H-1,2,3-triazol-1-yl)-2,5-dihydro-1H-pyrazol-1-yl]nicotinsäureethylester

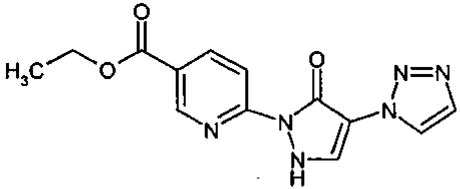

2.7 g (13.1 mmol) der Beispielverbindung 3A, 2.0 g (13.1 mmol) der Beispielverbindung 6A und 1.1 g (6.5 mmol) p-Toluolsulfonsäure werden in 50 ml Ethanol 16 h unter Rückfluss gerührt. Zur abgekühlten Reaktionsmischung werden 50 ml DMF gegeben und dann weitere 10 h bei 130°C Badtemperatur gerührt. Die Reaktionslösung wird im Vakuum aufkonzentriert und der Rückstand mit 10 ml Ethanol und 1 ml konz. Schwefelsäure versetzt. Man rührt 12 h in der Siedehitze nach. Zur abgekühlten Reaktionsmischung gibt man Wasser hinzu, filtriert den Feststoff ab, wäscht den Filterrückstand mit Ethanol nach und trocknet das Produkt im Vakuum.
Ausbeute: 0.14 g (3% d. Th.)
LC-MS (Methode 9): R, = 2.89 min; MS (ESIpos): m/z = 300 [M+H]⁺;
¹H-NMR (400 MHz, DMSO-d₆): δ = 9.00 (s, 1H), 8.57-8.54 (m, 1H), 8.53-8.46 (m, 2H), 8.45 (s, 1H), 7.91 (s, 1H), 4.38 (q, 2H), 1.35 (t, 3H).

### Beispiel 30

### 2-[5-(Aminomethyl)pyridin-2-yl]-4-(1H-1,2,3-triazol-1-yl)-1,2-dihydro-3H-pyrazol-3-on-Hydrochlorid

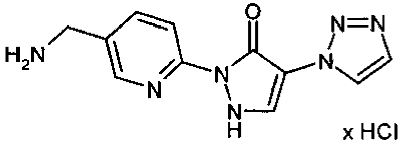

83 mg (0.2 mmol) der Beispielverbindung 16 werden in 5 ml einer 4 M Lösung von Chlorwasserstoff in Dioxan 2 h bei RT gerührt. Man konzentriert danach im Vakuum auf und chromatographiert den Rückstand mittels präparativer HPLC (RP18-Säule; Gradient: Acetonitril/Wasser unter Zusatz von 0.1% TFA). Man engt die vereinigten Produktfraktionen ein und versetzt den Rückstand mit 2 ml 1 N Salzsäure. Die resultierende Suspension wird gefriergetrocknet. Das Lyophilisat wird in Ethanol verrührt, der Feststoff abfiltriert und die Kristalle im Vakuum getrocknet.
Ausbeute: 14 mg (14% d. Th.)
LC-MS (Methode 8): Rₜ = 0.76 min; MS (ESIpos): m/z = 258 [M+H]⁺;
¹H-NMR (400 MHz, DMSO-d₆): δ = 8.64 (s, 1 H), 8.53 (s, 3H), 8.47 (s, 2H), 8.31 (s, 1H), 8.18 (s, 1H), 7.91 (s, 1H), 4.12-4.10 (m, 2H).

### Beispiel 31

### 2-(6-Morpholin-4-ylpyrimidin-4-yl)-4-(1H-1,2,3-triazol-1-yl)-1,2-dihydro-3H-pyrazol-3-on

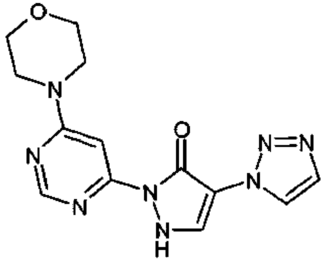

1.9 g (8.8 mmol) der Verbindung aus Beispiel 3A und 1.9 g (9.7 mmol) der Verbindung aus Beispiel 16A werden in 25 ml Essigsäureethylester vorgelegt und bei RT mit 504 mg (4.4 mmol) TFA versetzt. Man rührt 16 h unter Rückfluss, kühlt dann auf 5°C ab und rührt weitere 2 h nach. Man filtriert den entstandenen Feststoff ab, wäscht mit Essigsäureethylester und trocknet zunächst an der Luft und danach im Hochvakuum. Man erhält 1.7 g Produkt.

Die Mutterlauge wird mit der Waschlösung vereinigt und das Lösungsmittel entfernt. Der Rückstand (2.4 g) enthält laut LC-MS noch das Intermediat 3-[2-(6-Morpholin-4-ylpyrimidin-4-yl)-hydrazino]-2-(1*H*-1,2,3-triazol-1-yl)prop-2-ensäureethylester (Zwischenstufe der Cyclisierung), welches direkt für die Herstellung von Beispiel 72 verwendet wird *(siehe dort).*
Ausbeute: 1.7 g (61% d. Th.)
LC-MS (Methode 9): Rₜ = 0.90 min; MS (ESIpos): m/z = 31 [M+H]⁺;
¹H-NMR (400 MHz, DMSO-d₆): δ = 8.42 (s, 1H), 8.38 (s, 1H), 8.01 (s, 1H), 7.73 (s, 1H), 7.70 (s, 1 H), 3.71-3.65 (m, 4H), 3.57-3.51 (m, 4H).

### Beispiel 32

### 2-(6-Morpholin-4-ylpyrimidin-4-yl)-4-(1H-1,2,3-triazol-1-yl)-1,2-dihydro-3H-pyrazol-3-on-Hydrochlorid

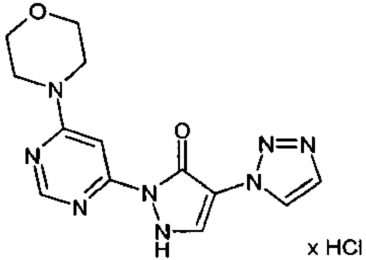

*Charge 1:* 1.7 g (5.4 mmol) der Verbindung aus Beispiel 31 werden mit 7.5 ml einer 4 N Lösung von Chlorwasserstoff in Dioxan versetzt. Man rührt bei RT, versetzt mit 5 ml Dioxan und rührt 16 h bei RT. Man filtriert den Feststoff ab und wäscht mit 5 ml Dioxan. Man trocknet 16 h im Hochvakuum, versetzt dann mit 10 ml Methanol und rührt 1 h bei RT. Der Feststoff wird abfiltriert, mit 4 ml Methanol gewaschen und im Hochvakuum getrocknet. Man erhält 1.6 g der Titelverbindung.

*Charge 2:* Eine weitere Menge der Titelverbindung wird wie folgt gewonnen: Man löst den bei der Synthese von Beispielverbindung 31 erhaltenen Rückstand aus der Mutterlauge (2.4 g), welcher die Ring-offene Zwischenstufe der Cyclisierung, 3-[2-(6-Morpholin-4-ylpyrimidin-4-yl)hydrazinol-2-(1*H*-1,2,3-triazol-1-yl)prop-2-ensäureethylester, enthält, in 12 ml Ethanol und versetzt unter Rühren bei RT mit 1.5 ml 30%-iger Natriummethylat-Lösung in Methanol. Man rührt 45 min bei RT nach, stellt dann mit 2 N Salzsäure auf pH 5 ein und rührt weitere 16 h bei RT nach. Man kühlt auf 10°C ab, filtriert den Feststoff ab und wäscht mit 3.5 ml Dioxan. Man trocknet 16 h im Hochvakuum, versetzt dann mit 5 ml Methanol und rührt 1 h bei RT nach. Man filtriert den Feststoff ab, wäscht mit 2 ml Methanol, trocknet im Hochvakuum und erhält so weitere 997 mg der Titelverbindung.
Ausbeute: zusammen 2.6 g (83% d. Th.)
LC-MS (Methode 6): Rₜ = 0.89 min; MS (ESIpos): m/z = 315 [M+H]⁺;
¹H-NMR (400 MHz, DMSO-d₆): δ = 8.54 (s, 1H), 8.39 (s, 1H), 8.28 (s, 1H), 7.88 (s, 1H), 7.42 (s, 1H), 3.71 (s, 8H).

Die in Tabelle 7 aufgeführten Verbindungen werden aus den entsprechenden Edukten analog der angegebenen Beispiele hergestellt. Statt Campher-10-sulfonsäure kann auch Toluol-4-sulfonsäure-Monohydrat eingesetzt werden.

**Tabelle 7**

| **Beispiel Nr.** | **Struktur** | **Edukte; Darstellung analog [Beispiel]**; **Ausbeute (% d. Th.)** | **MS (ESI) [M+H]⁺; LC-MS/ HPLC: Rₜ (Methode)** | **¹H-NMR (DMSO-d₆)** |
|---|---|---|---|---|
| **33** | 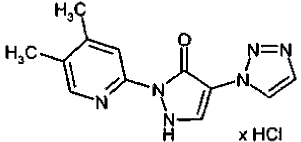 | 44A, 26A 68 [68] 27% | m/z = 257; 1.32 min (Methode 8) | (400 MHz): δ = 8.42 (s, 1H), 8.25 (s, 2H), 8.05 (s, 1H), 7.89 (s, 1H), 2.38 (s, 3H), 2.27 (s, 3H). |
| **34** | 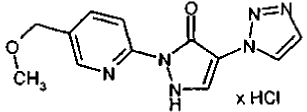 | 3A, 29A [68] 85% | m/z = 273; 0.64 min (Methode 10) | (500 MHz): δ = 8.48 (s, 1H), 8.44 (s, 1H), 8.37 (s, 1H), 8.27 (s, 1H), ^{:} 8.02 (d, 1H), 7.90 (s, 1H), 4.50 (s, 2H), 3.35 (s, 3H). |

### Beispiel35

### 2-[5-(tert.-Butoxymethyl)pyridin-2-yl]-4-(1H-1,2,3-triazol-1-yl)-1,2-dihydro-3H-pyrazol-3-on-Hydrochlorid

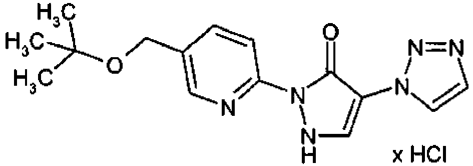

631 mg (3.0 mmol) der Verbindung aus Beispiel 3A und 586 mg (3.0 mmol) der Verbindung aus Beispiel 18A werden in 10 ml Ethanol vorgelegt. Man gibt 114 mg (0.6 mmol) p-Toluolsulfonsäure-Monohydrat hinzu und rührt 16 h bei RT. Man entfernt dann das Lösungsmittel und reinigt den Rückstand mittels präparativer HPLC (RP18-Säule; Laufmittel: Acetonitril/Wasser-Gradient unter Zusatz von 0.1% TFA). Die Produktfraktionen werden verein igt, eingeengt und der Rückstand im Hochvakum getrocknet. Man gibt 10 ml einer 4 N Lösung von Chlorwasserstoff in Dioxan hinzu und rührt 1 h bei RT. Man filtriert den Feststoff ab, wäscht mit *tert.*-Butylmethylether und trocknet im Hochvakuum.
Ausbeute: 260 mg (25% d. Th.)
LC-MS (Methode 8): Rₜ = 1.77 min; MS (ESIpos): m/z = 315 [M+H]⁺;
¹H-NMR (400 MHz, DMSO-d₆): δ = 8.47-8.42 (m, 2H), 8.35 (s, 1H), 8.22 (d, 1H), 7.99 (dd, 1H), 7.90 (s, 1H), 4.50 (s, 2H), 1.25 (s, 9H).

### Beispiel 36

### 4-(1H-1,2,3-Triazol-1-yl)-2-[4-(trifluormethyl)pyridin-2-yl]-1,2-dihydro-3H-pyrazol-3-on-Hydrochlorid

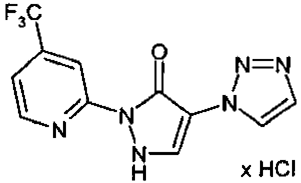

631 mg (3.0 mmol) der Verbindung aus Beispiel 3A und 531 mg (3.0 mmol) der Verbindung aus Beispiel 25A werden in 10 ml Ethanol vorgelegt. Man gibt 114 mg (0.6 mmol) p-Toluolsulfonsäure-Monohydrat hinzu und rührt 16 h unter Rückfluss. Man engt dann ein und reinigt den Rückstand mittels präparativer HPLC (RP18-Säule; Laufmittel: Acetonitril/Wasser-Gradient unter Zusatz von 0.1% TFA). Die Produktfraktionen werden vereinigt, das Lösungsmittel entfernt und der Rückstand im Hochvakuum getrocknet. Man versetzt mit 20 ml einer 4 N Lösung von Chlorwasserstoff in Dioxan und rührt 1 h bei RT. Der Feststoff wird abfiltriert, zweimal mit *tert*.-Butylmethylether gewaschen und im Hochvakuum getrocknet.
Ausbeute: 231 mg (23% d. Th.)
LC-MS (Methode 8): Rₜ = 1.65 min; MS (ESIpos): m/z = 297 [M+H]⁺;
¹H-NMR (400 MHz, DMSO-d₆): δ = 8.81 (d, 1H), 8.70 (s, 1H), 8.59 (s, 1H), 8.47 (s, 1H), 7.92 (s, 1H), 7.77(d, 1H).

### Beispiel 37

### 2-[5-(2,2-Dimethylpropoxy)pyridin-2-yl]-4-(1H-1,2,3-triazol-1-yl)-1,2-dihydro-3H-pyrazol-3-on

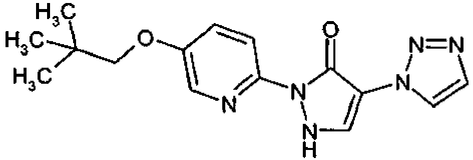

171 mg (0.8 mmol) der Verbindung aus Beispiel 3A und 455 mg (Reinheit 35%, 0.8 mmol) der Verbindung aus Beispiel 12A werden in 7 ml Ethanol vorgelegt. Man gibt 31 mg (0.2 mmol) p-Toluelsulfonsäure-Monohydrat hinzu und rührt 64 h unter Rückfluss. Man engt danach ein und reinigt den Rückstand mittels präparativer HPLC (RP18-Säule; Laufmittel: Acetonitril/Wasser-Gradient unter Zusatz von 0.1% TFA). Die Produktfraktionen werden vereinigt, das Lösungsmittel entfernt und der Rückstand im Hochvakuum getrocknet.
Ausbeute: 28 mg (11% d. Th.)
LC-MS (Methode 10): Rₜ = 1.17 min; MS (ESIpos): m/z= 315 [M+H]⁺;
¹H-NMR (500 MHz, DMSD-d₆): δ = 8.42 (s, 1H), 8.29 (s, 1H), 8.23 (s, 1 H), 8.18-8.08 (m, 1H), 7.90 (s, 1H), 7.69 (dd, 1H), 3.78 (s, 2H), 1.02 (s, 9H).

### Beispiel 38

### 2-[5-(2,2-Dimethylpropoxy)pyridin-2-yl]-4-(1H-1,2,3-triazol-1-yl)-1,2-dihydro-3H-pyrazol-3-on-Hydrochlorid

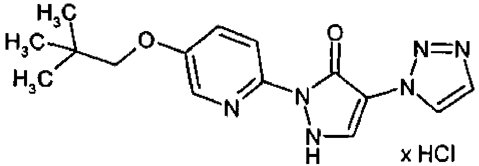

Man versetzt 22 mg (0.1 mmol) der Verbindung aus Beispiel 37 mit 2 ml einer 4 N Lösung von chlorwasserstoff in Dioxan und rührt 1 h bei RT. Der Feststoff wird abfiltriert und im Hochvakuum getrocknet.
Ausbeute: 17 mg (97% d. Th.)
LC-MS (Methode 10): Rₜ= 1.17 min; MS (ESIpos): m/z = 315 [M+H]⁺;
¹H-NMR (500 MHz, DMSO-d₆): δ = 8.42 (s, 1H), 8.29 (s, 1H), 8.23 (s, 1H), 8.18-8.08 (m, 1H), 7.90 (s, 1H), 7.69 (dd, 1 H), 3.78 (s, 2H), 1.02 (s, 9H).

### Beispiel 39

### 1-[2-(6-Morpholin-4-ylpyrimidin-4-yl)-3-oxo-2,3-dihydro-1H-pyrazol-4-yl]-1H-1,2,3-triazol-4-carbonitril-Trifluoracetat

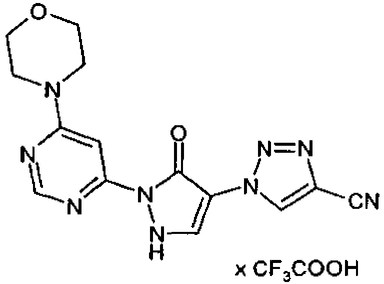

250 mg (1.1 mmol) der Verbindung aus Beispiel 41A, 207 mg (1.1 mmol) der Verbindung aus Beispiel 16A und 40 mg (0.2 mmol) p-Toluolsulfonsäure-Monohydrat werden in 10 ml THF vorgelegt. Man setzt in einer *single* mode-Mikrowelle (CEM Explorer) zunächst 3.5 h bei 120°C, dann 4 h bei 130°C um. Man lässt auf RT abkühlen, versetzt das Gemisch mit 5 ml Acetonitril und lässt 24 h lang stehen. Man dekantiert den Überstand ab und wäscht den Niederschlag einmal mit Acetonitril. Die Waschlösung wird mit dem abdekantierten Überstand vereinigt und eingeengt. Der so erhaltene Rückstand wird mittels präparativer HPLC (RP18-Säule; Laufmittel: Acetonitril/Wasser-Gradient unter Zusatz von 0.1% TFA) gereinigt. Die produkthaltigen Fraktionen werden vereinigt und eingeengt. Der feste Rückstand wird zweimal in einem Gemisch aus wenig *tert.-*Butylmethylether und einigen Tropfen Acetonitril verrührt. Der Überstand wird jeweils abdekantiert und der Feststoff zuletzt im Hochvakuum getrocknet.
Ausbeute: 15 mg (3% d. Th.)
LC-MS (Methode 4): Rₜ = 1.57 mine MS (ESIpos): m/z = 340 [M+H]⁺;
¹H-NMR (400 MHz, DMSO-d₆): δ = 8.58 (s, 1H), 8.24 (s, 1H), 7.42 (s, 1H), 3.80-3.63 (m, 8H).

### Beispiel 40

### 1-[2-(6-Morpholin-4-ylpyrimidin-4-yl)-3-oxo-2,3-dihydro-1H-pyrazol-4-yl]-1H-1,2,3-triazol-4-carbonitril-Hydrochlorid

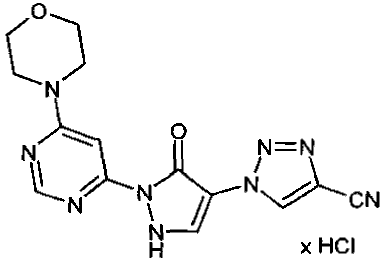

18 mg (0.04 mmol) der Verbindung aus Beispiel 39 werden mit 3 ml einer 4 N Lösung von Chlorwasserstoff in Dioxan versetzt und 1 h bei RT gerührt. Man filtriert den Feststoff ab, wäscht mit *tert*.-Butylmethylether und trocknet im Hochvakuum.
Ausbeute: 15 mg (97% d. Th.)
LC-MS (Methode 8): Rₜ = 1.38 min; MS (ESIpos): m/z = 340 [M+H]⁺;
¹H-NMR (400 MHz, DMSO-d₆): δ= 8.58 (s, 1H], 8.25 (s, 1H), 7.41 (s, 1H), 3.82-3.57 (m, 8H).

### Beispiel 41

### 2-[6-(3-Hydroxyazetidin-1-yl)pyrimidin-4-yl]-4-(1H-1,2,3-triazol-1-yl)-1,2-dihydro-3H-pyrazol-3-on

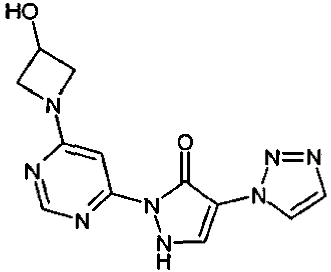

348 mg (1.7 mmol) der Verbindung aus Beispiel 3A und 300 mg (1.7 mmol) der Verbindung aus Beispiel 11A werden in einem Gemisch aus 6 ml THF und 6 ml Ethanol vorgelegt. Man gibt 63 mg (0.3 mmol) p-Toluolsulfonsäure-Monohydrat hinzu und setzt in einer *single mode*-Mikrowelle (CEM Explorer) für 1.5 h bei 130°C um. Man lässt auf RT abkühlen, gibt weitere 50 mg der Verbindung aus Beispiel 3A sowie eine Spatelspitze p-Toluolsulfonsäure-Monohydrat hinzu und erhitzt für 1 h auf 150°C. Man lässt auf RT abkühlen, dekantiert vom Feststoff ab und wäscht den Feststoff mehrmals mit THF (Feststoff-Charge 1). Der abdekantierte Überstand und die Waschlösung werden vereinigt, eingeengt und der erhaltene Rückstand mit 5 ml Ethanol und 5 ml THF sowie einer Spatelspitze p-Toluolsulfonsäure-Monohydrat versetzt. Man setzt abermals in einer *single mode*-Mikrowelle (CEM Explorer) für 2 h bei 150°C um. Man lässt auf RT abkühlen und filtriert den gebildeten Feststoff ab (Feststoff-Charge 2). Das Filtrat wird eingeengt, mit 3 ml DMSO versetzt und mittels präparativer HPLC (RP18-Säule; Laufmittel: Acetonitril/Wasser-Gradient unter Zusatz von 0.1% TFA) gereinigt. Die produkthaltigen Fraktionen werden vereinigt und eingeengt. Man löst den Rückstand in Ethanol, versetzt mit etwas THF und konzentriert das Gemisch unter Bildung eines Niederschlages auf. Der Feststoff wird abfiltriert und mit THF gewaschen (Feststoff-Charge 3). Man vereinigt die drei erhaltenen Feststoff-Chargen und trocknet im Hochvakuum.
Ausbeute: 108 mg (22% d. Th.)
LC-MS (Methode 8): Rₜ = 1.00 min; MS (ESIpos): m/z = 301 [M+H]⁺;
¹H-NMR (400 MHz, DMSO-d₆): δ = 8.47 (s, 1H), 8.36 (s, 1H), 8.18 (s, 1H), 7.84 (s, 1H), 6.97 (s, 1 H), 5.92 (d, 1H), 4.69-4.49 (m, 1H), 4.41-4.33 (m, 2H), 3.95-3.86 (m, 2H).

### Beispiel 42

### 2-[6-(3-Hydroxyazetidin-1-yl)pyrimidin-4-yl]-4-(1H-1,2,3-triazol-1-yl)-1,2-dihydro-3H-pyrazol-3-on-Hydrochlorid

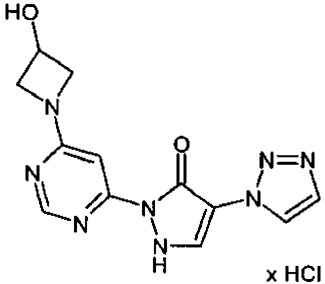

105 mg (0.4 mmol) der Verbindung aus Beispiel 41 werden mit 3 ml einer 4 N Lösung von Chlorwasserstoff in Dioxan versetzt und 1 h bei RT gerührt. Man filtriert den Feststoff ab, wäscht mit *tert.*-Butylmethylether und trocknet im Hochvakuum.
Ausbeute: 117 mg (99% d. Th.)
LC-MS (Methode 8): Rₜ = 0.99 min; MS (ESIpos): m/z = 301 [M+H]⁺;
¹H-NMR (400 MHz, DMSO-d₆): δ = 8.50 (s, 1H), 8.38 (s, 1H), 8.22 (s, 1H), 7.87 (s, 1H), 6.95 (s, 1H), 4.68-4.60 (m, 1H), 4.43-4.35 (m, 2H), 3.97-3.88 (m, 2H).

### Beispiel, 43

### 1-{2-[5-(2,2-Dimethylpropoxy)pyridin-2-yl]-3-oxo-2,3-dihydro-1H-pyrazol-4-yl}-1H-1,2,3-triazol-4-carbonitril-Hydrochlorid

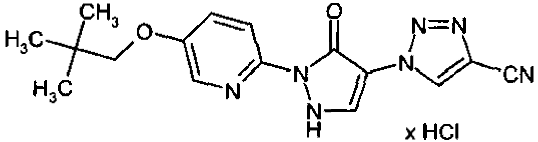

150 mg (0.6 mmol) der Verbindung aus Beispiel 41A werden in 2.5 ml Ethanol vorgelegt. Man gibt 166 mg (0.6 mmol, Reinheit 75%) der Verbindung aus Beispiel 12A und 24 mg (0.1 mmol) p-Toluolsulfonsäure-Monohydrat hinzu und rührt 16 h unter Rückfluss. Die Reaktionslösung wird direkt mittels präparativer HPLC (RP18-Säule; Laufmittel: Acetonitril/Wasser-Gradient unter Zusatz von 0.1% TFA) gereinigt. Die produkthaltigen Fraktionen werden vereinigt und eingeengt. Der erhaltene Rückstand wird mit 1 ml einer 4 N Lösung von Chlorwasserstoff in Dioxan versetzt und 1 h bei RT gerührt. Man engt ein und trocknet den Rückstand im Hochvakuum.
Ausbeute: 3 mg (1% d. Th.)
LC-MS (Methode 8): Rₜ = 2.42 min; MS (ESIpos): m/z = 340 [M+H]⁺;
¹H-NMR (400 MHz, DMSO-d₆): δ = 9.39 (s, 1H), 8.41 (s, 1H), 8.24 (d, 1H), 8.12 (d, 1H), 7.70 (dd, 1H), 3.78 (s, 2H), 1.03 (s, 9H).

### Beispiel 44

### 6-[4-(4-Cyano-1H-1,2,3-triazol-1-yl)-5-oxo-2,5-dihydro-1H-pyrazol-1-yl)pyridin-3-tert.-butylester-Hydrochlorid

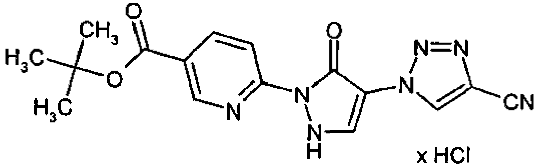

150 mg (0.6 mmol) der Verbindung aus Beispiel 41A werden in 2.5 ml Ethanol vorgelegt. Man gibt 178 mg (0.6 mmol) der Verbindung aus Beispiel 22A und 24 mg (0.1 mmol) p-Toluolsulfonsäure-Monohydrat hinzu und rührt 16 h unter Rückfluss. Die Reaktionslösung wird direkt mittels präparativer HPLC (RP18-Säule; Laufmittel: Acetonitril/Wasser-Gradient unter Zusatz von 0.1% TFA) gereinigt. Die produkthaltigen Fraktionen werden vereinigt, eingeengt und im Hochvakuum getrocknet. Der erhaltene Rückstand wird mit 5 ml einer 4 N Lösung von Chlorwasserstoff in Dioxan versetzt und 30 min bei RT gerührt. Der Feststoff wird abfiltriert, mit *tert*.-Butylmethylether gewaschen und im Hochvakuum getrocknet.
Ausbeute: 21 mg (8% d. Th.)
LC-MS (Methode 7): Rₜ = 1.96 min; MS (ESIpos): m/z = 354 [M+H]⁺;
¹H-NMR (400 MHz, DMSO-d₆): δ = 9.39 (s, 1H), 8.94 (s, 1H), 8.63 (s, 1H), 8.46 (s, 2H), 1.58 (s, 9H).

### Beispiel 45

### 2-[6-(3,5-Dimethylpiperidin-1-yl)pyrimidin-4-yl]-4-(1H-1,2,3-triaxol-1-yl)-1,2-dihydro-3H-pyrazol-3-on

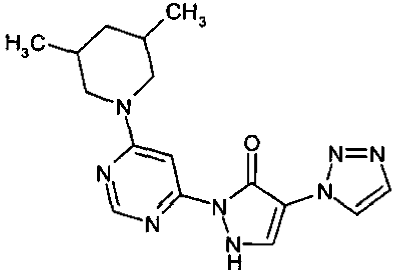

2.9 g (9.6 mmol) der Beispielverbindung 52A werden in 40 ml DMF gelöst und als Stammlösung bereitgestellt.

23 mg (0.1 mmol) 3,5-Dimethylpiperidin werden in 200 µl DMF vorgelegt und nacheinander mit 400 µl (0.1 mmol) der Stammlösung aus der Beispielverbindung 52A und 35 mg (0.3 mmol) Kaliumcarbonat versetzt. Man rührt die Reaktionsmischung 16 h bei 100°C. Zur Aufarbeitung wird die Suspension filtriert und das Filtrat mittels präparativer LC-MS (Methode 16) chromatographiert. Die Produktfraktionen werden im Vakuum aufkonzentriert und der Rückstand getrocknet.
Ausbeute: 3 mg (10% d. Th.)
LC-MS (Methode 16): Rₜ = 1.90 min; MS (ESIpos): m/z = 341 [M+H]⁺.

Analog zur Arbeitsvorschrift von Beispiel 45 werden die in Tabelle 8 aufgeführten Verbindungen aus 0.1 mmol der Beispielverbindung 52A und 0.1 mmol des entsprechenden sekundären Amins hergestellt:

**Tabelle 8**

| **Beispiel Nr.** | **Struktur** | **Ausbeute (% d.Th.)** | **MS (ESI) [M+H]⁺; LG-MS: Rₜ (Methode 16)** |
|---|---|---|---|
| **46** | 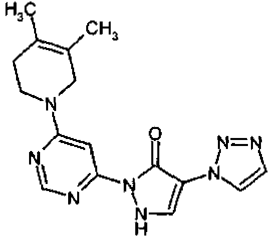 | 2% | m/z = 339; 1.88 min |
| **47** | 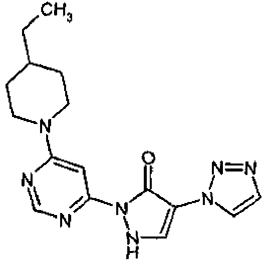 | 1% | m/z = 341; 1.91 min |
| **48** | 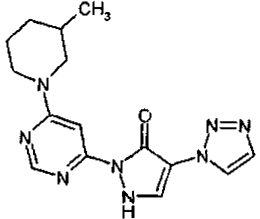 | 7% | m/z = 327; 1.77 min |
| **49** | 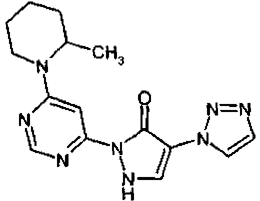 | 5% | m/z = 327; 1.77 min |
| **50** | 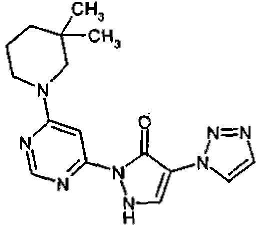 | 4% | m/z = 341; 1.82 min |
| **51** | 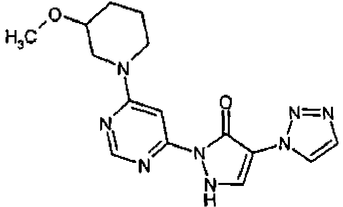 | 5% | m/z = 343; 1.54 min |
| **52** | 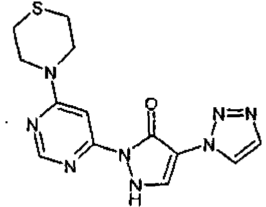 | 5% | m/z = 331; 1.58 min |
| **53** | 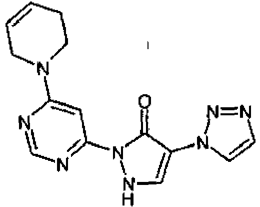 | 5% | m/z = 311; 1.63 min |
| **54** | 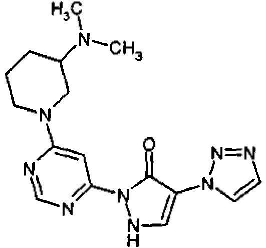 | 2% | m/z = 356; 1.18 min |
| **55** | 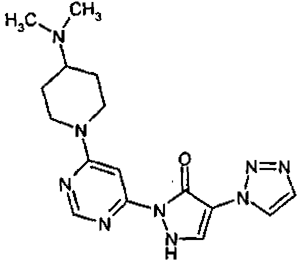 | 7% | m/z = 356; 1.18 min |
| **56** | 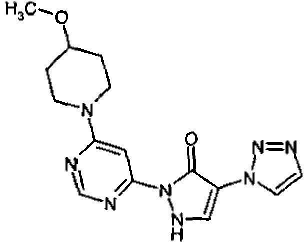 | 6% | m/z = 343; 1.53 min |
| **57** | 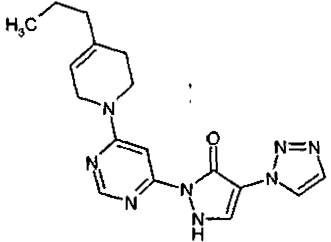 | 9% | m/z = 353; 2.01 min |
| **58** | 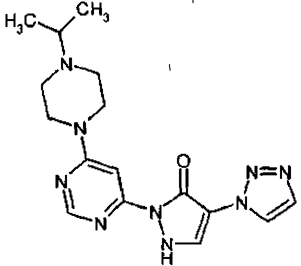 | 9% | m/z = 356; 1.18 min |
| **59** | 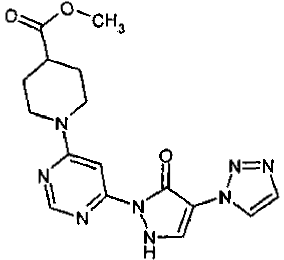 | 8% | m/z = 371; 1.60 min |
| **60** | 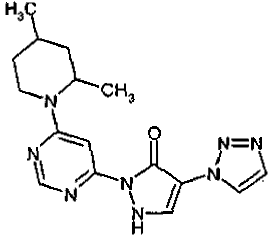 | 2% | m/z = 341; 1.81 min |
| **61** | 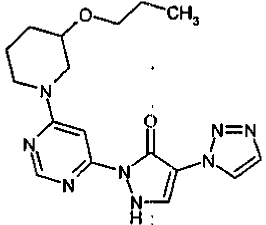 | 10% | m/z = 371; 1.79 min |
| **62** | 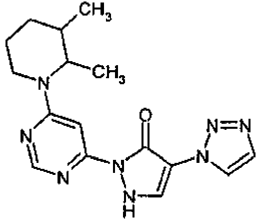 | 3% | m/z = 341; 1.88 min |
| **63** | 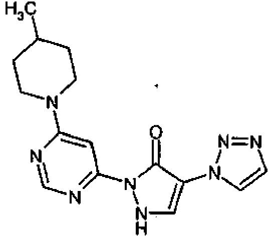 | 7% | m/z = 327; 1.78 min |
| **64** | 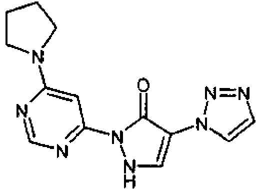 | 3% | m/z = 299; 1.51 min |
| **65** | 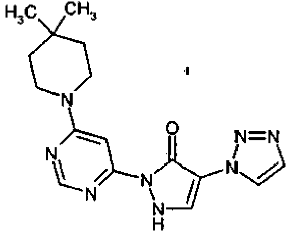 | 11% | m/z = 341; 1.87 min |
| **66** | 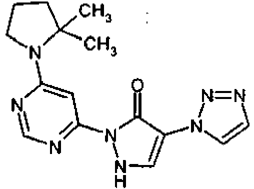 | 2% | m/z = 327; 1.79 min |

### Beispiel 67

### 2-(6-Methoxypyrimidin-4-yl)-4-(1H-1,2,3-triazol-1-yl)-1,2-dihydro-3H-pyrazol-3-on-Hydrochlorid

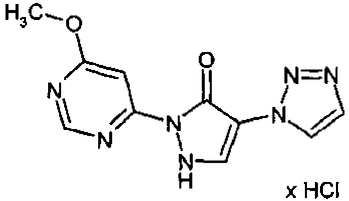

0.3 ml (6.6 mmol) Methanol werden in 15 ml Dioxan vorgelegt. Man gibt unter Rühren langsam 1.3 ml (2.7 mmol) einer 2 M Lösung der Phosphazen-Base P2-*tert*.-Butyl in THF hinzu und rührt 15 min bei RT. Anschließend gibt man 350 mg (1.3 mmol) der Verbindung aus Beispiel 52A hinzu und setzt 2 h bei 150°C in einer *single mode*-Mikrowelle (CEM Explorer) um. Man gibt dann weitere 2 ml (49.2 mmol) Methanol hinzu und setzt erneut für 2 h unter den gleichen Bedingungen in einer *single* mode-Mikrowelle (CEM Explorer) um. Nach dem Abkühlen engt man die Reaktionsmischung ein und reinigt den Rückstand über präparative HPLC (RP18-Säule; Laufmittel: Acetonitril/Wasser-Gradient unter Zusatz von 0.1% TFA). Die produkthaltigen Fraktionen werden vereinigt und am Rotationsverdampfer eingeengt. Der Rückstand wird im Hochvakuum getrocknet und anschließend mit 3 ml einer 4 N Lösung von Chlorwasserstoff in Dioxan versetzt. Man rührt 30 min bei RT, filtriert dann den Feststoff ab und trocknet im Hochvakuum.
Ausbeute: 60 mg (15% d. Th.)
LC-MS (Methode 7); Rₜ = 0.82 min; MS (ESIpos): m/z = 260 [M+H]⁺;
¹H-NMR (400 MHz, DMSO-d₆); δ = 8.80 (s, 1H), 8.59 (s, 1H), 8.43 (s, 1H), 7.90 (s, 1H), 7.70 (s, 1H), 4.00 (s, 3H).

### Beispiel 68 (Verleichsbeispiel)

### 1-[3-Oxo-2-(6-piperidin-1-ylpyrimidin-4-yl)-2,3-dihydro-1H-pyrazol-4-yl]-1H-imidazol-4-carbonitril-Hydrochlorid

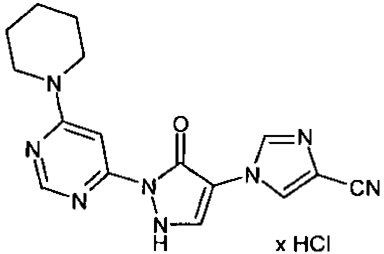

200 mg (0.9 mmol) der Verbindung aus Beispiel 45A und 165 mg (0.9 mmol) der Verbindung aus Beispiel 8A werden in 2 ml Ethanol gelöst und mit 29 mg (0.2 mmol) p-Toluolsulfonsäure versetzt. Es wird 48 h bei 90°C gerührt. Nach Abkühlen auf RT wird die Reaktionsmischung eingeengt und der Rückstand mittels präparativer HPLC (RP18-Säule; Laufmittel: Acetonitril/Wasser-Gradient unter Zusatz von 0.1% Ameisensäure) gereinigt. Die eingeengte Produktfraktion wird mit einem Überschuß an einer 4 N Lösung von Chlorwasserstoff in Dioxan und Diethylether versetzt, die ausgefallenen Kristalle abfiltriert, der Filterrückstand mit Diethylether gewaschen und das Produkt im Vakuum getrocknet.
Ausbeute: 18 mg (6% d. Th.)
HPLC (Methode 11): Rₜ = 3.64 min; MS (ESIpos): m/z = 337 [M+H]⁺;
¹H-MMR (400 MHz, DMSO-d₆): δ = 8.49 (s, 1H), 8.40 (s, 1H), 8.21 (s, 1H), 8.18 (s, 1H), 7.42 (s, 1H), 3.75-3.65 (m, 4H), 1.73-1.51 (m, 6H).

### Beispiel 69 (Vergleichsbeispiel)

### 2-[5-(Hydroxymethyl)pyridin-2-yl]-4-(1H-imidazol-1-yl)-1,2-dihydro-3H-pyrazol-3-on-Hydrochlorid

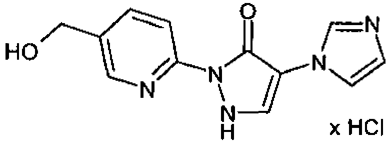

200 mg (1.0 mmol) der Verbindung aus Beispiel 42A und 133 mg (1.0 mmol) der Verbindung aus Beispiel 4A werden in 2 ml Ethanol gelöst und mit 44 mg (0.2 mmol) Campher-10-sulfonsäure versetzt. Es wird 12 h unter Rückfluss gerührt. Die abgekühlte Reaktionsmischung wird im Vakuum aufkonzentriert. Nach zweimaliger Reinigung mittels präparativer HPLC (RP18-Säule; Laufmittel: Acetonitril/Wasser-Gradient unter Zusatz von 0.1% Ameisensäure) wird die Produktfraktion mit 1 ml einer 4 N Lösung von Chlorwasserstoff in Dioxan versetzt, 1 h gerührt, das Lösungsmittel am Rotationsverdampfer vollständig entfernt und der Rückstand im Vakuum getrocknet.
Ausbeute: 86 mg (31% d. Th.)
LC-MS (Methode 1): Rₜ = 1.77 min; MS (ESIpos): m/z = 258 [M+H]⁺;
¹H-NMR (400 MHz, DMSO-d₆): δ = 9.50 (s, 1H), 8.51 (s, 1H), 8.47 (s, 1H), 8.29 (d, 1H), 8.09-8.01 (m, 2H), 7.87 (s, 1H), 3.58 (s, 2H).

### B. Bewertung der pharmakologischen Wirksamkeit

Die pharmakologischen Eigenschaften der erfindungsgemäßen Verbindungen können in folgenden Assays gezeigt werden:

### Abkürzungen:

| | |
|---|---|
| DMEM | Dulbecco's Modified Eagle Medium |
| FCS | Fetal Calf Serum |
| TMB | 3,3',5,5'-Tetramethylbenzidin |
| Tris | Tris(hydroxymethyl)-aminomethan |

### 1. In vitro-Tests zur Bestimmung der Aktivität und Selektivität von HIF-Prolyl-4-Hydroxylase-Inhibitoren

### 1.a) Hemmung der Aktivität von HIF-Prolylhydroxylase:

Hydroxylierter HIF bindet spezifisch an den von Hippel-Lindau Protein-Elongin B-Elongin C-Komplex (VBC-Komplex). Diese Interaktion tritt nur auf, wenn HIF an einem konservierten Prolylrest hydroxyliert ist. Sie ist die Grundlage für die biochemische Bestimmung der HIF-Prolylhydroxylase-Aktivität. Der Test wird wie beschrieben durchgerührt [Oehme F., Jonghaus W., Narouz-Ott L., Huetter J., Flamme 1., Anal. Biochem. 330 (1), 74-80 (2004)]:

Eine klare, mit NeutrAvidin HBC beschichtete 96-Loch-Mikrotiterplatte (Fa. Pierce) wird für 30 Minuten mit Blocker-Casein inkubiert. Anschließend wird die Platte dreimal mit je 200 µl Waschpuffer (50 mM Tris, pH 7.5, 100 mM NaCl, 10% (v/v) Blocker-Casein, 0.05% (v/v) Tween 20) pro Loch gewaschen. Das Peptid Biotin-DLDLEMLAPYIPMDDDFQL (Fa. Eurogentec, 4102 Seraing, Belgien) wird in einer Konzentration von 400 nM in 100 µl Waschpuffer zugegeben. Dieses Peptid dient als Substrat für die Prolylhydroxylierung und wird an die Mikrotiterplatte gebunden. Nach 60 Minuten Inkubation wird die Platte dreimal mit Waschpuffer gewaschen, 30 Minuten mit 1 mM Biotin in Blocker-Casein inkubiert und dann erneut dreimal mit Waschpuffer gewaschen.

Zur Durchführung der Prolylhydroxylase-Reaktion wird das an die Platte gebundene Peptidsubstrat 1 bis 60 Minuten mit einem Prolylhydroxylase-haltigen Zelllysat inkubiert. Die Reaktion findet in 100 µl Reaktionspuffer (20 mM Tris, pH 7.5, 5 mM KCI, 1.5 mM MgCl₂, 1 µM-1 mM 2-Oxoglutarat, 10 µM FeSO₄, 2 mM Ascorbat) bei Raumtemperatur statt. Die Reaktionsmischung enthält außerdem den zu testenden Hemmstoff der Prolylhydroxylase in verschiedenen Konzentrationen.

Die Testsubstanz wird bevorzugt, aber nicht ausschließlich bei Konzentrationen zwischen 1 nM und 100 µM eingesetzt, Durch dreimaliges Waschen der Platte mit Waschpuffer wird die Reaktion gestoppt.

Zur quantitativen Bestimmung der Prolylhydroxylierung wird ein Fusionsprotein, das sowohl Thioredoxin aus E. coli als auch den VBC-Komplex enthält, in 80 µl Bindungspuffer (50 mM Tris, pH 7.5, 120 mM NaCl) zugegeben. Nach 15 Minuten werden 10 µl einer Lösung von polykionalem Anti-Thioredoxin-Antikörper aus Kaninchen in Bindungspuffer zugefügt. Nach weiteren 30 Minuten setzt man 10 µl einer Lösung von mit Meerrettich-Peroxidase gekoppeltem Anti-Kaninchen-Immunglobulin in Bindungspuffer hinzu. Nach 30 Minuten Inkubation bei Raumtemperatur wird dreimal mit Waschpuffer gewaschen, um ungebundenen VBC-Komplex und Antikörper zu entfernen. Um die Menge an gebundenem VBC-Komplex zu bestimmen, wird 15 Minuten mit TMB inkubiert. Die Farbreaktion wird durch Zugabe von 100 µl 1 M Schwefelsäure beendet. Durch Messung der optischen Dichte bei 450 nm wird die Menge an gebundenem VBC-Komplex bestimmt. Sie ist proportional zur Menge an hydroxyliertem Prolin im Peptidsubstrat.

Zur Detektion der Prolylhydroxylierung kann alternativ ein mit Europium (Fa. Perkin Elmer) gekoppelter VBC-Komplex verwendet werden. In diesem Fall wird die Menge an gebundenem VBC-Komplex durch zeistaufgelöste Fluoreszenz bestimmt. Außerdem ist die Verwendung von mit [³⁵S]-Methionin markiertem VBC-Komplex möglich. Hierfür kann der radioaktiv markierte VBC-Komplex durch *in* vitro-Transkription-Translation in Retikulorytenlysat hergestellt werden.

Die Ausführungsbeispiele hemmen die Aktivität der HIF-Prolylhydroxylase in diesem Test mit einem IC₅₀-Wert von ≤ 30 µM. In der nachstehenden Tabelle 1 sind repräsentative IC₅₀-Werte zu den Ausführungsbeispielen wiedergegeben:

**Tabelle 1**

| **Beispiel Nr.** | **IC₅₀ [µM]** |
|---|---|
| 5 | 1.2 |
| 7 | 0.34 |
| 20 | 0.78 |
| 25 | 1.1 |
| 26 | 0.05 |
| 32 | 0.49 |
| 36 | 1.9 |
| 38 | 0.19 |
| 52 | 0.74 |
| 63 | 0.36 |

### 1.b) Zellulärer, funktioneller in vitro-Test:

Die Quantifizierung der Wirksamkeit der erfindungsgemäßen Verbindungen erfolgt mit Hilfe einer rekombinanten Zelllinie. Die Zelle leitet sich ursprünglich von einer humanen Lungencarcinom-Zelllinie ab (A549, ATCC: American Type Culture Collection, Manassas, VA 20108, USA). Die Testzelllinie wird stabil mit einem Vektor transfiziert, der das Reportergen der *Pholinus pyralis-*Luciferase (im folgenden Luciferase genannt) unter der Kontrolle eines artifiziellen Minimalpromotors enthält. Der Minimalpromotor besteht aus zwei Hypoxie-responsiblen Elementen stromaufwärts einer TATA-Box [Oehme F., Ellinghaus P., Kolkhof P., Smith T.J., Ramakrishnan S., Hütter J., Schramm M., Flamme L, Biochem. Biophys. Res. Commun. 296 (2), 343-9 (2002)]. Unter Einwirkung von Hypoxie (z.B. Kultivierung in Gegenwart von 1% Sauerstoff für 24 Stunden) oder unter Einwirkung unselektiver Dioxygenase-Inhibitoren (z.B. Desferroxamin in einer Konzentration von 100 µM, Cobaltchlorid in einer Konzentration von 100 µM oder *N*-Oxalylglycindiethylester in einer Konzentration von 1 mM) produziert die Testzelllinie Luciferase, die mit Hilfe geeigneter Biolumineszenz-Reagenzien (z.B. Steady-Glo^{®} Luciferase Assay System, Promega Corporation, Madison, WI 53711, USA) und eines geeigneten Luminometers detektiert und quantifiziert werden kann.

Testablauf: Die Zellen werden am Tag vor dem Test in einer exakt bemessenen Menge Kulturmedium (DMEM, 10% FCS, 2 mM Glutamin) in 384- oder 1536-Loch-Mikrotiterplatten ausplattiert und in einem Zellinkubator (96% Luftfeuchtigkeit, 5% v/v CO₂, 37°C) gehalten. Am Testtag werden dem Kulturmedium die Testsubstanzen in abgestuften Konzentrationen zugesetzt. In als Negativkontrolle dienenden Ansätzen wird den Zellen keine Testsubstanz zugesetzt. Als Positivkontrolle zur Bestimmung der Empfindlichkeit der Zelle für Inhibitoren wird z.B. Desferroxamin in einer Endkonzentration von 100 µM zugesetzt. Sechs bis 24 Stunden nach Übertragung der Testsubstanzen in die Löcher der Mikrotiterplatten wird das resultierende Lichtsignal im Luminometer gemessen. Anhand der Meßwerte wird eine Dosiswirkungsbeziehung aufgestellt, die als Grundlage für die Ermittlung der halbmaximalen Wirkkonzentration (als EC₅₀-Wert bezeichnet) dient.

### 1.c) Zellulärer, funktioneller in vitro-Test zur Veränderung der Genexpression:

Um die Veränderung der Expression spezifischer mRNAs in menschlichen Zelllinien nach Behandlung mit Testsubstanzen zu untersuchen, werden folgende Zelllinien auf 6- oder 24-Lochplatten kultiviert: humane Hepatomzellen (HUH, JCRB Cell Bank, Japan), humane embryonale Nierenfibroblasten (HEK/293, ATCC, Manassas, VA 20108, USA), humane Cervixcarcinomzellen (HeLa, ATCC, Manassas, VA 20108, USA), humane Nabelschnurvenenendothelzellen (HUVEC, Cambrex, East Rutherford, New Jersey 07073, USA). 24 Stunden nach Zugabe der Testsubstanzen werden die Zellen mit Phosphat-gepufferter Saline gewaschen und aus ihnen die Gesamt-RNA unter Verwendung einer geeigneten Methode gewonnen (z.B. Trizol^{®}-Reagenz, Invitrogen GmbH, 76131 Karlsruhe, Deutschland).

Für ein typisches Analyseexperiment wird je 1 µg der so gewonnenen Gesamt-RNA mit DNase I verdaut und unter Verwendung einer geeigneten Reversen-Transkriptase-Reaktion (ImProm-II Reverse Transcription System, Promega Corporation, Madison, WI 53711, USA) in eine komplementäre DNA (cDNA) übersetzt. 2.5% des so gewonnenen cDNA-Ansatzes werden jeweils für die Polymerase-Kettenreaktion verwendet. Das Expressionsniveau der mRNA der zu untersuchenden Gene wird mittels der *real time quantitative polymerase chain reaction* [TaqMan-PCR; Heid C.A., Stevens J., Livak K.J., Williams P.M., *Genome Res.* **6** (10), 986-94 (1996)] unter Verwendung eines ABI Prism 7700 Sequenz-Detektionsinstruments (Fa. Applied Biosystems, Inc.) untersucht. Die hierbei benutzten Primer-Sonden-Kombinationen werden mittels der Primer Express 1.5 Software (Fa. Applied Biosystems, Inc.) generiert. Im einzelnen werden die mRNAs von Erythropoetin, Carboanhydrase IX, Lactatdehydrogenase A und vaskulärem Endothelzellwachstumsfaktor untersucht.

Substanzen gemäß der vorliegenden Erfindung führen zu einem signifikanten, dosisabhängigen Anstieg der mRNA Hypoxie-induzierter Gene in Zellen menschlichen Ursprungs.

### 2. In vivo-Tests zum Nachweis der Wirkung im Kardiovaskularsystem

### 2.a) In vivo-Test zur Veränderung der Genexpression:

Mäusen oder Ratten werden die in geeigneten Lösungsmitteln gelösten Prüfverbindungen entweder oral durch Schlundsonden-Applikation, intraperitoneal oder intravenös verabfolgt. Typische Dosierungen sind 0.1, 0.5, 1, 5, 10, 20, 50, 100 und 300 mg Substanz pro kg Körpergewicht und Verabfolgung. Kontrolltiere erhalten nur Lösungsmittel. 4, 8 oder 24 Stunden nach Gabe der Prüfsubstanz werden die Tiere mit einer Überdosis Isofluran und anschließendem Genickbruch getötet und die zu untersuchenden Organe entnommen. Teile der Organe werden in flüssigem Stickstoff schockgefroren. Aus den Organteilen wird wie unter B.1.a) beschrieben Gesamt-RNA gewonnen und diese in eine cDNA übersetzt. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht.

Substanzen gemäß der vorliegenden Erfindung führen im Vergleich mit der Placebo-Kontrolle nach oraler oder parenteraler Verabreichung zu einem signifikanten, dosisabhängigen Anstieg der mRNA des Erythropoetins in der Niere.

### 2.b) Bestimmung des Erythropoetin-Spiegels im Serum:

Mäusen oder Ratten wird die Prüfsubstanz in einem geeigneten Lösungsmittel entweder intraperitoneal oder oral einmal oder zweimal täglich verabreicht. Typische Dosierungen sind 0.1, 0.5, 1, 5, 10, 20, 50, 100 und 300 mg Substanz pro kg Körpergewicht und Verabfolgung. Placebo-Kontrolltiere erhalten nur Lösungsmittel. Vor der Applikation und vier Stunden nach der letzten Substanzgabe wird den Tieren in Kurznarkose aus dem retroorbitalen Venenplexus oder der Schwanzvene 50 µl Blut entnommen. Das Blut wird durch Zusatz von Lithium-Heparin ungerinnbar gemacht. Durch Zentrifugieren wird das Blutplasma gewonnen. In dem Blutplasma wird mit Hilfe eines Erythropoetin-ELISA (Quantikine^{®} mouse Epo Immunoassay, R&D Systems, Inc., Minneapolis, USA) entsprechend der Anleitung des Herstellers der Gehalt an Erythropoetin bestimmt. Die Meßwerte werden anhand einer für Maus-Erythropoetin erhobenen Referenzmessung in pg/ml umgerechnet.

Substanzen gemäß der vorliegenden Erfindung führen nach oraler und parenteraler Verabreichung zu einem signifikanten, dosisabhängigen Anstieg des Plasma-Erythropoetins gegenüber dem Ausgangswert und der Placebo-Kontrolle.

### 2.c) Bestimmung der zellulären Zusammensetzung des peripheren Blutes:

Mäusen oder Ratten wird die Prüfsubstanz in einem geeigneten Lösungsmittel entweder intraperitoneal oder oral einmal oder zweimal täglich über mehrere Tage verabreicht. Typische Dosierungen sind z.B. 0.1, 0.5, 1, 5, 10, 20, 50, 100 und 300 mg Substanz pro kg Körpergewicht und Verabfolgung. Kontrolltiere erhalten nur Lösungsmittel. Am Versuchsende wird den Tieren in Kurznarkose aus dem Venenplexus des Augenwinkels oder der Schwanzvene Blut entnommen und durch Zusatz von Natriumcitrat ungerinnbar gemacht. In einem geeigneten elektronischen Messgerät werden in den Blutproben die Konzentrationen von Erythrozyten, Leukozyten und Thrombozyten bestimmt. Die Konzentration der Retikulozyten wird anhand von Blutausstrichen, die mit einer dafür geeigneten Farblösung (Fa. KABE Labortechnik, Nümbrecht) gefärbt werden, durch mikroskopische Durchmusterung von je 1000 Erythrozyten bestimmt. Für die Bestimmung des Hämatokrits wird Blut aus dem retroorbitalen Venenplexus mittels einer Hämatokritkapillare entnommen und der Hämatokritwert nach Zentrifugieren der Kapillare in einer dafür geeigneten Zentrifuge manuell abgelesen.

Substanzen gemäß der vorliegenden Erfindung führen nach oraler und parenteraler Verabreichung zu einem signifikanten, dosisabhängigen Anstieg des Hämatokrits, der Erythrozytenzahl und der Retikulozyten gegenüber dem Ausgangswert und der Placebo-Kontrolle.

### 3. Bestimmung der Löslichkeit

### Herstellung der Ausgangslösung (Urlösung):

Mindestens 1.5 mg der Testsubstanz werden in ein Wide Mouth 10 mm Screw V-Vial (Fa. Glastechnik Gräfenroda GmbH, Art.-Nr. 8004-WM-H/V15µ) mit passender Schraubkappe und Septum genau eingewogen, mit DMSO zu einer Konzentration von 50 mg/ml versetzt und 30 Minuten mittels eines Vortexers geschüttelt.

### Herstellung der Kalibrierlösungen:

Die notwendigen Pipettierschritte erfolgen in 1.2 ml 96er Deep Well Plate (DWP) mittels eines Liquid-Handling-Roboters. Als Lösemittel wird ein Gemisch aus Acetonitril/Wasser 8:2 verwendet.

*Herstellung der Ausgangslösung für Kalibrierlösungen (Stammlösung):* 10 µl der Urlösung werden mit 833 µl des Lösemittelgemisch versetzt (Konzentration = 600 µg/ml) und homogenisiert. Es werden von jeder Testsubstanz 1:100 Verdünnungen in separaten DWP's hergestellt und wiederum homogenisiert.

*Kalibrierlösung 5 (600 nglml):* 30 µl der Stammlösung werden mit 270 µl Lösemittelgemisch versetzt und homogenisiert.

*Kalibrierlösung 4 (60 nglml):* 30 µl der Kalibrierlösung 5 werden mit 270 µl Lösemittelgemisch versetzt und homogenisiert.

*Kalibrierlösung 3 (12 nglml):* 100 µl der Kalibrierlösung 4 werden mit 400 µl Lösemittelgemisch versetzt und homogenisiert.

*Kalibrierlösung 2 (1.2 nglml):* 30 µl der Kalibrierlösung 3 werden mit 270 µl Lösemittelgemisch versetzt und homogenisiert.

*Kalibrierlösung 1 (0.6 nglml):* 150 µl der Kalibrierlösung 2 werden mit 150 µl Lösemittelgemisch versetzt und homogenisiert.

### Herstellung der Probenlösungen:

Die notwendigen Pipettierschritte erfolgen in 1.2 ml 96er DWP mittels eines Liquid-Handling-Roboters. 10.1 µl der Stammlösung werden mit 1000 µl PBS-Puffer pH 6.5 versetzt. (PBS-Puffer pH 6.5: 61.86 g Natriumchlorid, 39.54 g Natriumdihydrogenphosphat und 83.35 g 1 N Natronlauge werden in einen 1 Liter-Messkolben eingewogen, mit Wasser aufgefüllt und ca. 1 Stunde gerührt. Von dieser Lösung werden 500 ml in einen 5 Liter-Messkolben gegeben und mit Wasser aufgefüllt. Es wird mit 1 N Natronlauge auf pH 6.5 einstellen.)

### Durchführung:

Die notwendigen Pipettierschritte erfolgen in 1.2 ml 96er DWP mittels eines Liquid-Handling-Roboters. Die so hergestellten Probenlösungen werden 24 Stunden bei 1400 rpm mittels eines temperierbaren Schüttlers bei 20°C geschüttelt. Von diesen Lösungen werden jeweils 180 µl abgenommen und in Beckman Polyallomer Centrifuge Tubes überführt. Diese Lösungen werden 1 Stunde mit ca. 223.000 x g zentrifugiert. Von jeder Probenlösung werden 100 µl des Überstandes abgenommen und 1:10 0 und 1:1000 mit PBS-Puffer 6.5 verdünnt.

### Analytik:

Die Proben werden mittels HPLC/MS-MS analysiert. Quantifiziert wird über eine Fünf-Punkt-Kalibrationskurve der Testverbindung. Die Löslichkeit wird in mg/l ausgedrückt. Analysensequenz: 1) Blank (Lösemittelgemisch); 2) Kalibrierlösung 0.6 ng/ml; 3) Kalibrierlösung 1.2 ng/ml; 4) Kalibrierlösung 12 ng/ml; 5) Kalibrierlösung 60 ng/ml; 6) Kalibrierlösung 600 ng/ml; 7) Blank (Lösemittelgemisch); 8) Probenlösung 1:1000; 7) Probenlösung 1:10.

### HPLC/MS-MSMethode

HPLC: Agilent 1100, quat. Pumpe (G1311A), Autosampler CTC HTS PAL, Degaser (G1322A) und Säulenthermostat (G1316A); Säule: Oasis HLB 20 mm x 2.1 mm, 25 µ; Temperatur: 40°C; Eluent A: Wasser + 0.5 ml Ameisensäure/1; Eluent B: Acetonitril + 0.5 ml Ameisensäure/1; Flussrate: 2.5 ml/min; Stoptime 1.5 min; Gradient: 0 min 95% A, 5% B; Rampe: 0-0.5 min 5% A, 95% B; 0.5-0.84 min 5% A, 95% B; Rampe: 0.84-0.85 min 95% A, 5% B; 0.85-1.5 min 95% A, 5% B.

MS/MS: WATERS Quattro Micro Tandem MS/MS; Z-Spray API-Interface; HPLC-MS-Eingangssplitter 1:20; Messung im ESI-Mode.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 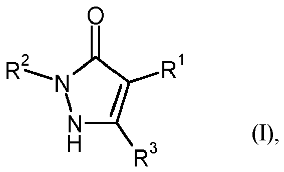 in welcher
R¹ für eine Heteroaryl-Gruppe der Formel 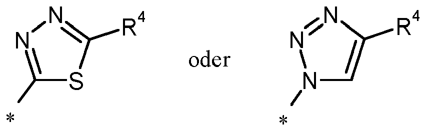 steht, worin
* die Verknüpfungsstelle mit dem Dihydropyrazolon-Ring bedeutet
und
R⁴ Wasserstoff, Fluor, Chlor, Brom, Cyano, (C₁-C₄)-Alkyl, Trifluormethyl, Hydroxymethyl, (C₁-C₄)-Alkoxy, Trifluormethoxy, Hydroxycarbonyl oder (C₁-C₄)-Alkoxycarbonyl bedeutet,
R² für eine Heteroaryl-Gruppe der Formel 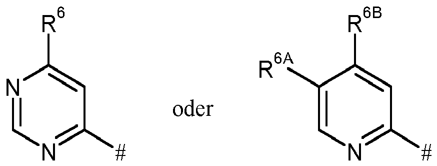 steht, worin
# die Verknüpfungsstelle mit dem Dihydropyrazolon-Ring bedeutet
und
R⁶, R^{6A} und R^{6B} gleich oder verschieden sind und unabhängig voneinander Wasserstoff oder einen Substituenten ausgewählt aus der Reihe Fluor, Chlor, Brom, Cyano, (C₁-C₆)-Alkyl, Trifluormethyl, Hydroxy, (C₁-C₆)-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Heterocycloalkyl, Phenyl und 5- oder 6-gliedriges Heteroaryl bedeuten, wobei
(C₁-C₆)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können,
und
R³ für Wasserstoff steht,
sowie ihre Salze, Solvate und Solvate der Salze.

2. Verbindung der Formel (I) nach Anspruch 1, in welcher
R¹ für eine Heteroaryl-Gruppe der Formel 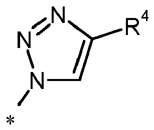 steht, worin
* die Verknüpfungsstelle mit dem Dihydropyrazolon-Ring bedeutet
und
R⁴ Wasserstoff, Fluor, Chlor, Brom, Cyano, (C₁-C₄)-Alkyl, Trifluormethyl, Hydroxymethyl, (C₁-C₄)-Alkoxy, Trifluormethoxy, Hydroxycarbonyl oder (C₁-C₄)-Alkoxycarbonyl bedeutet,
R² für eine Heteroaryl-Gruppe der Formel 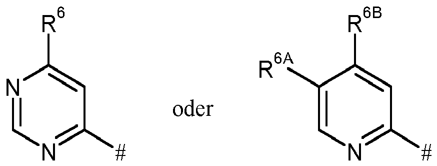 steht, worin
# die Verknüpfungsstelle mit dem Dihydropyrazolon-Ring bedeutet
und
R⁶, R^{6A} und R^{6B} gleich oder verschieden sind und unabhängig voneinander Wasserstoff oder einen Substituenten ausgewählt aus der Reihe Fluor, Chlor, Brom, Cyano, (C₁-C₆)-Alkyl, Trifluormethyl, Hydroxy, (C₁-C₆)-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Heterocycloalkyl, Phenyl und 5- oder 6-gliedriges Heteroaryl bedeuten, wobei
(C₁-C₆)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können,
und
R³ für Wasserstoff steht,
sowie ihre Salze, Solvate und Solvate der Salze.

3. Verbindung der Formel (I) nach Anspruch 1, in welcher
R¹ für eine Heteroaryl-Gruppe der Formel 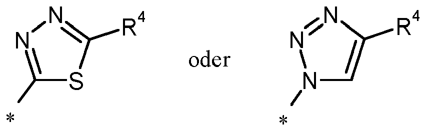 steht, worin
* die Verknüpfungsstelle mit dem Dihydropyrazolon-Ring bedeutet
und
R⁴ Wasserstoff, Fluor, Chlor, Brom, Cyano, (C₁-C₄)-Alkyl, Trifluormethyl, Hydroxymethyl, (C₁-C₄)-Alkoxy, Trifluormethoxy, Hydroxycarbonyl oder (C₁-C₄)-Alkoxycarbonyl bedeutet,
R² für eine Heteroaryl-Gruppe der Formel 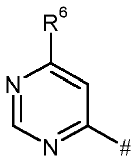 steht, worin
# die Verknüpfungsstelle mit dem Dihydropyrazolon-Ring bedeutet
und
R⁶ Wasserstoff oder einen Substituenten ausgewählt aus der Reihe Fluor, Chlor, Brom, Cyano, (C₁-C₆)-Alkyl, Trifluormethyl, Hydroxy, (C₁-C₆)-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Heterocycloalkyl, Phenyl und 5- oder 6-gliedriges Heteroaryl bedeutet, wobei
(C₁-C₆)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können,
und
R³ für Wasserstoff steht,
sowie ihre Salze, Solvate und Solvate der Salze.

4. 2-(6-Morpholin-4-ylpyrimidin-4-yl)-4-(1*H*-1,2,3-triazol-1-yl)-1,2-dihydro-3*H*-pyrazol-3-on nach Anspruch 1 mit der folgenden Formel 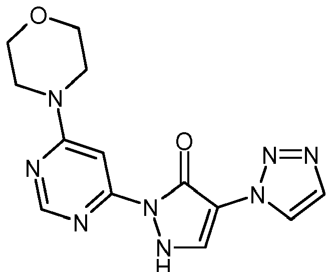 oder eines ihrer Salze, ihrer Solvate oder der Solvate ihrer Salze.

5. 2-(6-Morpholin-4-ylpyrimidin-4-yl)-4-(1*H*-1,2,3-triazol-1-yl)-1,2-dihydro-3*H*-pyrazol-3-on nach Anspruch 1 mit der folgenden Formel

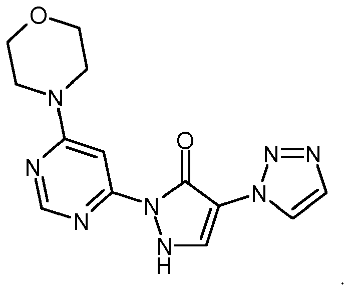

6. 2-(6-Morpholin-4-ylpyrimidin-4-yl)-4-(1*H*-1,2,3-triazol-1-yl)-1,2-dihydro-3*H*-pyrazol-3-one-Hydrochlorid nach Anspruch 1 mit der folgenden Formel

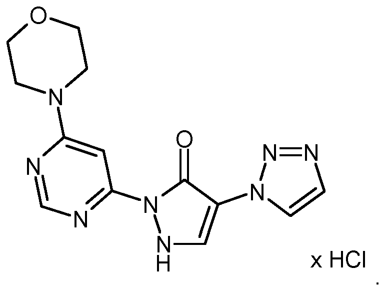

7. Verfahren zur Herstellung einer Verbindung der Formel (I), wie in den Ansprüchen 1 bis 6 definiert, in welcher R³ Wasserstoff bedeutet, **dadurch gekennzeichnet, dass** man zunächst eine Verbindung der Formel (V) 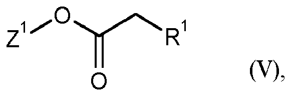 in welcher R¹ die in den Ansprüchen 1 bis 6 angegebene Bedeutung aufweist und
Z¹ für Methyl oder Ethyl steht,
mit einer Verbindung der Formel (VI) 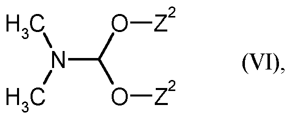 in welcher
Z² für Methyl oder Ethyl steht,
zu Verbindungen der Formel (VII) 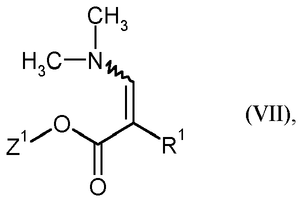 in welcher Z¹ und R¹ die oben angegebenen Bedeutungen aufweisen,
kondensiert und anschließend in Gegenwart einer Säure mit einer Verbindung der Formel (III) 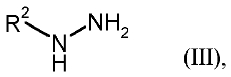 in welcher R² die in den Ansprüchen 1 bis 6 angegebene Bedeutung aufweist,
zu Verbindungen der Formel (IV-A) 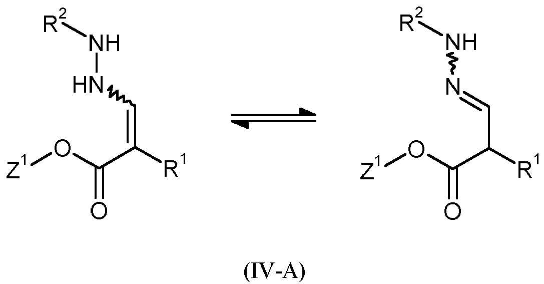 in welcher Z¹, R¹ und R² die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I), worin R³ für Wasserstoff steht, cyclisieren.

8. Verbindung, wie in einem der Ansprüche 1 bis 6 definiert, zur Behandlung und/oder Prophylaxe von Krankheiten.

9. Verwendung einer Verbindung, wie in einem der Ansprüche 1 bis 6 definiert, zur Herstellung eines Arzneimittels zur Behandlung und/oder Prophylaxe von Herz-Kreislauf-Erkrankungen, Herzinsuffizienz, Anämie, chronischen Nierenerkrankungen und Niereninsuffizienz.

10. Arzneimittel enthaltend eine Verbindung, wie in einem der Ansprüche 1 bis 6 definiert, in Kombination mit einem inerten, nicht-toxischen, pharmazeutisch geeigneten Hilfsstoff.

11. Arzneimittel enthaltend eine Verbindung, wie in einem der Ansprüche 1 bis 6 definiert, in Kombination mit einem oder mehreren weiteren Wirkstoffen ausgewählt aus der Gruppe bestehend aus ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika und Antibiotika.

12. Arzneimittel nach Anspruch 10 oder 11 zur Behandlung und/oder Prophylaxe von Herz-Kreislauf-Erkrankungen, Herzinsuffizienz, Anämie, chronischen Nierenerkrankungen und Niereninsuffizienz.

## Claims

1. Compound of the formula (I) 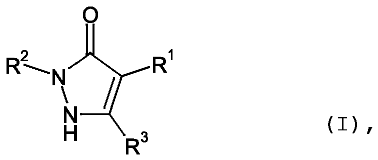 in which
R¹ represents a heteroaryl group of the formula 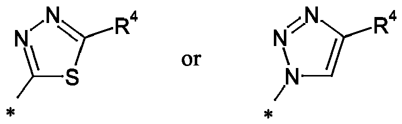 wherein
* denotes the linkage point with the dihydropyrazolone ring
and
R⁴ denotes hydrogen, fluorine, chlorine, bromine, cyano, (C₁-C₄)-alkyl, trifluoromethyl, hydroxymethyl, (C₁-C₄)-alkoxy, trifluoromethoxy, hydroxycarbonyl or (C₁-C₄)-alkoxycarbonyl,
R² represents a heteroaryl group of the formula 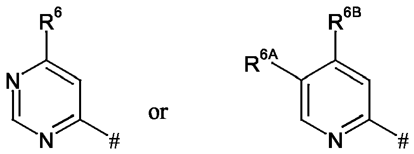 wherein
# denotes the linkage point with the dihydropyrazolone ring
and
R⁶, R^{6A} and R^{6B} are identical or different and independently of one another denote hydrogen or a substituent chosen from the series consisting of fluorine, chlorine, bromine, cyano, (C₁-C₆)-alkyl, trifluoromethyl, hydroxyl, (C₁-C₆)-alkoxy, trifluoromethoxy, amino, mono-(C₁-C₄)-alkylamino, di-(C₁-C₄)-alkylamino, hydroxycarbonyl, (C₁-C₄)-alkoxycarbonyl, 4- to 6-membered heterocycloalkyl, phenyl and 5- or 6-membered heteroaryl, wherein
(C₁-C₆)-alkyl in its turn can be substituted by hydroxyl, (C₁-C₄)-alkoxy or amino
and
4- to 6-membered heterocycloalkyl, phenyl and 5- or 6-membered heteroaryl in their turn can in each case be substituted once or twice in an identical or different manner by fluorine, chlorine, bromine, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and/or (C₁-C₄)-alkoxycarbonyl,
and
R³ represents hydrogen,
and its salts, solvates and solvates of the salts.

2. Compound of the formula (I) according to Claim 1, in which
R¹ represents a heteroaryl group of the formula 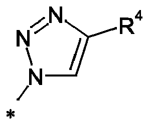 wherein
* denotes the linkage point with the dihydropyrazolone ring
and
R⁴ denotes hydrogen, fluorine, chlorine, bromine, cyano, (C₁-C₄)-alkyl, trifluoromethyl, hydroxymethyl, (C₁-C₄)-alkoxy, trifluoromethoxy, hydroxycarbonyl or (C₁-C₄)-alkoxycarbonyl,
R² represents a heteroaryl group of the formula 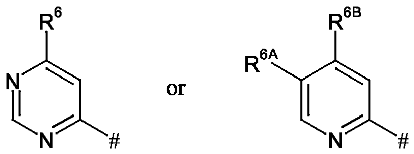 wherein
# denotes the linkage point with the dihydropyrazolone ring
and
R⁶, R^{6A} and R^{6B} are identical or different and independently of one another denote hydrogen or a substituent chosen from the series consisting of fluorine, chlorine, bromine, cyano, (C₁-C₆)-alkyl, trifluoromethyl, hydroxyl, (C₁-C₆)-alkoxy, trifluoromethoxy, amino, mono-(C₁-C₄)-alkylamino, di-(C₁-C₄)-alkylamino, hydroxycarbonyl, (C₁-C₄)-alkoxycarbonyl, 4- to 6-membered heterocycloalkyl, phenyl and 5- or 6-membered heteroaryl, wherein
(C₁-C₆)-alkyl in its turn can be substituted by hydroxyl, (C₁-C₄)-alkoxy or amino
and
4- to 6-membered heterocycloalkyl, phenyl and 5- or 6-membered heteroaryl in their turn can in each case be substituted once or twice in an identical or different manner by fluorine, chlorine, bromine, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and/or (C₁-C₄)-alkoxycarbonyl,
and
R³ represents hydrogen,
and its salts, solvates and solvates of the salts.

3. Compound of the formula (I) according to Claim 1, in which
R¹ represents a heteroaryl group of the formula 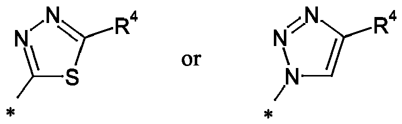 wherein
* denotes the linkage point with the dihydropyrazolone ring
and
R⁴ denotes hydrogen, fluorine, chlorine, bromine, cyano, (C₁-C₄)-alkyl, trifluoromethyl, hydroxymethyl, (C₁-C₄)-alkoxy, trifluoromethoxy, hydroxycarbonyl or (C₁-C₄)-alkoxycarbonyl,
R² represents a heteroaryl group of the formula 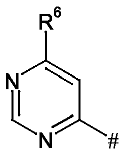 wherein
# denotes the linkage point with the dihydropyrazolone ring
and
R⁶ hydrogen or a substituent chosen from the series consisting of fluorine, chlorine, bromine, cyano, (C₁-C₆)-alkyl, trifluoromethyl, hydroxyl, (C₁-C₆)-alkoxy, trifluoromethoxy, amino, mono-(C₁-C₄)-alkylamino, di-(C₁-C₄)-alkylamino, hydroxycarbonyl, (C₁-C₄)-alkoxycarbonyl, 4- to 6-membered heterocycloalkyl, phenyl and 5- or 6-membered heteroaryl, wherein
(C₁-C₆)-alkyl in its turn can be substituted by hydroxyl, (C₁-C₄)-alkoxy or amino
and
4- to 6-membered heterocycloalkyl, phenyl and 5- or 6-membered heteroaryl in their turn can in each case be substituted once or twice in an identical or different manner by fluorine, chlorine, bromine, cyano, (C₁-C₄)-alkyl, trifluoromethyl, hydroxyl, (C₁-C₄)-alkoxy, trifluoromethoxy, oxo, amino, mono- (C₁-C₄)-alkylamino, di- (C₁-C₄)-alkylamino, hydroxycarbonyl, and/or (C₁-C₄)-alkoxycarbonyl,
and
R³ represents hydrogen,
and its salts, solvates and solvates of the salts.

4. 2-(6-Morpholin-4-ylpyrimidin-4-yl)-4-(1*H*-1,2,3-triazol-1-yl)-1,2-dihydro-3*H*-pyrazol-3-one according to Claim 1 having the following formula 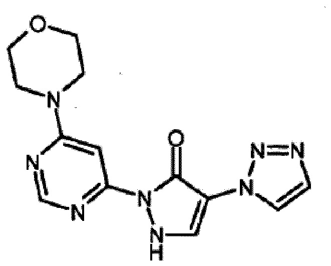 or one of its salts, its solvates or the solvates of its salts.

5. 2-(6-Morpholin-4-ylpyrimidin-4-yl)-4-(1*H*-1,2,3-triazol-1-yl)-1,2-dihydro-3*H*-pyrazol-3-one according to Claim 1 having the following formula

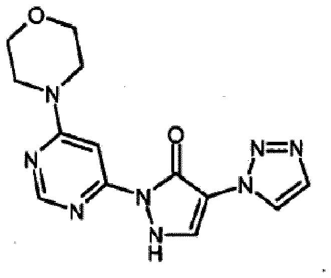

6. 2-(6-Morpholin-4-ylpyrimidin-4-yl)-4-(1*H*-1,2,3-triazol-1-yl)-1,2-dihydro-3*H*-pyrazol-3-one hydrochloride according to Claim 1 having the following formula

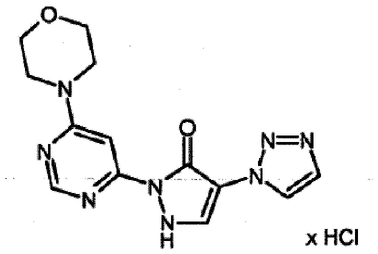

7. Process for the preparation of a compound of the formula (I) as defined in Claims 1 to 6 in which R³ denotes hydrogen, **characterized in that** a compound of the formula (V) 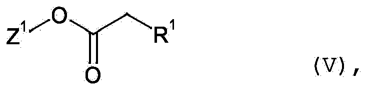 in which R¹ has the meaning given in Claims 1 to 6 and
Z¹ represents methyl or ethyl,
is subjected to a condensation reaction with a compound of the formula (VI) 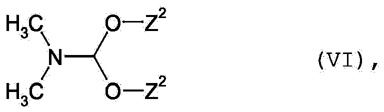 in which
Z² represents methyl or ethyl,
to give compounds of the formula (VII) 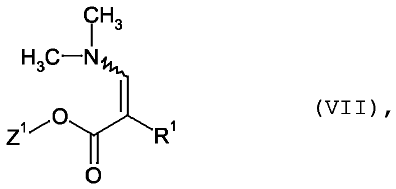 in which Z¹ and R¹ have the abovementioned meanings,
and these are subsequently reacted in the presence of an acid with a compound of the formula (III) 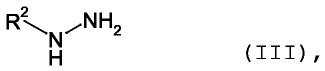 in which R² has the meaning given in Claims 1 to 6,
to give compounds of the formula (IV-A) 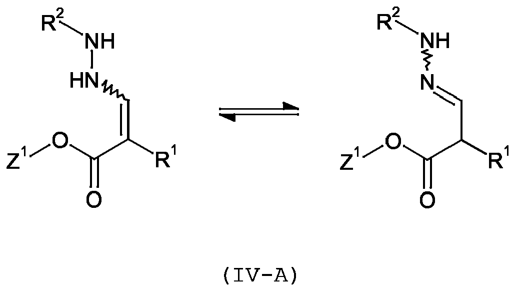 in which Z¹, R¹ and R² have the abovementioned meanings,
which cyclize already under these reaction conditions or in a subsequent reaction step under the influence of a base to give the compounds of the formula (I) wherein R³ represents hydrogen.

8. Compound as defined in one of Claims 1 to 6 for treatment and/or prophylaxis of diseases.

9. Use of a compound as defined in one of Claims 1 to 6 for the preparation of a medicament for treatment and/or prophylaxis of cardiovascular diseases, cardiac insufficiency, anemia, chronic kidney diseases and renal insufficiency.

10. Medicament comprising a compound as defined in one of Claims 1 to 6 in combination with an inert, non-toxic, pharmaceutically suitable auxiliary substance.

11. Medicament comprising a compound as defined in one of Claims 1 to 6 in combination with one or more further active compounds chosen from the group consisting of ACE inhibitors, angiotensin II receptor antagonists, beta receptor blockers, calcium antagonists, PDE inhibitors, mineralocorticoid receptor antagonists, diuretics, aspirin, iron supplements, vitamin B12 and folic acid supplements, statins, digitalis (digoxin) derivatives, tumor chemotherapeutics and antibiotics.

12. Medicament according to Claim 10 or 11 for treatment and/or prophylaxis of cardiovascular diseases, cardiac insufficiency, anemia, chronic kidney diseases and renal insufficiency.

## Revendications

1. Composé de formule (I) 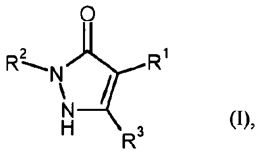 dans laquelle
R¹ représente un groupe hétéroaryle de formule 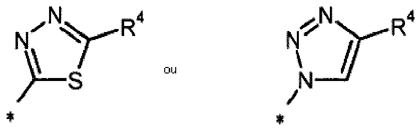 où
* signifie le site de liaison avec le cycle dihydropyrazolone et
R⁴ signifie hydrogène, fluor, chlore, brome, cyano, (C₁-C₄)-alkyle, trifluorométhyle, hydroxyméthyle, (C₁-C₄)-alcoxy, trifluorométhoxy, hydroxycarbonyle ou (C₁-C₄)-alcoxycarbonyle,
R² représente un groupe hétéroaryle de formule 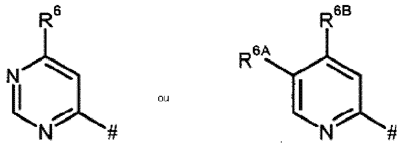 où
# signifie le site de liaison avec le cycle dihydropyrazolone et
R⁶, R^{6A} et R^{6B} sont identiques ou différents et signifient, indépendamment l'un de l'autre, hydrogène ou un substituant choisi dans la série fluor, chlore, brome, cyano, (C₁-C₆)-alkyle, trifluorométhyle, hydroxy, (C₁-C₆)-alcoxy, trifluorométhoxy, amino, mono-(C₁-C₄)-alkylamino, di-(C₁-C₄)-alkylamino, hydroxycarbonyle, (C₁-C₄)-alcoxycarbonyle, hétérocycloalkyle de 4 à 6 chaînons, phényle et hétéroaryle de 5 ou 6 chaînons, (C₁-C₆)-alkyle pouvant être substitué à son tour par hydroxy, (C₁-C₄)-alcoxy ou amino et hétérocycloalkyle de 4 à 6 chaînons, phényle et hétéroaryle de 5 ou 6 chaînons pouvant à leur tour, chacun, être monosubstitué ou disubstitué, de manière identique ou différente, par fluor, chlore, brome, cyano, (C₁-C₄)-alkyle, trifluorométhyle, hydroxy, (C₁-C₄)-alcoxy, trifluorométhoxy, oxo, amino, mono-(C₁-C₄)-alkylamino, di-(C₁-C₄)-alkylamino, hydroxycarbonyle et/ou (C₁-C₄)-alcoxycarbonyle
et
R³ représente hydrogène,
ainsi que ses sels, solvates et les solvates des sels.

2. Composé de formule (I) selon la revendication 1, dans laquelle
R¹ représente un groupe hétéroaryle de formule 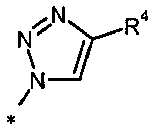 où
* signifie le site de liaison avec le cycle dihydropyrazolone et
R⁴ signifie hydrogène, fluor, chlore, brome, cyano, (C₁-C₄)-alkyle, trifluorométhyle, hydroxyméthyle, (C₁-C₄)-alcoxy, trifluorométhoxy, hydroxycarbonyle ou (C₁-C₄)-alcoxycarbonyle,
R² représente un groupe hétéroaryle de formule 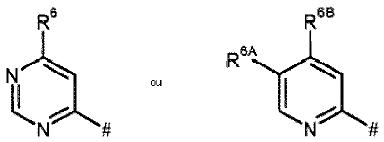 où
# signifie le site de liaison avec le cycle dihydropyrazolone et
R⁶, R^{6A} et R^{6B} sont identiques ou différents et signifient, indépendamment l'un de l'autre, hydrogène ou un substituant choisi dans la série fluor, chlore, brome, cyano, (C₁-C₆)-alkyle, trifluorométhyle, hydroxy, (C₁-C₆)-alcoxy, trifluorométhoxy, amino, mono-(C₁-C₄)-alkylamino, di-(C₁-C₄)-alkylamino, hydroxycarbonyle, (C₁-C₄)-alcoxycarbonyle, hétérocycloalkyle de 4 à 6 chaînons, phényle et hétéroaryle de 5 ou 6 chaînons, (C₁-C₆)-alkyle pouvant être substitué à son tour par hydroxy, (C₁-C₄)-alcoxy ou amino et hétérocycloalkyle de 4 à 6 chaînons, phényle et hétéroaryle de 5 ou 6 chaînons pouvant à leur tour, chacun, être monosubstitué ou disubstitué, de manière identique ou différente, par fluor, chlore, brome, cyano, (C₁-C₄)-alkyle, trifluorométhyle, hydroxy, (C₁-C₄)-alcoxy, trifluorométhoxy, oxo, amino, mono-(C₁-C₄)-alkylamino, di-(C₁-C₄)-alkylamino, hydroxycarbonyle et/ou (C₁-C₄)-alcoxycarbonyle
et
R³ représente hydrogène,
ainsi que ses sels, solvates et les solvates des sels.

3. Composé de formule (I) selon la revendication 1, dans laquelle
R¹ représente un groupe hétéroaryle de formule 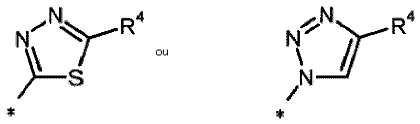 où
* signifie le site de liaison avec le cycle dihydropyrazolone et
R⁴ signifie hydrogène, fluor, chlore, brome, cyano, (C₁-C₄)-alkyle, trifluorométhyle, hydroxyméthyle, (C₁-C₄)-alcoxy, trifluorométhoxy, hydroxycarbonyle ou (C₁-C₄)-alcoxycarbonyle,
R² représente un groupe hétéroaryle de formule 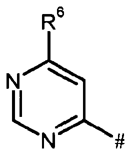 où
# signifie le site de liaison avec le cycle dihydropyrazolone et
R⁶ signifie hydrogène ou un substituant choisi dans la série fluor, chlore, brome, cyano, (C₁-C₆)-alkyle, trifluorométhyle, hydroxy, (C₁-C₆)-alcoxy, trifluorométhoxy, amino, mono-(C₁-C₄)-alkylamino, di-(C₁-C₄)-alkylamino, hydroxycarbonyle, (C₁-C₄)-alcoxycarbonyle, hétérocycloalkyle de 4 à 6 chaînons, phényle et hétéroaryle de 5 ou 6 chaînons, (C₁-C₆)-alkyle pouvant être substitué à son tour par hydroxy, (C₁-C₄)-alcoxy ou amino et hétérocycloalkyle de 4 à 6 chaînons, phényle et hétéroaryle de 5 ou 6 chaînons pouvant à leur tour, chacun, être monosubstitué ou disubstitué, de manière identique ou différente, par fluor, chlore, brome, cyano, (C₁-C₄)-alkyle, trifluorométhyle, hydroxy, (C₁-C₄)-alcoxy, trifluorométhoxy, oxo, amino, mono-(C₁-C₄)-alkylamino, di-(C₁-C₄)-alkylamino, hydroxycarbonyle et/ou (C₁-C₄)-alcoxycarbonyle
et
R³ représente hydrogène,
ainsi que ses sels, solvates et les solvates des sels.

4. 2-(6-morpholin-4-ylpyrimidin-4-yl)-4-(1H-1,2,3-triazol-1-yl)-1,2-dihydro-3H-pyrazol-3-one selon la revendication 1 présentant la formule suivante 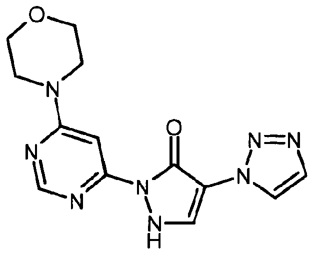 ou un de ses sels, ses solvates ou les solvates de ses sels.

5. 2-(6-morpholin-4-ylpyrimidin-4-yl)-4-(1H-1,2,3-triazol-1-yl)-1,2-dihydro-3H-pyrazol-3-one selon la revendication 1 présentant la formule suivante

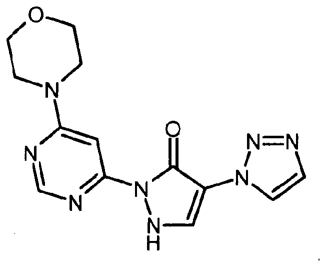

6. Chlorhydrate de 2-(6-morpholin-4-ylpyrimidin-4-yl)-4-(1H-1,2,3-triazol-1-yl)-1,2-dihydro-3H-pyrazol-3-one selon la revendication 1 présentant la formule suivante

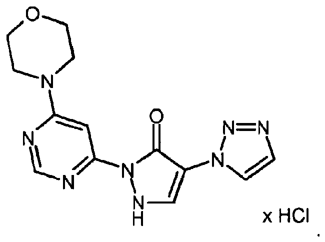

7. Procédé pour la préparation d'un composé de formule (I), tel que défini dans les revendications 1 à 6, dans laquelle R³ signifie hydrogène, **caractérisé en ce qu'**on condense d'abord un composé de formule (V) 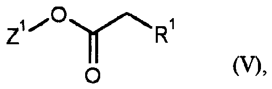 dans laquelle R¹ présente la signification indiquée dans les revendications 1 à 6 et
Z¹ représente méthyle ou éthyle,
avec un composé de formule (VI) 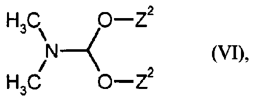 dans laquelle
Z² représente méthyle ou éthyle,
en composés de formule (VII) 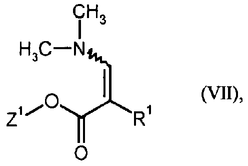 dans laquelle Z¹ et R¹ présentent les significations indiquées ci-dessus,
puis on les transforme en présence d'un acide avec un composé de formule (III) 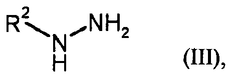 dans laquelle R² présente la signification indiquée dans les revendications 1 à 6,
en composés de formule (IV-A) 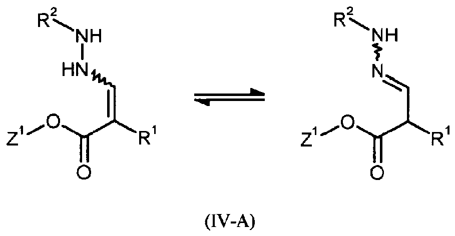 dans laquelle Z¹, R¹ et R² présentent les significations indiquées ci-dessus,
qui cyclisent déjà dans ces conditions de réaction ou dans une étape de réaction consécutive sous l'influence d'une base en composés de formule (I), dans laquelle R³ représente hydrogène.

8. Composé tel que défini dans l'une quelconque des revendications 1 à 6, destiné au traitement et/ou à la prophylaxie de maladies.

9. Utilisation d'un composé tel que défini dans l'une quelconque des revendications 1 à 6, pour la préparation d'un médicament destiné au traitement et/ou à la prophylaxie de maladies cardio-vasculaires, de l'insuffisance cardiaque, de l'anémie, de maladies rénales chroniques et de l'insuffisance rénale.

10. Médicament contenant un composé, tel que défini dans l'une quelconque des revendications 1 à 6, en combinaison avec un adjuvant inerte, non toxique, pharmaceutiquement approprié.

11. Médicament contenant un composé, tel que défini dans l'une quelconque des revendications 1 à 6, en combinaison avec une ou plusieurs autres substances actives, choisies dans le groupe constitué par les inhibiteurs d'ACE, les antagonistes du récepteur de l'angiotensine II, les bloquants des récepteurs bêta, les antagonistes de calcium, les inhibiteurs de PDE, les antagonistes du récepteur des corticoïdes minéraux, les diurétiques, l'aspirine, les suppléments de fer, les suppléments de vitamine B12 et d'acide folique, les statines, les dérivés de digitalis (Digoxine), les agents chimiothérapeutiques ciblant les tumeurs et les antibiotiques.

12. Médicament selon la revendication 10 ou 11 destiné au traitement et/ou à la prophylaxie de maladies cardio-vasculaires, de l'insuffisance cardiaque, de l'anémie, de maladies rénales chroniques et de l'insuffisance cardiaque.
